# EUROPEAN PATENT APPLICATION

(11) **EP 2 174 931 A1**
(43) Date of publication of application: **14.04.2010**
(21) Application number: 08778088.8
(22) Date of filing: 11.07.2008
(51) Int. Cl.: C07D 213/81, A01N 43/40, A01P 3/00

(54) **AMIDE COMPOUND AND METHOD FOR CONTROLLING PLANT DISEASE USING THE SAME**

(30) Priority: 13.07.2007 JP 2007184033
(71) Applicant: Sumitomo Chemical Company, Limited, Tokyo 104-8260 (JP)
(72) Inventor: KOMORI, Takashi, Tyoko 176-0013 (JP); KUBOTA, Mayumi, Toyonaka-shi Osaka 561-0802 (JP); MATSUZAKI, Yuichi, Toyonaka-shi Osaka 560-0021 (JP)
(74) Representative: Vossius & Partner
(86) International application number: PCT/JP2008/062573
(87) International publication number: WO 2009/011305

(57) **Abstract**

Disclosed is a plant disease control agent containing an amide compound represented by formula (1) below which has an excellent plant disease controlling effect as an active ingredient. 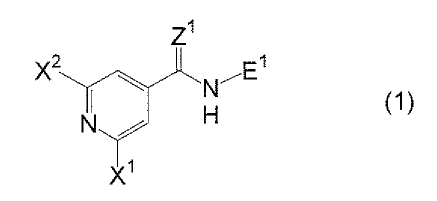 (In the formula, X¹, X², Z¹ and E¹ are as defined in the description.)

## Description

### Technical Field

The present invention relates to an amide compound and a method for controlling plant diseases using the same.

### Background Art

Heretofore, various plant disease control agents have been developed and put into practice. However, these plant disease control agents do not necessarily exhibit sufficient control activity.

### Disclosure of the Invention

### Problems to be Solved by the Invention

An object of the present invention is to provide a compound having an excellent plant disease controlling effect.

### Means for Solving the Problems

The present inventors have studied in order to find out a compound having an excellent plant disease controlling effect and, as a result, have found that an amide compound represented by formula (1) shown below has an excellent plant disease controlling effect. Thus, the present invention has been completed.
The present invention provides [1] to [10] shown below.
[1] An amide compound represented by formula (1): 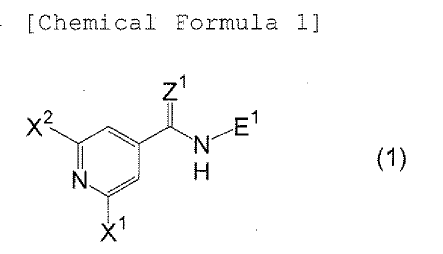 wherein
   X¹ represents a fluorine atom, a C1-C4 alkoxy group, a C3-C4 alkenyloxy group, a C3-C4 alkynyloxy group or an NR¹R² group,
   X² represents a hydrogen atom, a fluorine atom, a C1-C4 alkyl group, a C2-C4 alkenyl group, a C2-C4 alkynyl group, a C1-C4 haloalkyl group, a C1-C4 alkoxy group, a C1-C4 alkylthio group, a C1-C4 hydroxyalkyl group, a cyano group, a formyl group, an NR³R⁴ group, a CO₂R⁵ group, a CONR⁶R⁷ group, or a phenyl group optionally substituted with at least one member selected from the group consisting of a methyl group, a halogen atom, a cyano group and a nitro group,
   Z¹ represents an oxygen atom or a sulfur atom,
   E¹ represents an A¹-Cy¹ group or an A²-CR⁸R⁹R¹⁰ group,
   A¹ represents a single bond, methylene, -CH(CH₃)-, -C(CH₃)₂-, -CH(CH₂CH₃)-, -CH₂CH₂-, -CH₂CH₂CH₂-, cyclopropylidene, or methylene substituted with at least one member selected from the group consisting of a C1-C3 haloalkyl group, a C2-C4 alkenyl group, a C2-C4 alkynyl group, a cyano group and a C2-C5 alkoxycarbonyl group,
   A² represents methylene, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group,
   Cy¹ represents a C3-C6 cycloalkyl group optionally substituted with at least one member selected from the group [a-1] shown below, a C3-C6 cycloalkenyl group optionally substituted with at least one member selected from the group [a-1] shown below, a C3-C6 cycloalkyl group optionally substituted with at least one member selected from the group [a-1] shown below wherein one methylene forming ring is replaced by a carbonyl group, or a C3-C6 hydroxyiminocycloalkyl group optionally substituted with at least one member selected from the group [a-1] shown below,
   R¹ and R² independently represent a hydrogen atom, a C1-C4 alkyl group, a C3-C4 alkenyl group, a C3-C4 alkynyl group, a C2-C4 haloalkyl group, a C2-C5 alkylcarbonyl group, a C2-C5 alkoxycarbonyl group or a C1-C4 alkylsulfonyl group,
   R³ and R⁴ independently represent a hydrogen atom, a C1-C4 alkyl group, a C3-C4 alkenyl group, a C3-C4 alkynyl group, a C2-C4 haloalkyl group, a C2-C5 alkylcarbonyl group, a C2-C5 alkoxycarbonyl group or a C1-C4 alkylsulfonyl group,
   R⁵ represents a C1-C4 alkyl group, a C3-C4 alkenyl group or a C3-C4 alkynyl group,
   R⁶ represents a hydrogen atom, a C1-C4 alkyl group, a C3-C4 alkenyl group, a C3-C4 alkynyl group, a C2-C4 haloalkyl group, a C2-C5 alkylcarbonyl group, a C2-C5 alkoxycarbonyl group or a C1-C4 alkylsulfonyl group,
   R⁷ represents a hydrogen atom, a C1-C4 alkyl group, a C3-C4 alkenyl group, a C3-C4 alkynyl group or a C2-C4 haloalkyl group,
   R⁸ and R⁹ independently represent a C1-C4 alkyl group,
   R¹⁰ represents a hydrogen atom, a C1-C4 alkyl group, a C2-C4 alkenyl group, a C2-C4 alkynyl group, a cyano group, a carboxyl group, a C2-C5 alkoxycarbonyl group, a halogen atom, a hydroxyl group, a C1-C6 alkoxy group, a C3-C6 alkenyloxy group, a C1-C6 haloalkyl group, a C2-C6 haloalkoxy group, a C1-C3 alkylthio group, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a C2-C6 cyanoalkyl group, a C1-C3 alkylsulfonyl group, or an NR¹¹R¹² group, in which R¹¹ and R¹² independently represent a hydrogen atom, a C1-C4 alkyl group, a C2-C5 alkylcarbonyl group, a C2-C5 alkoxycarbonyl group or a C1-C4 alkylsulfonyl group, and
   the group [a-1]:
   a halogen atom, a C1-C4 alkyl group, a C2-C4 alkenyl group, a C2-C4 alkynyl group, a hydroxyl group, a cyano group, a carboxyl group, a C2-C5 alkoxycarbonyl group, a C1-C6 alkoxy group, a C3-C6 alkenyloxy group, a C1-C6 haloalkyl group, a C1-C6 haloalkoxy group, a C1-C3 alkylthio group, a C1-C3 alkylidene group which forms a double bond with a ring-forming carbon atom, a C1-C6 hydroxyalkyl group, C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a formyl group, a C2-C6 cyanoalkyl group, a C1-C3 alkylsulfonyl group, a phenoxy group, or an NR¹³R¹⁴ group, in which R¹³ and R¹⁴ independently represent a hydrogen atom, a C1-C4 alkyl group, a C2-C5 alkylcarbonyl group, a C2-C5 alkoxycarbonyl group and a C1-C4 alkylsulfonyl group.
[2] The amide compound according to [1], wherein A¹ is a single bond, methylene, -CH(CH₃)-, -C(CH₃)₂-, -CH(CH₂CH₃)-, or methylene substituted with at least one member selected from the group consisting of a C2-C4 alkenyl group and a C2-C4 alkynyl group,
   A² is methylene, -CH(CH₃)-, -C(CH₃)₂-, -CH(CH₂CH₃)-, or methylene substituted with at least one member selected from the group consisting of a C2-C4 alkenyl group and a C2-C4 alkynyl group,
   R¹⁰ is a hydrogen atom, a C1-C4 alkyl group, a C2-C4 alkenyl group, a C2-C4 alkynyl group, a halogen atom, a hydroxyl group, a C1-C6 alkoxy group, a C3-C6 alkenyloxy group, a C1-C3 alkylthio group or a C1-C6 hydroxyalkyl group, and
   the group [a-1] consists of a halogen atom, a C1-C4 alkyl group, a C2-C4 alkenyl group, a C2-C4 alkynyl group, a hydroxyl group, a cyano group, a C1-C6 alkoxy group, a C3-C6 alkenyloxy group, a C1-C6 haloalkyl group, a C1-C6 haloalkoxy group, a C1-C3 alkylthio group, a C1-C3 alkylidene group which forms a double bond with a ring-forming carbon atom, a C1-C6 hydroxyalkyl group and a C2-C4 alkylcarbonyloxy group.
[3] The amide compound according to [1] or [2], wherein A¹ is a single bond, methylene or -CH(CH₃)-,
   A² is methylene, -CH(CH₃)- or -C(CH₃)₂-,
   R¹⁰ is a hydrogen atom, a C1-C4 alkyl group, a C2-C4 alkenyl group, a C2-C4 alkynyl group, a halogen atom, a hydroxyl group, a C1-C6 alkoxy group, a C3-C6 alkenyloxy group, a C1-C3 alkylthio group or a C1-C6 hydroxyalkyl group,
   Cy¹ is a C3-C6 cycloalkyl group optionally substituted with at least one member selected from a halogen atom, a C1-C4 alkyl group and a hydroxyl group, or a C3-C6 cycloalkenyl group optionally substituted with at least one member selected from a halogen atom, a C1-C4 alkyl group and a hydroxyl group.
[4] The amide compound according to [1] or [2], wherein E¹ is an A¹-Cy¹ group, A¹ is a single bond, methylene, -CH(CH₃)-, -C(CH₃)₂-, -CH(CH₂CH₃)-, or methylene substituted with at least one member selected from the group consisting of a C2-C4 alkenyl group and a C2-C4 alkynyl group, and
   the group [a-1] consists of a halogen atom, a C1-C4 alkyl group, a C2-C4 alkenyl group, a C2-C4 alkynyl group, a hydroxyl group, a cyano group, a C1-C6 alkoxy group, a C3-C6 alkenyloxy group, a C1-C6 haloalkyl group, a C1-C6 haloalkoxy group, a C1-C3 alkylthio group, a C1-C3 alkylidene group which forms a double bond with a ring-forming carbon atom, a C1-C6 hydroxyalkyl group and a C2-C4 alkylcarbonyloxy group.
[5] The amide compound according to [1] or [2], wherein E¹ is an A²-CR⁸R⁹R¹⁰ group, A² is methylene, -CH(CH₃)-, -C(CH₃)₂-, -CH(CH₂CH₃)-, or a methylene group substituted with at least one member selected from the group consisting of a C2-C4 alkenyl group and a C2-C4 alkynyl group, and R¹⁰ is a hydrogen atom, a C1-C4 alkyl group, a C2-C4 alkenyl group, a C2-C4 alkynyl group, a halogen atom, a hydroxyl group, a C1-C6 alkoxy group, a C3-C6 alkenyloxy group, a C1-C3 alkylthio group or a C1-C6 hydroxyalkyl group.
[6] The amide compound according to any one of [1] to [5], wherein X¹ is a fluorine atom, a C1-C4 alkoxy group or an NR¹R² group, R¹ is a hydrogen atom or a C1-C4 alkyl group, R² is a hydrogen atom or a C1-C4 alkyl group, and Z¹ is an oxygen atom.
[7] The amide compound according to any one of [1] to [5], wherein X¹ is a fluorine atom, a methoxy group or an amino group, X² is a hydrogen atom, a fluorine atom, a methoxy group, a methylthio group or an amino group, and Z¹ is an oxygen atom.
[8] A plant disease control composition comprising the amide compound according to any one of [1] to [7].
[9] A method for controlling plant diseases, which comprises applying an effective amount of the amide compound according to any one of [1] to [7] to plants or soil.
   Hereinafter, the plant disease control composition of [8] may be referred to as the control composition of the present invention, and the method for controlling plant diseases of [9] may be referred to as the control method of the present invention.

### Effect of the Invention

According to the present invention, plant diseases can be controlled.

### Best Mode for Carrying Out the Invention

The compound of the present invention is an amide compound represented by the above formula (1).
In the formula (1), X¹ represents a fluorine atom, a C1-C4 alkoxy group, a C3-C4 alkenyloxy group, a C3-C4 alkynyloxy group or an NR¹R² group.
Examples of the C1-C4 alkoxy group represented by X¹ include a methoxy group, an ethoxy group, a 1-methylethoxy group, a 1,1-dimethylethoxy group, a propoxy group, a 1-methylpropoxy group, a 2-methylpropoxy group, and a butoxy group.
Examples of the C3-C4 alkenyloxy group represented by X¹ include a 1-propenyloxy group, a 2-propenyloxy group, a 2-butenyloxy group, and a 3-butenyloxy group.
Examples of the C3-C4 alkynyloxy group represented by X¹ include a 1-propynyloxy group, a 2-propynyloxy group, and a 3-butynyloxy group.

R¹ and R² independently represent a hydrogen atom, a C1-C4 alkyl group, a C3-C4 alkenyl group, a C3-C4 alkynyl group, a C2-C4 haloalkyl group, a C2-C5 alkylcarbonyl group, a C2-C5 alkoxycarbonyl group or a C1-C4 alkylsulfonyl group.
Examples of the C1-C4 alkyl group represented by R¹ include a methyl group, an ethyl group, a 1-methylethyl group, a 1,1-dimethylethyl group, a propyl group, and a 1-methylpropyl group.
Examples of the C3-C4 alkenyl group represented by R¹ include a 1-propenyl group, a 2-propenyl group, a 2-butenyl group, and a 3-butenyl group.
Examples of the C3-C4 alkynyl group represented by R¹ include a 1-propynyl group, a 2-propynyl group, and a 3-butynyl group.
Examples of the C2-C4 haloalkyl group represented by R¹ include a 1,1-difluoroethyl group, a 2,2,2-trifluoroethyl group, a 2-fluoroethyl group, a 3-fluoropropyl group, a 4-fluorobutyl group, and a 1-chloroethyl group.
Examples of the C2-C5 alkylcarbonyl group represented by R¹ include an acetyl group, an ethylcarbonyl group, a 1-methylethylcarbonyl group, and a 1,1-dimethylethylcarbonyl group.
Examples of the C2-C5 alkoxycarbonyl group represented by R¹ include a methoxycarbonyl group, an ethoxycarbonyl group, a 1-methylethoxycarbonyl group, and a 1,1-dimethylethoxycarbonyl group.
Examples of the C1-C4 alkylsulfonyl group represented by R¹ include a methylsulfonyl group, an ethylsulfonyl group, a 1-methylethylsulfonyl group, and a 1,1-dimethylethylsulfonyl group.

Examples of the C1-C4 alkyl group represented by R² include a methyl group, an ethyl group, a 1-methylethyl group, a 1,1-dimethylethyl group, a propyl group, and a 1-methylpropyl group.
Examples of the C3-C4 alkenyl group represented by R² include a 1-propenyl group, a 2-propenyl group, a 2-butenyl group, and a 3-butenyl group.
Examples of the C3-C4 alkynyl group represented by R² include a 1-propynyl group, a 2-propynyl group, and a 3-butynyl group.
Examples of the C2-C4 haloalkyl group represented by R² include a 1,1-difluoroethyl group, a 2,2,2-trifluoroethyl group, a 2-fluoroethyl group, a 3-fluoropropyl group, a 4-fluorobutyl group, and a 1-chloroethyl group.
Examples of the C2-C5 alkylcarbonyl group represented by R² include an acetyl group, an ethylcarbonyl group, a 1-methylethylcarbonyl group, and a 1,1-dimethylethylcarbonyl group.
Examples of the C2-C5 alkoxycarbonyl group represented by R² include a methoxycarbonyl group, an ethoxycarbonyl group, a 1-methylethoxycarbonyl group, and a 1,1-dimethylethoxycarbonyl group.
Examples of the C1-C4 alkylsulfonyl group represented by R² include a methylsulfonyl group, an ethylsulfonyl group, a 1-methylethylsulfonyl group, and a 1,1-dimethylethylsulfonyl group.

Examples of the NR¹R² group include an amino group, a methylamino group, a dimethylamino group, an ethylamino group, a 2-propenylamino group, a 2-propynylamino group, a 2-chloroethylamino group, an acetylamino group, a propionylamino group, a 1,1-dimethylethylcarbonylamino group, a methoxycarbonylamino group, an ethoxycarbonylamino group, a methanesulfonylamino group, an N-acetyl-N-methylamino group, an N-ethoxycarbonyl-N-methylamino group, and a methanesulfonylmethylamino group.

In the formula (1), X² represents a hydrogen atom, a fluorine atom, a C1-C4 alkyl group, a C2-C4 alkenyl group, a C2-C4 alkynyl group, a C1-C4 haloalkyl group, a C1-C4 alkoxy group, a C1-C4 alkylthio group, a C1-C4 hydroxyalkyl group, a cyano group, a formyl group, an NR³R⁴ group, a CO₂R⁵ group, a CONR⁶R⁷ group, or a phenyl group optionally substituted with at least one member selected from the group consisting of a methyl group, a halogen atom, a cyano group and a nitro group.
Examples of the C1-C4 alkyl group represented by X² include a methyl group, an ethyl group, a 1-methylethyl group, a 1,1-dimethylethyl group, a propyl group, and a 1-methylpropyl group.
Examples of the C2-C4 alkenyl group represented by X² include a vinyl group, a 1-propenyl group, a 2-propenyl group, a 2-butenyl group, and a 3-butenyl group.
Examples of the C2-C4 alkynyl group represented by X² include an ethynyl group, a 1-propynyl group, a 2-propynyl group, and a 3-butynyl group.
Examples of the C1-C4 haloalkyl group represented by X² include a fluoromethyl group, a chloromethyl group, a bromomethyl group, a difluoromethyl group, a dichloromethyl group, a dibromomethyl group, a trifluoromethyl group, a trichloromethyl group, a dichlorofluoromethyl group, a chlorodifluoromethyl group, a 1,1-difluoroethyl group, a 2,2,2-trifluoroethyl group, a 2-fluoroethyl group, a 3-fluoropropyl group, a 4-fluorobutyl group, and a 1-chloroethyl group.
Examples of the C1-C4 alkoxy group represented by X² include a methoxy group, an ethoxy group, a 1-methylethoxy group, a 1,1-dimethylethoxy group, a propoxy group, a 1-methylpropoxy group, a 2-methylpropoxy group, and a butoxy group.
Examples of the C1-C4 alkylthio group represented by X² include a methylthio group, an ethylthio group, a 1-methylethylthio group, a 1,1-dimethylethylthio group, a propylthio group, and a 1-methylpropylthio group.
Examples of the C1-C4 hydroxyalkyl group represented by X² include a hydroxymethyl group, a 1-hydroxyethyl group, and a 2-hydroxyethyl group.
Examples of the phenyl group optionally substituted with at least one member selected from the group consisting of a methyl group, a halogen atom, a cyano group and a nitro group which is represented by X² include a phenyl group, a 2-methylphenyl group, a 3-methylphenyl group, a 4-methylphenyl group, a 2-fluorophenyl group, a 3-fluorophenyl group, a 4-fluorophenyl group, a 2-chlorophenyl group, a 3-chlorophenyl group, a 4-chlorophenyl group, a 4-cyanophenyl group, and a 4-nitrophenyl group.

R³ and R⁴ independently represent a hydrogen atom, a C1-C4 alkyl group, a C3-C4 alkenyl group, a C3-C4 alkynyl group, a C2-C4 haloalkyl group, a C2-C5 alkylcarbonyl group, a C2-C5 alkoxycarbonyl group or a C1-C4 alkylsulfonyl group.
Examples of the C1-C4 alkyl group represented by R³ include a methyl group, an ethyl group, a 1-methylethyl group, a 1,1-dimethylethyl group, a propyl group, and a 1-methylpropyl group.
Examples of the C3-C4 alkenyl group represented by R³ include a 1-propenyl group, a 2-propenyl group, a 2-butenyl group, and a 3-butenyl group.
Examples of the C3-C4 alkynyl group represented by R³ include a 1-propynyl group, a 2-propynyl group, and a 3-butynyl group.
Examples of the C2-C4 haloalkyl group represented by R³ include a 1,1-difluoroethyl group, a 2,2,2-trifluoroethyl group, a 2-fluoroethyl group, a 3-fluoropropyl group, a 4-fluorobutyl group, and a 1-chloroethyl group.
Examples of the C2-C5 alkylcarbonyl group represented by R³ include an acetyl group, an ethylcarbonyl group, a 1-methylethylcarbonyl group, and a 1,1-dimethylethylcarbonyl group.
Examples of the C2-C5 alkoxycarbonyl group represented by R³ include a methoxycarbonyl group, an ethoxycarbonyl group, a 1-methylethoxycarbonyl group, and a 1,1-dimethylethoxycarbonyl group.
Examples of the C1-C4 alkylsulfonyl group represented by R³ include a methylsulfonyl group, an ethylsulfonyl group, a 1-methylethylsulfonyl group, and a 1,1-dimethylethylsulfonyl group.

Examples of the C1-C4 alkyl group represented by R⁴ include a methyl group, an ethyl group, a 1-methylethyl group, a 1,1-dimethylethyl group, a propyl group, and a 1-methylpropyl group.
Examples of the C3-C4 alkenyl group represented by R⁴ include a 1-propenyl group, a 2-propenyl group, a 2-butenyl group, and a 3-butenyl group.
Examples of the C3-C4 alkynyl group represented by R⁴ include a 1-propynyl group, a 2-propynyl group, and a 3-butynyl group.
Examples of the C2-C4 haloalkyl group represented by R⁴ include a 1,1-difluoroethyl group, a 2,2,2-trifluoroethyl group, a 2-fluoroethyl group, a 3-fluoropropyl group, a 4-fluorobutyl group, and a 1-chloroethyl group.
Examples of the C2-C5 alkylcarbonyl group represented by R⁴ include an acetyl group, an ethylcarbonyl group, a 1-methylethylcarbonyl group, and a 1,1-dimethylethylcarbonyl group.
Examples of the C2-C5 alkoxycarbonyl group represented by R⁴ include a methoxycarbonyl group, an ethoxycarbonyl group, a 1-methylethoxycarbonyl group, and a 1,1-dimethylethoxycarbonyl group.
Examples of the C1-C4 alkylsulfonyl group represented by R⁴ include a methylsulfonyl group, an ethylsulfonyl group, a 1-methylethylsulfonyl group, and a 1,1-dimethylethylsulfonyl group.
Examples of the NR³R⁴ group include an amino group, a methylamino group, a dimethylamino group, an ethylamino group, a 2-propenylamino group, a 2-propynylamino group, a 2-chloroethylamino group, an acetylamino group, a propionylamino group, a 1,1-dimethylethylcarbonylamino group, a methoxycarbonylamino group, an ethoxycarbonylamino group, a methanesulfonylamino group, an N-acetyl-N-methylamino group, an N-ethoxycarbonyl-N-methylamino group, and a methanesulfonylmethylamino group.

R⁵ of the CO₂R⁵ group represents a C1-C4 alkyl group, a C3-C4 alkenyl group or a C3-C4 alkynyl group.
Examples of the C1-C4 alkyl group represented by R⁵ include a methyl group, an ethyl group, a 1-methylethyl group, a 1,1-dimethylethyl group, a propyl group, and a 1-methylpropyl group.
Examples of the C3-C4 alkenyl group represented by R⁵ include a 1-propenyl group, a 2-propenyl group, a 2-butenyl group, and a 3-butenyl group.
Examples of the C3-C4 alkynyl group represented by R⁵ include a 1-propynyl group, a 2-propynyl group, and a 3-butynyl group.

R⁶ of the CONR⁶R⁷ group represents a hydrogen atom, a C1-C4 alkyl group, a C3-C4 alkenyl group, a C3-C4 alkynyl group, a C2-C4 haloalkyl group, a C2-C5 alkylcarbonyl group, a C2-C5 alkoxycarbonyl group or a C1-C4 alkylsulfonyl group.
Examples of the C1-C4 alkyl group represented by R⁶ include a methyl group, an ethyl group, a 1-methylethyl group, a 1,1-dimethylethyl group, a propyl group, and a 1-methylpropyl group.
Examples of the C3-C4 alkenyl group represented by R⁶ include a 1-propenyl group, a 2-propenyl group, a 2-butenyl group, and a 3-butenyl group.
Examples of the C3-C4 alkynyl group represented by R⁶ include a 1-propynyl group, a 2-propynyl group, and a 3-butynyl group.
Examples of the C2-C4 haloalkyl group represented by R⁶ include a 1,1-difluoroethyl group, a 2,2,2-trifluoroethyl group, a 2-fluoroethyl group, a 3-fluoropropyl group, a 4-fluorobutyl group, and a 1-chloroethyl group.
Examples of the C2-C5 alkylcarbonyl group represented by R⁶ include an acetyl group, an ethylcarbonyl group, a 1-methylethylcarbonyl group, and a 1,1-dimethylethylcarbonyl group.
Examples of the C2-C5 alkoxycarbonyl group represented by R⁶ include a methoxycarbonyl group, an ethoxycarbonyl group, a 1-methylethoxycarbonyl group, and a 1,1-dimethylethoxycarbonyl group.
Examples of the C1-C4 alkylsulfonyl group represented by R⁶ include a methylsulfonyl group, an ethylsulfonyl group, a 1-methylethylsulfonyl group, and a 1,1-dimethylethylsulfonyl group.

R⁷ of the CONR⁶R⁷ group represents a hydrogen atom, a C1-C4 alkyl group, a C3-C4 alkenyl group, a C3-C4 alkynyl group or a C2-C4 haloalkyl group.
Examples of the C1-C4 alkyl group represented by R⁷ include a methyl group, an ethyl group, a 1-methylethyl group, a 1,1-dimethylethyl group, a propyl group, and a 1-methylpropyl group.
Examples of the C3-C4 alkenyl group represented by R⁷ include a 1-propenyl group, a 2-propenyl group, a 2-butenyl group, and a 3-butenyl group.
Examples of the C3-C4 alkynyl group represented by R⁷ include a 1-propynyl group, a 2-propynyl group, and a 3-butynyl group.
Examples of the C2-C4 haloalkyl group represented by R⁷ include a 1,1-difluoroethyl group, a 2,2,2-trifluoroethyl group, a 2-fluoroethyl group, a 3-fluoropropyl group, a 4-fluorobutyl group, and a 1-chloroethyl group.
Examples of the CONR⁶R⁷ group include a carbamoyl group, a methylcarbamoyl group, a dimethylcarbamoyl group, an ethylmethylcarbamoyl group, a (2-propenyl)carbamoyl group, a (2-propynyl)carbamoyl group, and a 2-chloroethylcarbamoyl group.

In the formula (1), Z¹ represents an oxygen atom or a sulfur atom, and E¹ represents an A¹-Cy¹ group or an A²-CR⁸R⁹R¹⁰ group.

A¹ represents a single bond, methylene, -CH(CH₃)-, -C(CH₃)₂-, -CH(CH₂CH₃)-, -CH₂CH₂-, -CH₂CH₂CH₂-, cyclopropylidene, or methylene substituted with at least one member selected from the group consisting of a C1-C3 haloalkyl group, a C2-C4 alkenyl group, a C2-C4 alkynyl group, a cyano group and a C2-C5 alkoxycarbonyl group (hereinafter, this methylene may be referred to as a "substituted methylene a¹").
Examples of the C1-C3 haloalkyl group for the substituted methylene a¹ include a fluoromethyl group, a chloromethyl group, a bromomethyl group, a difluoromethyl group, a dichloromethyl group, a dibromomethyl group, a trifluoromethyl group, a trichloromethyl group, a dichlorofluoromethyl group, a chlorodifluoromethyl group, a 1,1-difluoroethyl group, a 2,2,2-trifluoroethyl group, a 2-fluoroethyl group, a 3-fluoropropyl group, and a 1-chloroethyl group.
Examples of the C2-C4 alkenyl group for the substituted methylene a¹ include a vinyl group, a 1-propenyl group, a 2-propenyl group, a 2-butenyl group, and a 3-butenyl group.
Examples of the C2-C4 alkynyl group for the substituted methylene a¹ include an ethynyl group, a 1-propynyl group, a 2-propynyl group, and a 3-butynyl group.
Examples of the C2-C5 alkoxycarbonyl group for the substituted methylene a¹ include a methoxycarbonyl group, an ethoxycarbonyl group, a 1-methylethoxycarbonyl group, and a 1,1-dimethylethoxycarbonyl group.
Specific examples of the substituted methylene a¹ include a 2,2,2-trifluoroethane-1,1-diyl group, a 2,2-difluoroethane-1,1-diyl group, a 2-fluoroethane-1,1-diyl group, a 3,3,3-trifluoropropane-1,1-diyl group, a 2,2,2-trichloroethane-1,1-diyl group, a 2,2-dichloroethanediyl group, a 2-chloroethane-1,1-diyl group, a 2-propene-1,1-diyl group, a 2-butene-1,1-diyl group, a 3-methyl-2-butene-1,1-diyl group, a 2-propyne-1,1-diyl group, a 2-butyne-1,1-diyl group, a 3-butyne-1,1-diyl group, a cyanomethylene group, a (methoxycarbonyl)methylene group, an (ethoxycarbonyl)methylene group, and a (1-methylethoxycarbonyl)methyl group.

Cy¹ represents a C3-C6 cycloalkyl group optionally substituted with at least one member selected from the group [a-1] shown below, a C3-C6 cycloalkenyl group optionally substituted with at least one member selected from the group [a-1] shown below, a C3-C6 cycloalkyl group optionally substituted with at least one member selected from the group [a-1] shown below wherein one methylene forming ring is replaced by a carbonyl group (hereinafter, this cycloalkyl group may be referred to as a "substituted cycloalkyl group"), or a C3-C6 hydroxyiminocycloalkyl group optionally substituted with at least one member selected from the group [a-1] shown below.
The group [a-1] consists of:
a halogen atom, a C1-C4 alkyl group, a C2-C4 alkenyl group, a C2-C4 alkynyl group, a hydroxyl group, a cyano group, a carboxyl group, a C2-C5 alkoxycarbonyl group, a C1-C6 alkoxy group, a C3-C6 alkenyloxy group, a C1-C6 haloalkyl group, a C1-C6 haloalkoxy group, a C1-C3 alkylthio group, a C1-C3 alkylidene group which forms a double bond with a ring-forming carbon atom, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a formyl group, a C2-C6 cyanoalkyl group, a C1-C3 alkylsulfonyl group, a phenoxy group and an NR¹³R¹⁴ group.

Hereinafter, the description "optionally substituted with at least one member selected from the group [a-1]" may be abbreviated to "optionally substituted with a group of the group [a-1]".
Herein, the description "optionally substituted with" means that one or more hydrogen atoms may be replaced by the listed substituents unless otherwise specified.

The halogen atom in the group [a-1] includes a fluorine atom, a chlorine atom, a bromine atom and an iodine atom.
Examples of the C1-C4 alkyl group in the group [a-1] include a methyl group, an ethyl group, a 1-methylethyl group, a 1,1-dimethylethyl group, a propyl group, and a 1-methylpropyl group.
Examples of the C2-C4 alkenyl group in the group [a-1] include a vinyl group, a 1-propenyl group, a 2-propenyl group, a 2-butenyl group, and a 3-butenyl group.
Examples of the C2-C4 alkynyl group in the group [a-1] include an ethynyl group, a 1-propynyl group, a 2-propynyl group, and a 3-butynyl group.
Examples of the C2-C5 alkoxycarbonyl group in the group [a-1] include a methoxycarbonyl group, an ethoxycarbonyl group, a 1-methylethoxycarbonyl group, and a 1,1-dimethylethoxycarbonyl group.
Examples of the C1-C6 alkoxy group in the group [a-1] include a methoxy group, an ethoxy group, a propoxy group, an isopropoxy group, a butoxy group, an isobutoxy group, a sec-butoxy group, a tert-butoxy group, a pentyloxy group, an isopentyloxy group, and a hexyloxy group.
Examples of the C3-C6 alkenyloxy group in the group [a-1] include a 2-propenyloxy group, a 1-methyl-2-propenyloxy group, a 2-methyl-2-propenyloxy group, a 2-butenyloxy group, a 3-butenyloxy group, a 2-hexenyloxy group, and a 5-hexenyloxy group.
Examples of the C1-C6 haloalkyl group in the group [a-1] include a fluoromethyl group, a difluoromethyl group, a trifluoromethyl group, a trichloromethyl group, a chlorofluoromethyl group, a bromodifluoromethyl group, a 2-fluoroethyl group, a 2,2-difluoroethyl group, a 2,2,2-trifluoroethyl group, and a 6,6,6-trifluorohexyl group.
Examples of the C1-C6 haloalkoxy group in the group [a-1] include a trifluoromethoxy group, a difluoromethoxy group, a bromodifluoromethoxy group, a chlorodifluoromethoxy group, a fluoromethoxy group, a 2,2,2-trifluoroethoxy group, a 1,1,2,2-tetrafluoroethoxy group, a 5-chloropentyloxy group, a 4-fluoroisopentyloxy group, and a 2,2-dichlorohexyloxy group.
Examples of the C1-C3 alkylthio group in the group [a-1] include a methylthio group, an ethylthio group, a 1-methylethylthio group, and a propylthio group.
Examples of the C1-C3 alkylidene group which forms a double bond with a ring-forming carbon atom in the group [a-1] include methylene which forms a double bond with a ring-forming carbon atom, ethylidene which forms a double bond with a ring-forming carbon atom, isopropylidene which forms a double bond with a ring-forming carbon atom, and propylidene which forms a double bond with a ring-forming carbon atom.
Examples of the C1-C6 hydroxyalkyl group in the group [a-1] include a hydroxymethyl group, a 1-hydroxyethyl group, a 2-hydroxyethyl group, a 1-hydroxypropyl group, and a 2-hydroxypropyl group.
Examples of the C2-C4 alkylcarbonyloxy group in the group [a-1] include an acetoxy group, an ethylcarbonyloxy group, a 1-methylethylcarbonyloxy group, and a propylcarbonyloxy group.
Examples of the (C1-C3 alkylamino)C1-C6 alkyl group in the group [a-1] include an N-methylaminomethyl group, an N-ethylaminomethyl group, a 1-(N-methylamino)ethyl group, a 2-(N-methylamino)ethyl group, and a 1-(N-ethylamino)ethyl group.
Examples of the (di(C1-C3 alkyl)amino)C1-C6 alkyl group in the group [a-1] include an N,N-dimethylaminomethyl group, a 1-(N,N-dimethylamino)ethyl group, a 2-(N,N-dimethylamino)ethyl group, and an N,N-diethylaminomethyl group.
Examples of the C2-C6 cyanoalkyl group in the group [a-1] include a cyanomethyl group, a 1-cyanoethyl group, and a 2-cyanoethyl group.
Examples of the C1-C3 alkylsulfonyl group in the group [a-1] include a methanesulfonyl group, and an ethanesulfonyl group.

R¹³ and R¹⁴ independently represent a hydrogen atom, a C1-C4 alkyl group, a C2-C5 alkylcarbonyl group, a C2-C5 alkoxycarbonyl group or a C1-C4 alkylsulfonyl group.
Examples of the C1-C4 alkyl group represented by R¹³ include a methyl group, an ethyl group, a 1-methylethyl group, a 1,1-dimethylethyl group, a propyl group, and a 1-methylpropyl group.
Examples of the C2-C5 alkylcarbonyl group represented by R¹³ include an acetyl group, an ethylcarbonyl group, a 1-methylethylcarbonyl group, and a 1,1-dimethylethylcarbonyl group.
Examples of the C2-C5 alkoxycarbonyl group represented by R¹³ include a methoxycarbonyl group, an ethoxycarbonyl group, a 1-methylethoxycarbonyl group, and a 1,1-dimethylethoxycarbonyl group.
Examples of the C1-C4 alkylsulfonyl group represented by R¹³ include a methylsulfonyl group, an ethylsulfonyl group, a 1-methylethylsulfonyl group, and a 1,1-dimethylethylsulfonyl group.

Examples of the C1-C4 alkyl group represented by R¹⁴ include a methyl group, an ethyl group, a 1-methylethyl group, a 1,1-dimethylethyl group, a propyl group, and a 1-methylpropyl group.
Examples of the C2-C5 alkylcarbonyl group represented by R¹⁴ include an acetyl group, an ethylcarbonyl group, a 1-methylethylcarbonyl group, and a 1,1-dimethylethylcarbonyl group.
Examples of the C2-C5 alkoxycarbonyl group represented by R¹⁴ include a methoxycarbonyl group, an ethoxycarbonyl group, a 1-methylethoxycarbonyl group, and a 1,1-dimethylethoxycarbonyl group.
Examples of the C1-C4 alkylsulfonyl group represented by R¹⁴ include a methylsulfonyl group, an ethylsulfonyl group, a 1-methylethylsulfonyl group, and a 1,1-dimethylethylsulfonyl group.
Examples of the NR¹³R¹⁴ group include an amino group, a methylamino group, a dimethylamino group, an ethylamino group, an acetylamino group, a propionylamino group, a 1,1-dimethylethylcarbonylamino group, a methoxycarbonylamino group, an ethoxycarbonylamino group, a 1,1-dimethylethoxycarbonylamino group, a methanesulfonylamino group, an N-acetyl-N-methylamino group, an N-ethoxycarbonyl-N-methylamino group, and a methanesulfonylmethylamino group.

Examples of the C3-C6 cycloalkyl group in the C3-C6 cycloalkyl group optionally substituted with a group of the group [a-1] include a cyclopropyl group, a cyclobutyl group, a cyclopentyl group, and a cyclohexyl group.
Examples of the C3-C6 cycloalkenyl group in the C3-C6 cycloalkyl group optionally substituted with a group of the group [a-1] include a 2-cyclopropenyl group, a 1-cyclobutenyl group, a 2-cyclobutenyl group, a 1-cyclopentenyl group, a 2-cyclopentenyl group, a 3-cyclopentenyl group, a 1-cyclohexenyl group, a 2-cyclohexenyl group, and a 3-cyclohexenyl group.
Examples of the substituted cycloalkyl group include a 2-oxocyclopropyl group, a 2-oxocyclobutyl group, a 3-oxocyclobutyl group, a 2-oxocyclopentyl group, 3-oxocyclopentyl group, a 2-oxocyclohexyl group, a 3-oxocyclohexyl group, and a 4-oxocyclohexyl group.
Examples of the C3-C6 hydroxyiminocycloalkyl group in the C3-C6 hydroxyiminocycloalkyl group optionally substituted with a group of the group [a-1] include a 2-hydroxyiminocyclopropyl group, a 2-hydroxyiminocyclobutyl group, a 3-hydroxyiminocyclobutyl group, a 2-hydroxyiminocyclopentyl group, a 3-hydroxyiminocyclopentyl group, a 2-hydroxyiminocyclohexyl group, a 3-hydroxyiminocyclohexyl group, and a 4-hydroxyiminocyclohexyl group.

Examples of the A¹-Cy¹ group include the following groups:
a cyclopropyl group, a 2,2,3,3-tetramethylcyclopropyl group;
a cyclobutyl group;
a cyclopentyl group, a 2-methylcyclopentyl group, a 2-hydroxycyclopentyl group, a 2-chlorocyclopentyl group, a 2-bromocyclopentyl group, a 2,2-dimethylcyclopentyl group, a 2-fluoro-2-methylcyclopentyl group, a 2-chloro-2-methylcyclopentyl group, a 2-hydroxy-2-methylcyclopentyl group, a 2,2-difluorocyclopentyl group, and a 3-methylcyclopentyl group;
a cyclohexyl group, a 1-methylcyclohexyl group, a 2-methylcyclohexyl group, a 3-methylcyclohexyl group, a 4-methylcyclohexyl group, a 2-trifluoromethylcyclohexyl group, a 2,2-dimethylcyclohexyl group, a 2-fluoro-2-methylcyclohexyl group, a 2-chloro-2-methylcyclohexyl group, a 2-hydroxy-2-methylcyclohexyl group, a 2,2-difluorocyclohexyl group, a 2,3-dimethylcyclohexyl group, a 2,6-dimethylcyclohexyl group, a 2-ethylcyclohexyl group, a 2-chlorocyclohexyl group, a 2-fluorocyclohexyl group, a 2-bromocyclohexyl group, a 2-iodocyclohexyl group, a 2-hydroxycyclohexyl group, a 1-cyanocyclohexyl group, a 2-cyanocyclohexyl group, a 1-carboxycyclohexyl group, a 1-(methoxycarbonyl)cyclohexyl group, a 1-(ethoxycarbonyl)cyclohexyl group, a 2-(methoxycarbonyl)cyclohexyl group, a 2-(ethoxycarbonyl)cyclohexyl group;

a cyclopropylmethyl group, a (1-methylcyclopropyl)methyl group, a (2-methylcyclopropyl)methyl group, a (1-hydroxycyclopropyl)methyl group, a (2-hydroxycyclopropyl)methyl group, a (2,2,3,3-tetramethylcyclopropyl)methyl group, a (1-fluorocyclopropyl)methyl group, a (1-chlorocyclopropyl)methyl group, a (1-cyanocyclopropyl)methyl group, a (1-ethoxycarbonylcyclopropyl)methyl group, a (1-methoxycarbonylcyclopropyl)methyl group;
a cyclobutylmethyl group, a (1-methylcyclobutyl)methyl group, a (2-methylcyclobutyl)methyl group, a (3-methylcyclobutyl)methyl group, a (1-hydroxycyclobutyl)methyl group, a (2-hydroxycyclobutyl)methyl group, a (3-hydroxycyclobutyl)methyl group, a (1-fluorocyclobutyl)methyl group, a (1-chlorocyclobutyl)methyl group, (1-cyanocyclobutyl)methyl group, a (1-ethoxycarbonylcyclobutyl)methyl group, a (1-methoxycarbonylcyclobutyl)methyl group;
a cyclopentylmethyl group, a (1-methylcyclopentyl)methyl group, a (2-methylcyclopentyl)methyl group, a (3-methylcyclopentyl)methyl group, a (1-hydroxycyclopentyl)methyl group, a (2-hydroxycyclopentyl)methyl group, a (3-hydroxycyclopentyl)methyl group, a (1-fluorocyclopentyl)methyl group, a (1-chlorocyclopentyl)methyl group, a (1-cyanocyclopentyl)methyl group, a (1-ethoxycarbonylcyclopentyl)methyl group, a (1-methoxycarbonylcyclopentyl)methyl group;
a cyclohexylmethyl group, a (1-methylcyclohexyl)methyl group, a (2-methylcyclohexyl)methyl group, a (3-methylcyclohexyl)methyl group, a (4-methylcyclohexyl)methyl group, a (2,3-dimethylcyclohexyl)methyl group, a (1-hydroxycyclohexyl)methyl group, a (2-hydroxycyclohexyl)methyl group, a (3-hydroxycyclohexyl)methyl group, a (4-hydroxycyclohexyl)methyl group, a (1-fluorocyclohexyl)methyl group, a (1-chlorocyclohexyl)methyl group, a (1-cyanocyclohexyl)methyl group, a (1-ethoxycarbonylcyclohexyl)methyl group, a (1-methoxycarbonylcyclohexyl)methyl group, a (1-ethynylcyclohexyl)methyl group, a (2-fluorocyclohexyl)methyl group, a (2-chlorocyclohexyl)methyl group, a (2-cyanocyclohexyl)methyl group, a (2-ethoxycarbonylcyclohexyl)methyl group, a (2-methoxycarbonylcyclohexyl)methyl group;

a 1-(cyclopropyl)ethyl group, a 1-(1-methylcyclopropyl)ethyl group, a 1-(2-methylcyclopropyl)ethyl group, a 1-(1-hydroxycyclopropyl)ethyl group, a 1-(2-hydroxycyclopropyl)ethyl group, a 1-(2,2,3,3-tetramethylcyclopropyl)ethyl group, a 1-(1-fluorocyclopropyl)ethyl group, a 1-(1-chlorocyclopropyl)ethyl group, a 1-(1-cyanocyclopropyl)ethyl group, a 1-(1-ethoxycarbonylcyclopropyl)ethyl group, a 1-(1-methoxycarbonylcyclopropyl)ethyl group;
a 1-(cyclobutyl)ethyl group, a 1-(1-methylcyclobutyl)ethyl group, a 1-(2-methylcyclobutyl)ethyl group, a 1-(3-methylcyclobutyl)ethyl group, a 1-(1-hydroxycyclobutyl)ethyl group, a 1-(2-hydroxycyclobutyl)ethyl group, a 1-(3-hydroxycyolobutyl)ethyl group, a 1-(1-fluorocyclobutyl)ethyl group, a 1-(1-chlorocyclobutyl)ethyl group, a 1-(1-cyanocyclobutyl)ethyl group, a 1-(1-ethoxycarbonylcyclobutyl)ethyl group, a 1-(1-methoxycarbonylcyclobutyl)ethyl group;
a 1-(cyclopentyl)ethyl group, a 1-(1-methylcyclopentyl)ethyl group, a 1-(2-methylcyclopentyl)ethyl group, a 1-(3-methylcyclopentyl)ethyl group, a 1-(1-hydroxycyclopentyl)ethyl group, a 1-(2-hydroxycyclopentyl)ethyl group, a 1-(3-hydroxycyclopentyl)ethyl group, a 1-(1-fluorocyclopentyl)ethyl group, a 1-(1-chlorocyclopentyl)ethyl group, a 1-(1-cyanocyclopentyl)ethyl group, a 1-(1-ethoxycarbonylcyclopentyl)ethyl group, a 1-(1-methoxycarbonylcyclopentyl)ethyl group;
a 1-(cyclohexyl)ethyl group, a 1-(1-methylcyclohexyl)ethyl group, a 1-(2-methylcyclohexyl)ethyl group, a 1-(3-methylcyclohexyl)ethyl group, a 1-(4-methylcyclohexyl)ethyl group, a 1-(2,3-dimethylcyclohexyl)ethyl group, a 1-(1-hydroxycyclohexyl)ethyl group, a 1-(2-hydroxycyclohexyl)ethyl group, a 1-(3-hydroxycyclohexyl)ethyl group, a 1-(4-hydroxycyclohexyl)ethyl group, a 1-(1-fluorocyclohexyl)ethyl group, a 1-(1-chlorocyclohexyl)ethyl group, a 1-(1-cyanocyclohexyl)ethyl group, a 1-(1-ethoxycarbonylcyclohexyl)ethyl group, a 1-(1-methoxycarbonylcyclohexyl)ethyl group, a 1-(1-ethynylcyclohexyl)ethyl group, a 1-(2-fluorocyclohexyl)ethyl group, a 1-(2-chlorocyclohexyl)ethyl group, a 1-(2-cyanocyclohexyl)ethyl group, a 1-(2-ethoxycarbonylcyclohexyl)ethyl group, a 1-(2-methoxycarbonylcyclohexyl)ethyl group;

a 1-methyl-1-cyclopropylethyl group, a 1-methyl-1-cyclobutylethyl group, a 1-methyl-1-(1-hydroxycyclobutyl)ethyl group, a 1-methyl-1-(1-fluorocyclobutyl)ethyl group, a 1-methyl-1-cyclopentylethyl group, a 1-methyl-1-(1-hydroxycyclopentyl)ethyl group, a 1-methyl-1-(1-fluorocyclopentyl)ethyl group, a 1-methyl-1-cyclohexylethyl group, a 1-methyl-1-(1-hydroxycyclohexyl)ethyl group, a 1-methyl-1-(1-fluorocyclohexyl)ethyl group,
a (1-hydroxymethylcyclopropyl)methyl group, a (1-hydroxymethylcyclobutyl)methyl group, a (1-hydroxymethylcyclopentyl)methyl group, a (1-hydroxymethylcyclohexyl)methyl group, a (1-cyclohexenyl)methyl group, a (2-cyclohexenyl)methyl group, a (3-cyclohexenyl)methyl group, a (1-cyclopentenyl)methyl group, a (2-cyclopentenyl)methyl group, a (1-dimethylaminocyclopropyl)methyl group, a (1-dimethylaminocyclobutyl)methyl group, a (1-dimethylaminocyclopentyl)methyl group, a (1-dimethylaminocyclohexyl)methyl group,
a (1-acetoxycyclopropyl)methyl group, a (1-acetoxycyclobutyl)methyl group, a (1-acetoxycyclopentyl)methyl group, a (1-acetoxycyclohexyl)methyl group, a (2-acetoxycyclohexyl)methyl group, a 2-cyclohexenyl group, a 3-cyclohexenyl group, a 2-methoxycyclopropyl group, a 2-methoxycyclobutyl group, a 2-methoxycyclopentyl group, a 2-methoxycyclohexyl group, a 3-methoxycyclohexyl group, a 4-methoxycyclohexyl group, a (1-methoxycyclohexyl)methyl group, a (2-methoxycyclohexyl)methyl group, a 2-acetoxycyclobutyl group, a 2-acetoxycyclopentyl group, a 2-acetoxycyclohexyl group, a 2-methylthiocyclobutyl group, a 2-methylthiocyclopentyl group, a 2-methylthiocyclohexyl group, a 2-(1,1-dimethylethoxycarbonylamino)cyclobutyl group, a 2-(1,1-dimethylethoxycarbonylamino)cyclopentyl group, a 2-(1,1-dimethylethoxycarbonylamino)cyclohexyl group, a 2-aminocyclobutyl group, a 2-aminocyclopentyl group, a 2-aminocyclohexyl group, a 2-acetylaminocyclobutyl group, a 2-acetylaminocyclopentyl group, a 2-acetylaminocyclohexyl group, a 2-dimethylaminocyclobutyl group, a 2-dimethylaminocyclopentyl group, a 2-dimethylaminocyclohexyl group, a 2-phenylcyclobutyl group, 2-phenylcyclopentyl group, a 2-phenylcyclohexyl group, a 2-benzylcyclobutyl group, a 2-benzylcyclopentyl group, a 2-benzylcyclohexyl group, a 2-trifluoromethylcyclobutyl group, a 2-trifluoromethylcyclopentyl group, a 2-trifluoromethylcyclohexyl group, a 2-hydroxymethylcyclobutyl group, a 2-hydroxymethylcyclopentyl group, 2-hydroxymethylcyclohexyl group, a 2-methylenecyclohexyl group, a 3-methylenecyclohexyl group, a 4-methylenecyclohexyl group, a 2-methylenecyclopentyl group, a 3-methylenecyclopentyl group, a 2-oxocyclohexyl group, a 3-oxocyclohexyl group, a 4-oxocyclohexyl group, a 2-oxocyclopentyl group, a 3-oxocyclopentyl group, a 2-hydroxyiminocyclohexyl group, and a 2-hydroxyiminocyclopentyl group.

A² represents methylene, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group (hereinafter, this methylene may be referred to as a "substituted methylene a²").
Examples of the C1-C3 haloalkyl group in the substituted methylene a² include a fluoromethyl group, a chloromethyl group, a bromomethyl group, a difluoromethyl group, a dichloromethyl group, a dibromomethyl group, a trifluoromethyl group, a trichloromethyl group, a dichlorofluoromethyl group, a chlorodifluoromethyl group, a 1,1-difluoroethyl group, a 2,2,2-trifluoroethyl group, a 2-fluoroethyl group, a 3-fluoropropyl group, and a 1-chloroethyl group.
Examples of the C2-C4 alkenyl group in the substituted methylene a² include a vinyl group, a 1-propenyl group, a 2-propenyl group, a 2-butenyl group, and a 3-butenyl group.
Examples of the C2-C4 alkynyl group in the substituted methylene a² include an ethynyl group, a 1-propynyl group, a 2-propynyl group, and a 3-butynyl group.
Examples of the C2-C5 alkoxycarbonyl group for the substituted methylene a² include a methoxycarbonyl group, an ethoxycarbonyl group, a 1-methylethoxycarbonyl group, and a 1,1-dimethylethoxycarbonyl group.
Specific examples of the substituted methylene a² include a 2,2,2-trifluoroethane-1,1-diyl group, a 2,2-difluoroethane-1,1-diyl group, a 2-fluoroethane-1,1-diyl group, a 3,3,3-trifluoropropane-1,1-diyl group, a 2,2,2-trichloroethane-1,1-diyl group, a 2,2-dichloroethanediyl group, a 2-chloroethane-1,1-diyl group, a 2-propene-1,1-diyl group, a 2-butene-1,1-diyl group, a 3-methyl-2-butene-1,1-diyl group, a 2-propyne-1,1-diyl group, a 2-butyne-1,1-diyl group, a 3-butyne-1,1-diyl group, a cyanomethylene group, a (methoxycarbonyl)methylene group, an (ethoxycarbonyl)methylene group, and a (1-methylethoxycarbonyl)methyl group.

R⁸ and R⁹ of A²-CR⁸R⁹R¹⁰ independently represent a C1-C4 alkyl group.
Examples of the C1-C4 alkyl group represented by R⁸ include a methyl group, an ethyl group, a 1-methylethyl group, a 1,1-dimethylethyl group, a propyl group, and a 1-methylpropyl group.
Examples of the C1-C4 alkyl group represented by R⁹ include a methyl group, an ethyl group, a 1-methylethyl group, a 1,1-dimethylethyl group, a propyl group, and a 1-methylpropyl group.
R¹⁰ represents a hydrogen atom, a C1-C4 alkyl group, a C2-C4 alkenyl group, a C2-C4 alkynyl group, a cyano group, a carboxyl group, a C2-C5 alkoxycarbonyl group, a halogen atom, a hydroxyl group, a C1-C6 alkoxy group, a C3-C6 alkenyloxy group, a C1-C6 haloalkyl group, a C2-C6 haloalkoxy group, a C1-C3 alkylthio group, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a C2-C6 cyanoalkyl group, a C1-C3 alkylsulfonyl group or an NR¹¹R¹² group.

Examples of the C1-C4 alkyl group represented by R¹⁰ include a methyl group, an ethyl group, a 1-methylethyl group, a 1,1-dimethylethyl group, a propyl group, and a 1-methylpropyl group.
Examples of the C2-C4 alkenyl group represented by R¹⁰ include a vinyl group, a 1-propenyl group, a 2-propenyl group, a 2-butenyl group, and a 3-butenyl group.
Examples of the C2-C4 alkynyl group represented by R¹⁰ include an ethynyl group, a 1-propynyl group, a 2-propynyl group, and a 3-butynyl group.
Examples of the C2-C5 alkoxycarbonyl group represented by R¹⁰ include a methoxycarbonyl group, an ethoxycarbonyl group, a 1-methylethoxycarbonyl group, and a 1,1-dimethylethoxycarbonyl group.
The halogen atom represented by R¹⁰ includes a fluorine atom, a chlorine atom, a bromine atom and an iodine atom.
Examples of the C1-C6 alkoxy group represented by R¹⁰ include a methoxy group, an ethoxy group, a propoxy group, an isopropoxy group, a butoxy group, an isobutoxy group, a sec-butoxy group, a tert-butoxy group, a pentyloxy group, an isopentyloxy group, and a hexyloxy group.
Examples of the C3-C6 alkenyloxy group represented by R¹⁰ include a 2-propenyloxy group, a 1-methyl-2-propenyloxy group, a 2-methyl-2-propenyloxy group, a 2-butenyloxy group, a 3-butenyloxy group, a 2-hexenyloxy group, and a 5-hexenyloxy group.
Examples of the C1-C6 haloalkyl group represented by R¹⁰ include a fluoromethyl group, a difluoromethyl group, a trifluoromethyl group, a trichloromethyl group, a chlorofluoromethyl group, a bromodifluoromethyl group, a 2-fluoroethyl group, a 2,2-difluoroethyl group, a 2,2,2-trifluoroethyl group, and a 6, 6, 6-trifluorohexyl group.
Examples of the C2-C6 haloalkoxy group represented by R¹⁰ include a trifluoromethoxy group, a difluoromethoxy group, a bromodifluoromethoxy group, a chlorodifluoromethoxy group, a fluoromethoxy group, a 2,2,2-trifluoroethoxy group, a 1,1,2,2-tetrafluoroethoxy group, a 5-chloropentyloxy group, a 4-fluoroisopentyloxy group, and a 2,2-dichlorohexyloxy group.
Examples of the C1-C3 alkylthio group represented by R¹⁰ include a methylthio group, an ethylthio group, a 1-methylethylthio group, and a propylthio group.
Examples of the C1-C6 hydroxyalkyl group represented by R¹⁰ include a hydroxymethyl group, a 1-hydroxyethyl group, a 2-hydroxyethyl group, a 1-hydroxypropyl group, and a 2-hydroxypropyl group.
Examples of the C2-C4 alkylcarbonyloxy group represented by R¹⁰ include an acetoxy group, an ethylcarbonyloxy group, a 1-methylethylcarbonyloxy group, and a propylcarbonyloxy group.
Examples of the (C1-C3 alkylamino)C1-C6 alkyl group represented by R¹⁰ include an N-methylaminomethyl group, an N-ethylaminomethyl group, a 1-(N-methylamino)ethyl group, a 2-(N-methylamino)ethyl group, and a 1-(N-ethylamino)ethyl group.
Examples of the (di(C1-C3 alkyl)amino)C1-C6 alkyl group represented by R¹⁰ include an N,N-dimethylaminomethyl group, a 1-(N,N-dimethylamino)ethyl group, a 2-(N,N-dimethylamino)ethyl group, and an N,N-diethylaminomethyl group.
Examples of the C2-C6 cyanoalkyl group represented by R¹⁰ include a cyanomethyl group, a 1-cyanoethyl group, and a 2-cyanoethyl group.
Examples of the C1-C3 alkylsulfonyl group represented by R¹⁰ include a methanesulfonyl group, and an ethanesulfonyl group.

R¹¹ and R¹² independently represent a hydrogen atom, a C1-C4 alkyl group, a C2-C5 alkylcarbonyl group, a C2-C5 alkoxycarbonyl group or a C1-C4 alkylsulfonyl group.
Examples of the C1-C4 alkyl group represented by R¹¹ include a methyl group, an ethyl group, a 1-methylethyl group, a 1,1-dimethylethyl group, a propyl group, and a 1-methylpropyl group.
Examples of the C2-C5 alkylcarbonyl group represented by R¹¹ include an acetyl group, an ethylcarbonyl group, a 1-methylethylcarbonyl group, and a 1,1-dimethylethylcarbonyl group.
Examples of the C2-C5 alkoxycarbonyl group represented by R¹¹ include a methoxycarbonyl group, an ethoxycarbonyl group, a 1-methylethoxycarbonyl group, and a 1,1-dimethylethoxycarbonyl group.
Examples of the C1-C4 alkylsulfonyl group represented by R¹¹ include a methylsulfonyl group, an ethylsulfonyl group, a 1-methylethylsulfonyl group, and a 1,1-dimethylethylsulfonyl group.

Examples of the C1-C4 alkyl group represented by R¹² include a methyl group, an ethyl group, a 1-methylethyl group, a 1,1-dimethylethyl group, a propyl group, and a 1-methylpropyl group.
Examples of the C2-C5 alkylcarbonyl group represented by R¹² include an acetyl group, an ethylcarbonyl group, a 1-methylethylcarbonyl group, and a 1,1-dimethylethylcarbonyl group.
Examples of the C2-C5 alkoxycarbonyl group represented by R¹² include a methoxycarbonyl group, an ethoxycarbonyl group, a 1-methylethoxycarbonyl group, and a 1,1-dimethylethoxycarbonyl group.
Examples of the C1-C4 alkylsulfonyl group represented by R¹² include a methylsulfonyl group, an ethylsulfonyl group, a 1-methylethylsulfonyl group, and a 1,1-dimethylethylsulfonyl group.
Examples of the NR¹¹R¹² group include an amino group, a methylamino group, a dimethylamino group, an ethylamino group, an acetylamino group, a propionylamino group, a 1,1-dimethylethylcarbonylamino group, a methoxycarbonylamino group, an ethoxycarbonylamino group, a 1,1-dimethylethoxycarbonylamino group, a methanesulfonylamino group, an N-acetyl-N-methylamino group, an N-ethoxycarbonyl-N-methylamino group, and a methanesulfonylmethylamino group.

Examples of the A²-CR⁸R⁹R¹⁰ group include a 2-methylpropyl group, a 1,2-dimethylpropyl group, a 2,2-dimethylpropyl group, a 1,2,2-trimethylpropyl group, a 1,1,2,2-tetramethylpropyl group, a 2-methyl-2-hydroxypropyl group, a 1,2-dimethyl-2-hydroxypropyl group, a 2-chloro-1,2-dimethylpropyl group, a 2-chloro-2-methylpropyl group, a 1,2-dimethylbutyl group, a 2,2-dimethyl-3-hydroxypropyl group, and a 3-hydroxy-1,2,2-trimethylpropyl group.

Examples of the amide compound represented by formula (1) include the following aspects:
an amide compound of the formula (1), wherein Z¹ is an oxygen atom;
an amide compound of the formula (1), wherein X¹ is a fluorine atom, a C1-C4 alkoxy group or an NR¹R² group (R¹ and R² are as defined above);
an amide compound of the formula (1), wherein X¹ is a fluorine atom, a methoxy group or an amino group;
an amide compound of the formula (1), wherein X¹ is a fluorine atom;
an amide compound of the formula (1), wherein X¹ is a methoxy group;
an amide compound of the formula (1), wherein X¹ is an amino group;
an amide compound of the formula (1), wherein X¹ is a fluorine atom, a C1-C4 alkoxy group or an NR¹R² group (R¹ and R² are as defined above), and Z is an oxygen atom;
an amide compound of the formula (1), wherein X¹ is a fluorine atom, a methoxy group or an amino group, and Z is an oxygen atom;
an amide compound of the formula (1), wherein X¹ is a fluorine atom and Z¹ is an oxygen atom;
an amide compound of the formula (1), wherein X¹ is a methoxy group and Z¹ is an oxygen atom;
an amide compound of the formula (1), wherein X¹ is an amino group and Z¹ is an oxygen atom;

an amide compound of the formula (1), wherein X² is a hydrogen atom, a fluorine atom, a C1-C4 alkoxy group or an NR³R⁴ group (R³ and R⁴ are as defined above);
an amide compound of the formula (1), wherein X² is a hydrogen atom;
an amide compound of the formula (1), wherein X² is a fluorine atom;

an amide compound of the formula (1), wherein X¹ is a fluorine atom, a C1-C4 alkoxy group or an NR¹R² group (R¹ and R² are as defined above), and X² is a hydrogen atom, a fluorine atom, a C1-C4 alkoxy group or an NR³R⁴ group (R³ and R⁴ are as defined above);
an amide compound of the formula (1), wherein X¹ is a fluorine atom, a methoxy group or an amino group, and X² is a hydrogen atom, a fluorine atom, a C1-C4 alkoxy group or an NR³R⁴ group (R³ and R⁴ are as defined above);
an amide compound of the formula (1), wherein X¹ is a fluorine atom, and X² is a hydrogen atom, a fluorine atom, a C1-C4 alkoxy group or an NR³R⁴ group (R³ and R⁴ are as defined above);
an amide compound of the formula (1), wherein X¹ is a methoxy group, and X² is a hydrogen atom, a fluorine atom, a C1-C4 alkoxy group or an NR³R⁴ group (R³ and R⁴ are as defined above);
an amide compound of the formula (1), wherein X¹ is an amino group, and X² is a hydrogen atom, a fluorine atom, a C1-C4 alkoxy group or an NR³R⁴ group (R³ and R⁴ are as defined above);
an amide compound of the formula (1), wherein X¹ is a fluorine atom, a C1-C4 alkoxy group or an NR¹R² group (R¹ and R² are as defined above), and Z¹ is an oxygen atom;
an amide compound of the formula (1), wherein X¹ is a fluorine atom, a methoxy group or an amino group, X² is a hydrogen atom, a fluorine atom, a C1-C4 alkoxy group or an NR³R⁴ group (R³ and R⁴ are as defined above), and Z¹ is an oxygen atom;
an amide compound of the formula (1), wherein X¹ is a fluorine atom, X² is a hydrogen atom, a fluorine atom, a C1-C4 alkoxy group or an NR³R⁴ group (R³ and R⁴ are as defined above), and Z¹ is an oxygen atom;
an amide compound of the formula (1), wherein X¹ is a methoxy group, X² is a hydrogen atom, a fluorine atom, a C1-C4 alkoxy group or an NR³R⁴ group (R³ and R⁴ are as defined above), and Z¹ is an oxygen atom;
an amide compound of the formula (1), wherein X¹ is an amino group, X² is a hydrogen atom, a fluorine atom, a C1-C4 alkoxy group or an NR³R⁴ group (R³ and R⁴ are as defined above), and Z¹ is an oxygen atom;

an amide compound of the formula (1), wherein X¹ is a fluorine atom, a C1-C4 alkoxy group or an NR¹R² group (R¹ and R² are as defined above), and X² is a hydrogen atom;
an amide compound of the formula (1), wherein X¹ is a fluorine atom, a methoxy group or an amino group, and X² is a hydrogen atom;
an amide compound of the formula (1), wherein X¹ is a fluorine atom and X² is a hydrogen atom;
an amide compound of the formula (1), wherein X¹ is a methoxy group and X² is a hydrogen atom;
an amide compound of the formula (1), wherein X¹ is amino group and X² is a hydrogen atom;
an amide compound of the formula (1), wherein X¹ is a fluorine atom, a C1-C4 alkoxy group or an NR¹R² group (R¹ and R² are as defined above), X² is a hydrogen atom, and Z¹ is an oxygen atom;
an amide compound of the formula (1), wherein X¹ is a fluorine atom, a methoxy group or an amino group, X² is a hydrogen atom, and Z¹ is an oxygen atom;
an amide compound of the formula (1), wherein X¹ is a fluorine atom, X² is a hydrogen atom, and Z¹ is an oxygen atom;
an amide compound of the formula (1), wherein X¹ is a methoxy group, X² is a hydrogen atom, and Z¹ is an oxygen atom;
an amide compound of the formula (1), wherein X¹ is amino group, X² is a hydrogen atom, and Z¹ is an oxygen atom;

an amide compound of the formula (1), wherein X¹ is a fluorine atom, a C1-C4 alkoxy group or an NR¹R² group (R¹ and R² are as defined above), and X² is a fluorine atom;
an amide compound of the formula (1), wherein X¹ is a fluorine atom, a methoxy group or an amino group, and X² is a fluorine atom;
an amide compound of the formula (1), wherein X¹ is a fluorine atom and X² is a fluorine atom;
an amide compound of the formula (1), wherein X¹ is a methoxy group and X² is a fluorine atom;
an amide compound of the formula (1), wherein X¹ is an amino group and X² is a fluorine atom;
an amide compound of the formula (1), wherein X¹ is a fluorine atom, a C1-C4 alkoxy group or an NR¹R² group (R¹ and R² are as defined above), X² is a fluorine atom, and Z¹ is an oxygen atom;
an amide compound of the formula (1), wherein X¹ is a fluorine atom, a methoxy group or an amino group, X² is a fluorine atom, and Z¹ is an oxygen atom;
an amide compound of the formula (1), wherein X¹ is a fluorine atom, X² is a fluorine atom, and Z¹ is an oxygen atom;
an amide compound of the formula (1), wherein X¹ is a methoxy group, X² is a fluorine atom, and Z¹ is an oxygen atom;
an amide compound of the formula (1), wherein X¹ is an amino group, X² is a fluorine atom, and Z¹ is an oxygen atom;

an amide compound of the formula (1), wherein E¹ is an A¹ - Cy¹ group (A¹ and Cy¹ are as defined above);
an amide compound of the formula (1), wherein E¹ is an A¹ - Cy¹ group, A¹ is a single bond, methylene or -CH(CH₃)-, and Cy¹ is as defined above;
an amide compound of the formula (1), wherein E¹ is an A¹-Cy¹ group, A¹ is a single bond, and Cy¹ is as defined above;
an amide compound of the formula (1), wherein E¹ is an A¹ - Cy¹ group, A¹ is methylene, and Cy¹ is as defined above;
an amide compound of the formula (1), wherein E¹ is an A¹ - Cy¹ group, A¹ is -CH(CH₃)-, and Cy¹ is as defined above;
an amide compound of the formula (1), wherein E¹ is an A¹-Cy¹ group, A¹ is as defined above, and Cy¹ is a C3-C6 cycloalkyl group optionally substituted with at least one member selected from the group [a-1];
an amide compound of the formula (1), wherein E¹ is an A¹-Cy¹ group, A¹ is as defined above, and Cy¹ is a C3-C6 cycloalkyl group optionally substituted with at least one member selected from the group consisting of a halogen atom, a C1-C4 alkyl group, a C2-C4 alkenyl group, a C2-C4 alkynyl group, a hydroxyl group, a cyano group, a C1-C6 alkoxy group, a C1-C3 alkylidene group which forms a double bond with a ring-forming carbon atom, and a C1-C6 hydroxyalkyl group;
an amide compound of the formula (1), wherein E¹ is an A¹-Cy¹ group, A¹ is as defined above, and Cy¹ is a C3-C6 cycloalkyl group optionally substituted with at least one member selected from the group consisting of a halogen atom, a C1-C4 alkyl group, a hydroxyl group and a cyano group;
an amide compound of the formula (1), wherein E¹ is a 2-methylcyclopentyl group, a 2-fluorocyclopentyl group, a 2-chlorocyclopentyl group, a 2-hydroxycyclopentyl group, a 2-methylcyclohexyl group, a 2-fluorocyclohexyl group, a 2-chlorocyclohexyl group, a 2-hydroxycyclohexyl group, a cyclobutylmethyl group, a cyclopentylmethyl group, a cyclohexylmethyl group, a (1-hydroxycyclobutyl)methyl group, a (1-hydroxycyclopentyl)methyl group, a (1-hydroxycyclohexyl)methyl group, a 1-cyclobutylethyl group, a 1-cyclopentylethyl group, a 1-cyclohexylethyl group, a 1-(1-hydroxycyclobutyl)ethyl group, a 1-(1-hydroxycyclopentyl)ethyl group or a 1-(1-hydroxycyclohexyl)ethyl group;
an amide compound of the formula (1), wherein E¹ is a 2-methylcyclopentyl group, a 2-fluorocyclopentyl group, a 2-chlorocyclopentyl group, a 2-hydroxycyclopentyl group, a 2-methylcyclohexyl group, a 2-fluorocyclohexyl group, a 2-chlorocyclohexyl group or a 2-hydroxycyclohexyl group;
an amide compound of the formula (1), wherein E¹ is a cyclobutylmethyl group, a cyclopentylmethyl group, a cyclohexylmethyl group, a (1-hydroxycyclobutyl)methyl group, a (1-hydroxycyclopentyl)methyl group, a (1-hydroxycyclohexyl)methyl group, a 1-cyclobutylethyl group, a 1-cyolopentylethyl group, a 1-cyclohexylethyl group, a 1-(1-hydroxycyclobutyl)ethyl group, a 1-(1-hydroxycyclopentyl)ethyl group or a 1-(1-hydroxycyclohexyl)ethyl group;

an amide compound of the formula (1), wherein E¹ is a 2-methylcyclohexyl group;
an amide compound of the formula (1), wherein E¹ is a 2-chlorocyclohexyl group;
an amide compound of the formula (1), wherein E¹ is a cyclohexylmethyl group;
an amide compound of the formula (1), wherein E¹ is a 1-cyclohexylethyl group;
an amide compound of the formula (1), wherein E¹ is a (1-hydroxycyclohexyl)methyl group;
an amide compound of the formula (1), wherein E¹ is a 1-(1-hydroxycyclohexyl)ethyl group;
an amide compound of the formula (1), wherein E¹ is a cyclobutylmethyl group;
an amide compound of the formula (1), wherein E¹ is a 1-cyclobutylethyl group;
an amide compound of the formula (1), wherein E¹ is a (1-hydroxycyclobutyl)methyl group;
an amide compound of the formula (1), wherein E¹ is a 1-(1-hydroxycyclobutyl)ethyl group;

an amide compound of the formula (1), wherein E¹ is an A²-CR⁸R⁹R¹⁰ group (A², R⁸, R⁹ and R¹⁰ are as defined above); an amide compound of the formula (1), wherein E¹ is an A²-CR⁸R⁹R¹⁰ group, A² is methylene, -CH(CH₃)- or -C(CH₃)₂-, and R⁸, R⁹ and R¹⁰ are as defined above;
an amide compound of the formula (1), wherein E¹ is an A²-CR⁸R⁹R¹⁰ group, A² is methylene, and R⁸, R⁹ and R¹⁰ are as defined above;
an amide compound of the formula (1), wherein E¹ is an A² - CR⁸R⁹R¹⁰ group, A² is -CH(CH₃)-, and R⁸, R⁹ and R¹⁰ are as defined above;
an amide compound of the formula (1), wherein E¹ is an A²-CR⁸R⁹R¹⁰ group, A² is -C(CH₃)₂-, and R⁸, R⁹ and R¹⁰ are as defined above;
an amide compound of the formula (1), wherein E¹ is an A²-CR⁸R⁹R¹⁰ group, A² is as defined above, R⁸ is a methyl group, R⁹ is a methyl group, and R¹⁰ is as defined above; an amide compound of the formula (1), wherein E¹ is an A² - CR⁸R⁹R¹⁰ group, A² is methylene, -CH(CH₃)- or -C(CH₃)₂-, R⁸ is a methyl group, R⁹ is a methyl group, and R¹⁰ is as defined above;
an amide compound of the formula (1), wherein E¹ is an A²-CR⁸R⁹R¹⁰ group, A² is methylene, R⁸ is a methyl group, R⁹ is a methyl group, and R¹⁰ is as defined above;
an amide compound of the formula (1), wherein E¹ is an A²-CR⁸R⁹R¹⁰ group, A² is -CH(CH₃)-, R⁸ is a methyl group, R⁹ is a methyl group, and R¹⁰ is as defined above;
an amide compound of the formula (1), wherein E¹ is an A² - CR⁸R⁹R¹⁰ group, A² is -C(CH₃)₂-, R⁸ is a methyl group, R⁹ is a methyl group, and R¹⁰ is as defined above;
an amide compound of the formula (1), wherein E¹ is a 2-methylpropyl group, a 1,2-dimethylpropyl group, a 2,2-dimethylpropyl group, a 1,2,2-trimethylpropyl group, a 2-methyl-2-hydroxypropyl group or a 1,2-dimethyl-2-hydroxypropyl group;

an amide compound of the formula (1), wherein E¹ is an A¹ - Cy¹ group (A¹ and Cy¹ are as defined above), X¹ is a fluorine atom, and Z¹ is an oxygen atom;
an amide compound of the formula (1), wherein E¹ is an A¹-Cy¹ group, A¹ is a single bond, methylene or -CH(CH₃)-, Cy¹ is as defined above, X¹ is a fluorine atom, and Z¹ is an oxygen atom;
an amide compound of the formula (1), wherein E¹ is an A¹ - Cy¹ group, A¹ is a single bond, Cy¹ is as defined above, X¹ is a fluorine atom, and Z¹ is an oxygen atom;
an amide compound of the formula (1), wherein E¹ is an A¹ - Cy¹ group, A¹ is methylene, Cy¹ is as defined above, X¹ is a fluorine atom, and Z¹ is an oxygen atom;
an amide compound of the formula (1), wherein E¹ is an A¹ - Cy¹ group, A¹ is -CH(CH₃)-, Cy¹ is as defined above, X¹ is a fluorine atom, and Z¹ is an oxygen atom;
an amide compound of the formula (1), wherein E¹ is an A¹ - Cy¹ group, A¹ is as defined above, Cy¹ is a C3-C6 cycloalkyl group optionally substituted with a group of the group [a-1], X¹ is a fluorine atom, and Z¹ is an oxygen atom;
an amide compound of the formula (1), wherein E¹ is an A¹ - Cy¹ group, A¹ is as defined above, Cy¹ is a C3-C6 cycloalkyl group optionally substituted with at least one member selected from the group consisting of a halogen atom, a C1-C4 alkyl group, a C2-C4 alkenyl group, a C2-C4 alkynyl group, a hydroxyl group, a cyano group, a C1-C6 alkoxy group, a C1-C3 alkylidene group which forms a double bond with a ring-forming carbon atom, and a C1-C6 hydroxyalkyl group, X¹ is a fluorine atom, and Z¹ is an oxygen atom;
an amide compound of the formula (1), wherein E¹ is an A¹-Cy¹ group, A¹ is as defined above, Cy¹ is a C3-C6 cycloalkyl group optionally substituted with at least one member selected from the group consisting of a halogen atom, a C1-C4 alkyl group, a hydroxyl group and a cyano group, X¹ is a fluorine atom, and Z¹ is an oxygen atom;

an amide compound of the formula (1), wherein E¹ is a 2-methylcyclopentyl group, a 2-fluorocyclopentyl group, a 2-chlorocyclopentyl group, a 2-hydroxycyclopentyl group, a 2-methylcyclohexyl group, a 2-fluorocyclohexyl group, a 2-chlorocyclohexyl group, a 2-hydroxycyclohexyl group, a cyclobutylmethyl group, a cyclopentylmethyl group, a cyclohexylmethyl group, a (1-hydroxycyclobutyl)methyl group, a (1-hydroxycyclopentyl)methyl group, a (1-hydroxycyclohexyl)methyl group, a 1-cyclobutylethyl group, a 1-cyclopentylethyl group, a 1-cyclohexylethyl group, a 1-(1-hydroxycyclobutyl)ethyl group, a 1-(1-hydroxycyclopentyl)ethyl group or a 1-(1-hydroxycyclohexyl)ethyl group, X¹ is a fluorine atom, and Z¹ is an oxygen atom;
an amide compound of the formula (1), wherein E¹ is a 2-methylcyclopentyl group, a 2-fluorocyclopentyl group, a 2-chlorocyclopentyl group, a 2-hydroxycyclopentyl group, a 2-methylcyclohexyl group, a 2-fluorocyclohexyl group, a 2-chlorocyclohexyl group or a 2-hydroxycyclohexyl group, X¹ is a fluorine atom, and Z¹ is an oxygen atom;
an amide compound of the formula (1), wherein E¹ is a cyclobutylmethyl group, a cyclopentylmethyl group, a cyclohexylmethyl group, a (1-hydroxycyclobutyl)methyl group, a (1-hydroxycyclopentyl)methyl group, a (1-hydroxycyclohexyl)methyl group, a 1-cyclobutylethyl group, a 1-cyclopentylethyl group, a 1-cyclohexylethyl group, a 1-(1-hydrcxycyclobutyl)ethyl group, a 1-(1-hydroxycyclopentyl)ethyl group or a 1-(1-hydroxycyclohexyl)ethyl group, X¹ is a fluorine atom, and Z¹ is an oxygen atom;

an amide compound of the formula (1), wherein E¹ is a 2-methylcyclohexyl group, X¹ is a fluorine atom, and Z¹ is an oxygen atom;
an amide compound of the formula (1), wherein E¹ is a 2-chlorocyclohexyl group, X¹ is a fluorine atom, and Z¹ is an oxygen atom;
an amide compound of the formula (1), wherein E¹ is a cyclohexylmethyl group, X¹ is a fluorine atom, and Z¹ is an oxygen atom;
an amide compound of the formula (1), wherein E¹ is a 1-cyclohexylethyl group, X¹ is a fluorine atom, and Z¹ is an oxygen atom;
an amide compound of the formula (1), wherein E¹ is a (1-hydroxycyclohexyl)methyl group, X¹ is a fluorine atom, and Z¹ is an oxygen atom;
an amide compound of the formula (1), wherein E¹ is a 1-(1-hydroxycyclohexyl)ethyl group, X¹ is a fluorine atom, and Z¹ is an oxygen atom;
an amide compound of the formula (1), wherein E¹ is a cyclobutylmethyl group, X¹ is a fluorine atom, and Z¹ is an oxygen atom;
an amide compound of the formula (1), wherein E¹ is a 1-cyclobutylethyl group, X¹ is a fluorine atom, and Z¹ is an oxygen atom;
an amide compound of the formula (1), wherein E¹ is a (1-hydroxycyclobutyl)methyl group, X¹ is a fluorine atom, and Z¹ is an oxygen atom;
an amide compound of the formula (1), wherein E¹ is a 1-(1-hydroxycyclobutyl)ethyl group, X¹ is a fluorine atom, and Z¹ is an oxygen atom;

an amide compound of the formula (1), wherein E¹ is an A² - CR⁸R⁹R¹⁰ group (A², R⁸, R⁹ and R¹⁰ are as defined above), X¹ is a fluorine atom, and Z¹ is an oxygen atom;
an amide compound of the formula (1), wherein E¹ is an A² - CR⁸R⁹R¹⁰ group, A² is methylene, -CH(CH₃)- or -C(CH₃)₂-, R⁸, R⁹ and R¹⁰ are as defined above, X¹ is a fluorine atom, and Z¹ is an oxygen atom;
an amide compound of the formula (1), wherein E¹ is an A²-CR⁸R⁹R¹⁰ group, A² is methylene, R⁸, R⁹ and R¹⁰ are as defined above, X¹ is a fluorine atom, and Z¹ is an oxygen atom;
an amide compound of the formula (1), wherein E¹ is an A²-CR⁸R⁹R¹⁰ group, A² is -CH(CH₃)-, R⁸, R⁹ and R¹⁰ are as defined above, X¹ is a fluorine atom, and Z¹ is an oxygen atom;
an amide compound of the formula (1), wherein E¹ is an A²-CR⁸R⁹R¹⁰ group, A² is -C(CH₃)₂-, R⁸, R⁹ and R¹⁰ are as defined above, X¹ is a fluorine atom, and Z¹ is an oxygen atom;
an amide compound of the formula (1), wherein E¹ is an A²-CR⁸R⁹R¹⁰ group, A² is as defined above, R⁸ is a methyl group, R⁹ is a methyl group, R¹⁰ is as defined above, X¹ is a fluorine atom, and Z¹ is an oxygen atom;
an amide compound of the formula (1), wherein E¹ is an A²-CR⁸R⁹R¹⁰ group, A² is methylene, -CH(CH₃)- or -C(CH₃)₂-, R⁸ is a methyl group, R⁹ is a methyl group, R¹⁰ is as defined above, X¹ is a fluorine atom, and Z¹ is an oxygen atom;
an amide compound of the formula (1), wherein E¹ is an A²-CR⁸R⁹R¹⁰ group, A² is methylene, R⁶ is a methyl group, R⁹ is a methyl group, R¹⁰ is as defined above, X¹ is a fluorine atom, and Z¹ is an oxygen atom;
an amide compound of the formula (1), wherein E¹ is an A²-CR⁸R⁹R¹⁰ group, A² is -CH(CH₃)-, R⁸ is a methyl group, R⁹ is a methyl group, R¹⁰ is as defined above, X¹ is a fluorine atom, and Z¹ is an oxygen atom;
an amide compound of the formula (1), wherein E¹ is an A² - CR⁸R⁹R¹⁰ group, A² is -C(CH₃)₂-, R⁸ is a methyl group, R⁹ is a methyl group, R¹⁰ is as defined above, X¹ is a fluorine atom, and Z¹ is an oxygen atom;
an amide compound of the formula (1), wherein E¹ is a 2-methylpropyl group, a 1,2-dimethylpropyl group, a 2,2-dimethylpropyl group, a 1,2,2-trimethylpropyl group, a 2-methyl-2-hydroxypropyl group or a 1,2-dimethyl-2-hydroxypropyl group, X¹ is a fluorine atom, and Z¹ is an oxygen atom;

an amide compound of the formula (1), wherein E¹ is an A¹ - Cy¹ group (A¹ and Cy¹ are as defined above), X¹ is a methoxy group, and Z¹ is an oxygen atom;
an amide compound of the formula (1), wherein E¹ is an A¹ - Cy¹ group, A¹ is a single bond, methylene or -CH(CH₃)-, Cy¹ is as defined above, X¹ is a methoxy group, and Z¹ is an oxygen atom;
an amide compound of the formula (1), wherein E¹ is an A¹ - Cy¹ group, A¹ is a single bond, Cy¹ is as defined above, X¹ is a methoxy group, and Z¹ is an oxygen atom;
an amide compound of the formula (1), wherein E¹ is an A¹ - Cy¹ group, A¹ is methylene, Cy¹ is as defined above, X¹ is a methoxy group, and Z¹ is an oxygen atom;
an amide compound of the formula (1), wherein E¹ is an A¹-Cy¹ group, A¹ is a single bond, methylene or -CH(CH₃)-, Cy¹ is as defined above, X¹ is a methoxy group, and Z¹ is an oxygen atom;
an amide compound of the formula (1), wherein E¹ is an A¹ - Cy¹ group, A¹ is as defined above, Cy¹ is a C3-C6 cycloalkyl group optionally substituted with a group of the group [a-1], X¹ is a methoxy group, and Z¹ is an oxygen atom;
an amide compound of the formula (1), wherein E¹ is an A¹ - Cy¹ group, A¹ is as defined above, Cy¹ is a C3-C6 cycloalkyl group optionally substituted with at least one member selected from the group consisting of a halogen atom, a C1-C4 alkyl group, a C2-C4 alkenyl group, a C2-C4 alkynyl group, a hydroxyl group, a cyano group, a C1-C6 alkoxy group, a C1-C3 alkylidene group which forms a double bond with a ring-forming carbon atom, and a C1-C6 hydroxyalkyl group, X¹ is a methoxy group, and Z¹ is an oxygen atom;
an amide compound of the formula (1), wherein E¹ is an A¹-Cy¹ group, A¹ is as defined above, Cy¹ is a C3-C6 cycloalkyl group optionally substituted with at least one member selected from the group consisting of a halogen atom, a C1-C4 alkyl group, a hydroxyl group and a cyano group, X¹ is a methoxy group, and Z¹ is an oxygen atom;

an amide compound of the formula (1), wherein E¹ is a 2-methylcyclopentyl group, a 2-fluorocyclopentyl group, a 2-chlorocyclopentyl group, a 2-hydroxycyclopentyl group, a 2-methylcyclohexyl group, a 2-fluorocyclohexyl group, a 2-chlorocyclohexyl group, a 2-hydroxycyclohexyl group, a cyclobutylmethyl group, a cyclopentylmethyl group, a cyclohexylmethyl group, a (l-hydroxycyclobutyl)methyl group, a (1-hydroxycyclopentyl)methyl group, a (1-hydroxycyclohexyl)methyl group, a 1-cyclobutylethyl group, a 1-cyclopentylethyl group, a 1-cyclohexylethyl group, a 1-(l-hydroxycyclobutyl)ethyl group, a 1-(1-hydroxycyclopentyl)ethyl group or a 1-(1-hydroxycyclohexyl)ethyl group, X¹ is a methoxy group, and Z¹ is an oxygen atom;
an amide compound of the formula (1), wherein E¹ is a 2-methylcyclopentyl group, a 2-fluorocyclopentyl group, a 2-chlorocyclopentyl group, a 2-hydroxycyclopentyl group, a 2-methylcyclohexyl group, a 2-fluorocyclohexyl group, a 2-chlorocyclohexyl group or a 2-hydroxycyclohexyl group, X¹ is methoxy group, and Z¹ is an oxygen atom;
an amide compound of the formula (1), wherein E¹ is a cyclobutylmethyl group, a cyclopentylmethyl group, a cyclohexylmethyl group, a (1-hydroxycyclobutyl)methyl group, a (1-hydroxycyclopentyl)methyl group, a (1-hydroxycyclohexyl)methyl group, a 1-cyclobutylethyl group, 1-cyclopentylethyl group, a 1-cyclohexylethyl group, a 1-(1-hydroxycyclobutyl)ethyl group, a 1-(1-hydroxycyclopentyl)ethyl group or a 1-(1-hydroxycyclohexyl)ethyl group, X¹ is a methoxy group, and Z¹ is an oxygen atom;

an amide compound of the formula (1), wherein E¹ is a 2-methylcyclohexyl group, X¹ is a methoxy group, and Z¹ is an oxygen atom; an amide compound of the formula (1), wherein E¹ is a 2-chlorocyclohexyl group, X¹ is a methoxy group, and Z¹ is an oxygen atom;
an amide compound of the formula (1), wherein E¹ is a cyclohexylmethyl group, X¹ is a methoxy group, and Z¹ is an oxygen atom;
an amide compound of the formula (1), wherein E¹ is a 1-cyclohexylethyl group, X¹ is a methoxy group, and Z¹ is an oxygen atom;
an amide compound of the formula (1), wherein E¹ is a (1-hydroxycyclohexyl)methyl group, X¹ is a methoxy group, and Z¹ is an oxygen atom;
an amide compound of the formula (1), wherein E¹ is a 1-(1-hydroxycyclohexyl)ethyl group, X¹ is a methoxy group, and Z¹ is an oxygen atom;
an amide compound of the formula (1), wherein E¹ is a cyclobutylmethyl group, X¹ is a methoxy group, and Z¹ is an oxygen atom;
an amide compound of the formula (1), wherein E¹ is a 1-cyclobutylethyl group, X¹ is a methoxy group, and Z¹ is an oxygen atom;
an amide compound of the formula (1), wherein E¹ is a (1-hydroxycyclobutyl)methyl group, X¹ is a methoxy group, and Z¹ is an oxygen atom;
an amide compound of the formula (1), wherein E¹ is a 1-(1-hydroxycyclobutyl)ethyl group, X¹ is a methoxy group, and Z¹ is an oxygen atom;

an amide compound of the formula (1), wherein E¹ is an A²-CR⁸R⁹R¹⁰ group (A², R⁸, R⁹ and R¹⁰ are as defined above), X¹ is a methoxy group, and Z¹ is an oxygen atom;
an amide compound of the formula (1), wherein E¹ is an A²-CR⁸R⁹R¹⁰ group, A² is methylene, -CH(CE₃)- or -C(CH₃)₂-, R⁸, R⁹ and R¹⁰ are as defined above, X¹ is a methoxy group, and Z¹ is an oxygen atom;
an amide compound of the formula (1), wherein E¹ is an A²-CR⁸R⁹R¹⁰ group, A² is methylene, R⁸, R⁹ and R¹⁰ are as defined above, X¹ is a methoxy group, and Z¹ is an oxygen atom;
an amide compound of the formula (1), wherein E¹ is an A² - CR⁸R⁹R¹⁰ group, A² is -CH(CH₃)-, R⁸, R⁹ and R¹⁰ are as defined above, X¹ is a methoxy group, and Z¹ is an oxygen atom;
an amide compound of the formula (1), wherein E¹ is an A²-CR⁸R⁹R¹⁰ group, A² is -C(CH₃)₂- R⁸, R⁹ and R¹⁰ are as defined above, X¹ is a methoxy group, and Z¹ is an oxygen atom;
an amide compound of the formula (1), wherein E¹ is an A² - CR⁸R⁹R¹⁰ group, A² is as defined above, R⁸ is a methyl group, R⁹ is a methyl group, R¹⁰ is as defined above, X¹ is a methoxy group, and Z¹ is an oxygen atom;
an amide compound of the formula (1), wherein E¹ is an A²-CR⁸R⁹R¹⁰ group, A² is methylene, -CH(CH₃)- or -C(CH₃)₂-, R⁸ is a methyl group, R⁹ is a methyl group, R¹⁰ is as defined above, X¹ is a methoxy group, and Z¹ is an oxygen atom;
an amide compound of the formula (1), wherein E¹ is an A² - CR⁸R⁹R¹⁰ group, R² is methylene, R⁸ is a methyl group, R⁹ is a methyl group, R¹⁰ is as defined above, X¹ is a methoxy group, and Z¹ is an oxygen atom;
an amide compound of the formula (1), wherein E¹ is an A²-CR⁸R⁹R¹⁰ group, A² is -CH(CH₃)-, R⁸ is a methyl group, R⁹ is a methyl group, R¹⁰ is as defined above, X¹ is a methoxy group, and Z¹ is an oxygen atom;
an amide compound of the formula (1), wherein E¹ is an A²-CR⁸R⁹R¹⁰ group, A² is -C(CH₃)₂-, R⁸ is a methyl group, R⁹ is a methyl group, R¹⁰ is as defined above, X¹ is a methoxy group, and Z¹ is an oxygen atom;
an amide compound of the formula (1), wherein E¹ is a 2-methylpropyl group, a 1,2-dimethylpropyl group, a 2,2-dimethylpropyl group, a 1,2,2-trimethylpropyl group, a 2-methyl-2-hydroxypropyl group or a 1,2-dimethyl-2-hydroxypropyl group, X¹ is a methoxy group, and Z¹ is an oxygen atom;

an amide compound of the formula (1), wherein E¹ is an A¹-Cy¹ group (A¹ and Cy¹ are as defined above), X¹ is an amino group, and Z¹ is an oxygen atom;
an amide compound of the formula (1), wherein E¹ is an A¹-Cy¹ group, A¹ is a single bond, methylene or -CH(CH₃)-, Cy¹ is as defined above, X¹ is an amino group, and Z¹ is an oxygen atom;
an amide compound of the formula (1), wherein E¹ is an A¹-Cy¹ group, A¹ is a single bond, Cy¹ is as defined above, X¹ is an amino group, and Z¹ is an oxygen atom;
an amide compound of the formula (1), wherein E¹ is an A¹-Cy¹ group, A¹ is methylene, Cy¹ is as defined above, X¹ is an amino group, and Z¹ is an oxygen atom;
an amide compound of the formula (1), wherein E¹ is an A¹-Cy¹ group, A¹ is -CH(CH₃)-, Cy¹ is as defined above, X¹ is an amino group, and Z¹ is an oxygen atom;
an amide compound of the formula (1), wherein E¹ is an A¹-Cy¹ group, A¹ is as defined above, Cy¹ is a C3-C6 cycloalkyl group optionally substituted with at least one member selected from the group [a-1], X¹ is an amino group, and Z¹ is an oxygen atom;
an amide compound of the formula (1), wherein E¹ is an A¹-Cy¹ group, A¹ is as defined above, Cy¹ is a C3-C6 cycloalkyl group optionally substituted with at least one member selected from the group consisting of a halogen atom, a C1-C4 alkyl group, a C2-C4 alkenyl group, a C2-C4 alkynyl group, a hydroxyl group, a cyano group, a C1-C6 alkoxy group, a C1-C3 alkylidene group which forms a double bond with a ring-forming carbon atom, and a C1-C6 hydroxyalkyl group, X¹ is an amino group, and Z¹ is an oxygen atom;
an amide compound of the formula (1), wherein E¹ is an A¹-Cy¹ group, A¹ is as defined above, Cy¹ is a C3-C6 cycloalkyl group optionally substituted with at least one member selected from the group consisting of a halogen atom, a C1-C4 alkyl group, a hydroxyl group and a cyano group, X¹ is an amino group, and Z¹ is an oxygen atom;

an amide compound of the formula (1), wherein E¹ is a 2-methylcyclopentyl group, a 2-fluorocyclopentyl group, a 2-chlorocyclopentyl group, a 2-hydroxycyclopentyl group, a 2-methylcyclohexyl group, a 2-fluorocyclohexyl group, a 2-chlorocyclohexyl group, a 2-hydroxycyclohexyl group, a cyclobutylmethyl group, a cyclopentylmethyl group, a cyclohexylmethyl group, a (1-hydroxycyclobutyl)methyl group, a (1-hydroxycyclopentyl)methyl group, a (1-hydroxycyclohexyl)methyl group, a 1-cyclobutylethyl group, a 1-cyclopentylet.hyl group, a 1-cyclohexylethyl group, a 1-(1-hydroxycyclobutyl)ethyl group, a 1-(1-hydroxycyclopentyl)ethyl group or a 1-(1-hydroxycyclohexyl)ethyl group, X¹ is an amino group, and Z¹ is an oxygen atom;
an amide compound of the formula (1), wherein E¹ is a 2-methylcyclopentyl group, a 2-fluorocyclopentyl group, a 2-chlorocyclopentyl group, a 2-hydroxycyclopentyl group, a 2-methylcyclohexyl group, a 2-fluorocyclohexyl group, a 2-chlorocyclohexyl group or a 2-hydroxycyclohexyl group, X¹ is an amino group, and Z¹ is an oxygen atom;
an amide compound of the formula (1), wherein E¹ is a cyclobutylmethyl group, a cyclopentylmethyl group, a cyclohexylmethyl group, a (1-hydroxycyclobutyl)methyl group, a (1-hydroxycyclopentyl)methyl group, a (1-hydroxycyclohexyl)methyl group, a 1-cyclobutylethyl group, a 1-cyclopentylethyl group, a 1-cyclohexylethyl group, a 1-(1-hydroxycyclobutyl)ethyl group, a 1-(1-hydroxycyclopentyl)ethyl group or a 1-(1-hydroxycyclohexyl)ethyl group, X¹ is an amino group, and Z¹ is an oxygen atom;

an amide compound of the formula (1), wherein E¹ is a 2-methylcyclohexyl group, X¹ is an amino group, and Z¹ is an oxygen atom;
an amide compound of the formula (1), wherein E¹ is a 2-chlorocyclohexyl group, X¹ is an amino group, and Z¹ is an oxygen atom;
an amide compound of the formula (1), wherein, E¹ is a cyclohexylmethyl group, X¹ is an amino group, and Z¹ is an oxygen atom;
an amide compound of the formula (1), wherein E¹ is a 1-cyclohexylethyl group, X¹ is an amino group, and Z¹ is an oxygen atom;
an amide compound of the formula (1), wherein E¹ is a (1-hydroxycyclohexyl)methyl group, X¹ is an amino group, and Z¹ is an oxygen atom;
an amide compound of the formula (1), wherein E¹ is a 1-(1-hydroxycyclohexyl)ethyl group, X¹ is an amino group, and Z¹ is an oxygen atom;
an amide compound of the formula (1), wherein E¹ is a cyclobutylmethyl group, X¹ is an amino group, and Z¹ is an oxygen atom;
an amide compound of the formula (1), wherein E¹ is a 1-cyclobutylethyl group, X¹ is an amino group, and Z¹ is an oxygen atom;
an amide compound of the formula (1), wherein E¹ is a (1-hydroxycyclobutyl)methyl group, X¹ is an amino group, and Z¹ is an oxygen atom;
an amide compound of the formula (1), wherein E¹ is a 1-(1-hydroxycyclobutyl)ethyl group, X¹ is an amino group, and Z¹ is an oxygen atom;

an amide compound of the formula (1), wherein X¹ is a fluorine atom, a C1-C4 alkoxy group, a C3-C4 alkynyloxy group or an NR¹R² group (R¹ and R² are as defined above); an amide compound of the formula (1), wherein X¹ is a fluorine atom, a C1-C4 alkoxy group, a C3-C4 alkynyloxy group or an NR¹R² group (R¹ and R² are as defined above), and X² is a hydrogen atom, a fluorine atom or a C1-C4 alkoxy group;
an amide compound of the formula (1), wherein X¹ is a fluorine atom, a C1-C4 alkoxy group, a C3-C4 alkynyloxy group or an NR¹R² group, X² is a hydrogen atom, a fluorine atom or a C1-C4 alkoxy group, and R¹ and R² independently represent a hydrogen atom, a C1-C4 alkyl group or a C2-C5 alkylcarbonyl group;
an amide compound of the formula (1), wherein X¹ is a fluorine atom, a C1-C4 alkoxy group, a C3-C4 alkynyloxy group or an NR¹R² group, X² is a hydrogen atom, a fluorine atom or a C1-C4 alkoxy group, R¹ and R² independently represent a hydrogen atom, a C1-C4 alkyl group or a C2-C5 alkylcarbonyl group, and E¹ is A¹-Cy¹ (A¹ and Cy¹ are as defined above);
an amide compound of the formula (1), wherein X¹ is a fluorine atom, a C1-C4 alkoxy group, a C3-C4 alkynyloxy group or an NR¹R² group, X² is a hydrogen atom, a fluorine atom or a C1-C4 alkoxy group, R¹ and R² independently represent a hydrogen atom, a C1-C4 alkyl group or a C2-C5 alkylcarbonyl group, E¹ is an A¹-Cy¹ group, A¹ is a single bond, methylene, -CH(CH₃)-, -CH₂CH₂- or -CH₂CH₂CH₂-, and Cy¹ is as defined above;
an amide compound of the formula (1), wherein X¹ is a fluorine atom, a C1-C4 alkoxy group, a C3-C4 alkynyloxy group or an NR¹R² group, X² is a hydrogen atom, a fluorine atom or a C1-C4 alkoxy group, R¹ and R² independently represent a hydrogen atom, a C1-C4 alkyl group or a C2-C5 alkylcarbonyl group, E¹ is an A¹-Cy¹ group, A¹ is a single bond, methylene, -CH(CH₃)-, -CH₂CH₂- or -CH₂CH₂CH₂-, and Cy¹ is a C3-C6 cycloalkyl group optionally substituted with a group of the group [a-1];
an amide compound of the formula (1), wherein X¹ is a fluorine atom, a C1-C4 alkoxy group, a C3-C4 alkynyloxy group or an NR¹R² group, X² is a hydrogen atom, a fluorine atom or a C1-C4 alkoxy group, R¹ and R² independently represent a hydrogen atom, a C1-C4 alkyl group or a C2-C5 alkylcarbonyl group, E¹ is an A¹-Cy¹ group, A¹ is a single bond, methylene, -CH(CH₃)-, -CH₂CH₂- or -CH₂CH₂CH₂-, and Cy¹ is a C3-C6 cycloalkyl group optionally substituted with a group of the group [a-1];
an amide compound of the formula (1), wherein X¹ is a fluorine atom, a C1-C4 alkoxy group, a C3-C4 alkynyloxy group or an NR¹R² group, X² is a hydrogen atom, a fluorine atom or a C1-C4 alkoxy group, R¹ and R² independently represent a hydrogen atom, a C1-C4 alkyl group or a C2-C5 alkylcarbonyl group, E¹ is an A¹-Cy¹ group, A¹ is a single bond, methylene, -CH(CH₃)-, -CH₂CH₂- or -CH₂CH₂CH₂-, and Cy¹ is a C3-C6 cycloalkyl group optionally substituted with at least one member selected from the group consisting of a halogen atom, a C1-C4 alkyl group and a hydroxyl group;

an amide compound of the formula (1), wherein E¹ is an A¹-Cy¹ group, A¹ is a single bond, methylene, -CH(CH₃)-, -CH₂CH₂- or -CH₂CH₂CH₂-, and Cy¹ is as defined above;
an amide compound of the formula (1), wherein E¹ is an A¹-Cy¹ group, A¹ is a single bond, methylene, -CH(CH₃)-,
-CH₂CH₂- or -CH₂CH₂CH₂-, and Cy¹ is a C3-C6 cycloalkyl group optionally substituted with a group of the group [a-1];
an amide compound of the formula (1), wherein E¹ is an A¹-Cy¹ group, A¹ is a single bond, methylene, -CH(CH₃)-, -CH₂CH₂- or -CH₂CH₂CH₂-, and Cy¹ is a C3-C6 cycloalkyl group optionally substituted with a group of the group [a-1];
an amide compound of the formula (1), wherein E¹ is an A¹-Cy¹ group, A¹ is a single bond, methylene, -CH(CH₃)-, -CH₂CH₂- or -CH₂CH₂CH₂-, Cy¹ is a C3-C6 cycloalkyl group optionally substituted with at least one member selected from the group consisting of a halogen atom, a C1-C4 alkyl group and a hydroxyl group;

an amide compound of the formula (1), wherein X¹ is a fluorine atom or a C1-C4 alkyl group, and X² is a hydrogen atom or a fluorine atom;
an amide compound of the formula (1), wherein X¹ is a fluorine atom or a C1-C4 alkyl group, X² is a hydrogen atom or a fluorine atom, and E¹ is an A²-CR⁸R⁹R¹⁰ group (A², R⁸, R⁹ and R¹⁰ are as defined above);
an amide compound of the formula (1), wherein X¹ is a fluorine atom or a C1-C4 alkyl group, X² is a hydrogen atom or a fluorine atom, E¹ is an A²-CR⁸R⁹R¹⁰ group, A² is -CH(CH₃)- or -C(CH₃)₂-, and R⁸, R⁹ and R¹⁰ are as defined above;
an amide compound of the formula (1), wherein X¹ is a fluorine atom or a C1-C4 alkyl group, X² is a hydrogen atom or a fluorine atom, E¹ is an A²-CR⁸R⁹R¹⁰ group, A² is -CH(CH₃)- or -C(CH₃)₂-, R⁸ and R⁹ are as defined above, and R¹⁰ is a hydrogen atom or a C1-C4 alkyl group;

an amide compound of the formula (1), wherein E¹ is an A²-CR⁸R⁹R¹⁰ group, A² is -CH(CH₃)- or -C(CH₃)₂-, and R⁸, R⁹ and R¹⁰ are as defined above;
an amide compound of the formula (1), wherein E¹ is an A²-CR⁸R⁹R¹⁰ group, A² is -CH(CH₃)- or -C(CH₃)₂-, R⁸ and R⁹ are as defined above, and R¹⁰ is a hydrogen atom or a C1-C4 alkyl group;
an amide compound of the formula (1), wherein E¹ is an A²-CR⁸R⁹R¹⁰ group (A², R⁸, R⁹ and R¹⁰ are as defined above), X¹ is an amino group, and Z¹ is an oxygen atom;
an amide compound of the formula (1), wherein E¹ is an A²-CR⁸R⁹R¹⁰ group, A² is methylene, -CH(CH₃)- or -C(CH₃)₂-, R⁸, R⁹ and R¹⁰ are as defined above, X¹ is an amino group, and Z¹ is an oxygen atom;
an amide compound of the formula (1), wherein E¹ is an A²-CR⁸R⁹R¹⁰ group, A² is methylene, R⁸, R⁹ and R¹⁰ are as defined above, X¹ is an amino group, and Z¹ is an oxygen atom;
an amide compound of the formula (1), wherein E¹ is an A²-CR⁸R⁹R¹⁰ group, A² is -CH(CH₃)-, R⁸, R⁹ and R¹⁰ are as defined above, X¹ is an amino group, and Z¹ is an oxygen atom;
an amide compound of the formula (1), wherein E¹ is an A²-CR⁸R⁹R¹⁰ group, A² is -C(CH₃)₂-, R⁸, R⁹ and R¹⁰ are as defined above, X¹ is an amino group, and Z¹ is an oxygen atom;
an amide compound of the formula (1), wherein E¹ is an A²-CR⁸R⁹R¹⁰ group, A² is as defined above, R⁸ is a methyl group, R⁹ is a methyl group, R¹⁰ is as defined above, X¹ is an amino group, and Z¹ is an oxygen atom;
an amide compound of the formula (1), wherein E¹ is an A²-CR⁸R⁹R¹⁰ group, A² is methylene, -CH(CH₃)- or -C(CH₃)₂-, R⁸ is a methyl group, R⁹ is a methyl group, R¹⁰ is as defined above, X¹ is an amino group, and Z¹ is an oxygen atom;
an amide compound of the formula (1), wherein E¹ is an A²-CR⁸R⁹R¹⁰ group, A² is methylene, R⁸ is a methyl group, R⁹ is a methyl group, R¹⁰ is as defined above, X¹ is an amino group, and Z¹ is an oxygen atom;
an amides compound of the formula (1), wherein E¹ is an A²-CR⁸R⁹R¹⁰ group, A² is -CH(CH₃)-, R⁶ is a methyl group, R⁹ is a methyl group, R¹⁰ is as defined above, X¹ is an amino group, and Z¹ is an oxygen atom;
an amide compound of the formula (1), wherein E¹ is an A²-CR⁸R⁹R¹⁰ group, A² is -C(CH₃)₂-, R⁸ is a methyl group, R⁹ is a methyl group, R¹⁰ is as defined above, X¹ is an amino group, and Z¹ is an oxygen atom; and
an amide compound of the formula (1), wherein E¹ is a 2-methylpropyl group, a 1,2-dimethylpropyl group, a 2,2-dimethylpropyl group, a 1,2,2-trimethylpropyl group, a 2-methyl-2-hydroxypropyl group or a 1,2-dimethyl-2-hydroxypropyl group, X¹ is an amino group, and Z¹ is an oxygen atom.

Examples of the compound of the present invention further include:
an amide compound of the formula (1), wherein X¹ is a fluorine atom, a C1-C4 alkoxy group or an NR¹R² group, and R¹ and R² each represent a hydrogen atom, a methyl group or a methoxycarbonyl group;
an amide compound of the formula (1), wherein X² is a hydrogen atom;
an amide compound of the formula (1), wherein Z¹ is an oxygen atom;
an amide compound of the formula (1), wherein E¹ is an A¹-Cy¹ group or an A²-CR⁸R⁹R¹⁰ group, A¹ is a single bond, methylene, -CH(CH₃)-, -CH₂CH₂- or -CH₂CH₂CH₂-, Cy¹ is a C3-C6 cycloalkyl group optionally substituted with at least one member selected from the group consisting of a halogen atom, a C1-C4 alkyl group and a hydroxyl group, A² is -CH(CH₃)-, R⁸ and R⁹ each represent a methyl group, and R¹⁰ is a hydrogen atom or a methyl group;
an amide compound of the formula (1), wherein X¹ is a fluorine atom, a C1-C4 alkoxy group or an NR¹R² group, R¹ and R² each represent a hydrogen atom, a methyl group or a methoxycarbonyl group, X² is a hydrogen atom, Z¹ is an oxygen atom, E¹ is an A¹-Cy¹ group or an A²-CR⁸R⁹R¹⁰ group, A¹ is a single bond, methylene, -CH(CH₃)-, -CH₂CH₂- or -CH₂CH₂CH₂-, Cy¹ is a C3-C6 cycloalkyl group optionally substituted with at least one member selected from the group consisting of a halogen atom, a C1-C4 alkyl group and
a hydroxyl group, A² is -CH(CH₃)-, R⁸ and R⁹ each represents a methyl group, and R¹⁰ is a hydrogen atom or a methyl group;
an amide compound of the formula (1), wherein X² is a hydrogen atom, a fluorine atom, a C1-C4 alkoxy group or an NR³R⁴ group, and R³ and R⁴ each represent a hydrogen atom or a methyl group;
an amide compound of the formula (1), wherein X¹ is a fluorine atom, a C1-C4 alkoxy group or an NR¹R² group, X² is a hydrogen atom, a fluorine atom, a C1-C4 alkoxy group or an NR³R⁴ group, R¹ and R² each represent a hydrogen atom, a methyl group or a methoxycarbonyl group, R³ and R⁴ each represent a hydrogen atom or a methyl group, Z¹ is an oxygen atom, E¹ is an A¹-Cy¹ group, A¹ is a single bond, methylene or -CH(CH₃)-, and Cy¹ is a C3-C6 cycloalkyl group optionally substituted with at least one member selected from the group consisting of a halogen atom, a C1-C4 alkyl group and a hydroxyl group;

an amide compound of the formula (1), wherein X¹ is a fluorine atom, an amino group or a methoxy group;
an amide compound of the formula (1), wherein E¹ is an A¹-Cy¹ group, A¹ is a single bond, methylene or -CH(CH₃)-, and Cy¹ is a C5-C6 cycloalkyl group optionally substituted with a chlorine atom, a methyl group or a hydroxyl group;
an amide compound of the formula (1), wherein X¹ is a fluorine atom or a methoxy group, X² is a hydrogen atom, Z¹ is an oxygen atom, E¹ is an A¹-Cy¹ group, A¹ is a single bond, methylene or -CH(CH₃)-, and Cy¹ is a C5-C6 cycloalkyl group optionally substituted with a chlorine atom, a methyl group or a hydroxyl group;
an amide compound of the formula (1), wherein X² a hydrogen atom, a fluorine atom or amino group; and
an amide compound of the formula (1), wherein X¹ is a fluorine atom, a methoxy group or an amino group, X² is a hydrogen atom, a fluorine atom, a methoxy group or an amino group, Z¹ is an oxygen atom, E¹ is an A¹-Cy¹ group, A¹ is a single bond, methylene or -CH(CH₃)-, and Cy¹ is a C3-C6 cycloalkyl group optionally substituted with at least one member selected from the group consisting of a halogen atom, a C1-C4 alkyl group and a hydroxyl group.

Herein, for the sake of convenience, the structural formula of a compound may be represented in a certain form of an isomer. However, in the present invention, a compound includes all kinds of active isomers which may occur due to the structure, such as a geometric isomer, an optical isomer, a stereoisomer and a tautomeric isomer, and a mixture thereof. Thus, a compound is not limited to the formula described for the sake of convenience, and can be a single isomer or a mixture of isomers. Therefore, a compound may have an asymmetric carbon in the molecule and may be in an optically active form or a racemic form. The present invention is not particularly limited thereby, and includes any cases.

The compound of the present invention can be produced by, for example, Production Process 1 to Production Process 6 shown below.

### (Production Process 1)

Among amide compounds represented by formula (1), a compound (5) wherein Z¹ is an oxygen atom can be produced by reacting a compound (2) or a salt thereof (for example, hydrochloride) with a compound (3) in the presence of a dehydration condensing agent: 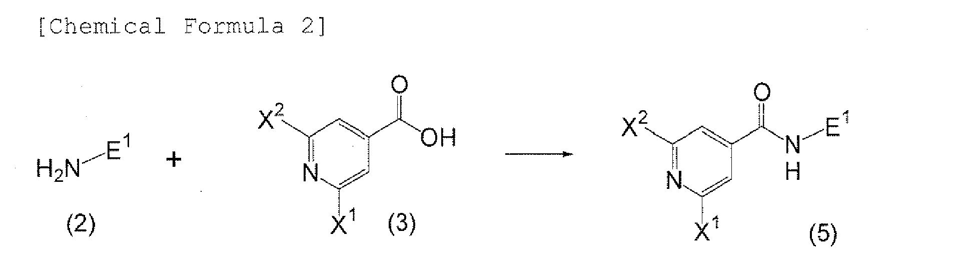 wherein E¹, X¹ and X² are as defined above.
The reaction is usually carried out in the presence of a solvent.
Examples of the solvent used for the reaction include ethers such as tetrahydrofuran (hereinafter, may be referred to as THF), ethylene glycol dimethyl ether and tert-butyl methyl ether (hereinafter, may be referred to as MTBE); aliphatic hydrocarbons such as hexane, heptane and octane; aromatic hydrocarbons such as toluene and xylene; halogenated hydrocarbons such as chlorobenzene; esters such as butyl acetate and ethyl acetate; nitriles such as acetonitrile; acid amides such as N,N-dimethyl formamide (hereinafter, may be referred to as DMF); sulfoxides such as dimethyl sulfoxide (hereinafter, may be referred to as DMSO); and their mixtures.
Examples of the dehydration condensing agent used for the reaction include carbodiimides such as 1-ethyl-3-(3-dimethylaminopropyl)carbodiimide hydrochloride (hereinafter, referred to as WSC) and 1,3-dicyclohexylcarbodiimide; and phosphonium salts such as (benzotriazol-1-yloxy)tris(dimethylamino)phosphonium hexafluorophosphate (hereinafter, referred to as a BOP reagent).
The used amount of the compound (3) is usually 1 to 3 mol per 1 mol of the compound (2) or a salt thereof, and the used amount of the dehydration condensing agent is usually 1 to 5 mol per 1 mol of the compound (2) or a salt thereof.
The reaction temperature is usually within a range from 0 to 140°C, and the reaction time is usually within a range from 1 to 24 hours.
After completion of the reaction, the compound (5) can be isolated by post-treatment such as filtration of the reaction mixture, extraction of the filtrate with an organic solvent, and drying and concentration of an organic layer. The isolated compound (5) can be further purified by chromatography, recrystallization and so on.

### (Production Process 2)

Among amide compounds represented by formula (1), the compound (5) wherein Z¹ is an oxygen atom can be produced by reacting the compound (2) or salt thereof (for example, hydrochloride) with a compound (4) or a hydrochloride thereof in the presence of a base: 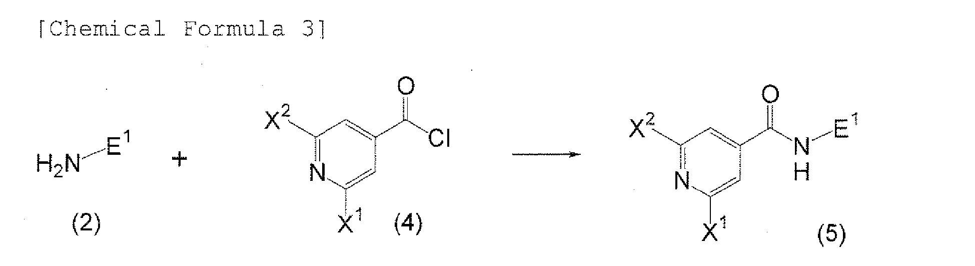 wherein E¹, X¹ and X² are as defined above.
The reaction is usually carried out in the presence of a solvent.
Examples of the solvent used for the reaction include ethers such as THF, ethylene glycol dimethyl ether and MTBE; aliphatic hydrocarbons such as hexane, heptane and octane; aromatic hydrocarbons such as toluene and xylene; halogenated hydrocarbons such as chlorobenzene; esters such as butyl acetate and ethyl acetate; nitriles such as acetonitrile; acid amides such as DMF; sulfoxides such as DMSO; and their mixtures.
Examples of the base used for the reaction include alkali metal carbonates such as sodium carbonate and potassium carbonate; tertiary amines such as triethylamine and diisopropylethylamine; and nitrogen-containing aromatic compounds such as pyridine and 4-dimethylaminopyridine.
The used amount of the compound (4) is usually 1 to 3 mol per 1 mol of the compound (2) or a salt thereof, and the used amount of the base is usually 1 to 10 mol per 1 mol of the compound (2) or a salt thereof.
The reaction temperature is usually within a range from -20 to 100°C, and the reaction time is usually within a range from 0.1 to 24 hours.
After completion of the reaction, the compound (5) can be isolated by post-treatment such as extraction of the reaction mixture with an organic solvent, and drying and concentration of an organic layer. The isolated compound (5) can be further purified by chromatography, recrystallization and so on.

### (Production Process 3)

Among amide compounds represented by formula (1), a compound (6) wherein Z¹ is a sulfur atom can be produced by reacting the compound (5) wherein Z¹ an oxygen atom with a sulfurizing agent such as 2,4-bis(4-methoxyphenyl)-1,3-dithia-2,4-diphosphethane-2,4-disulfide (hereinafter, referred to as Lawesson's reagent) or phosphorous pentasulphide: 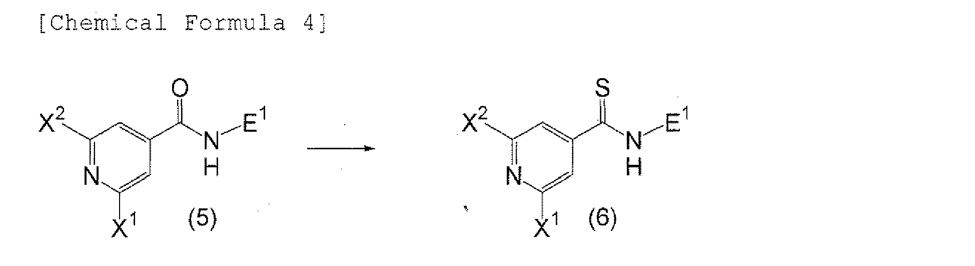 wherein E¹, X¹ and X² are as defined above.
The reaction is usually carried out in the presence of a solvent.
Examples of the solvent used for the reaction include ethers such as THF, ethylene glycol dimethyl ether and MTBE; aliphatic hydrocarbons such as hexane, heptane and octane; aromatic hydrocarbons such as toluene and xylene; halogenated hydrocarbons such as chlorobenzene; organic nitriles such as acetonitrile and butyronitrile; sulfoxides such as dimethyl sulfoxide; and their mixtures.
The used amount of the sulfurizing agent is usually 1 to 2 mol per 1 mol of the compound (5).
The reaction temperature is usually within a range from 25 to 150°C, and the reaction time is usually within a range from 0.1 to 24 hours.
After completion of the reaction, the compound (6) can be isolated by post-treatment such as extraction of the reaction mixture with an organic solvent, and drying and concentration of an organic layer. The isolated compound (6) can be further purified by chromatography, recrystallization and so on.

### (Production Process 4)

Among amide compounds represented by formula (1), a compound (9) wherein X¹ is a C1-C4 alkoxy group, a C3-C4 alkenyloxy group, a C3-C4 alkynyloxy group or an NR¹R² group and Z¹ is an oxygen atom can be produced by reacting a compound (7) with a compound (8) in the presence of a base: 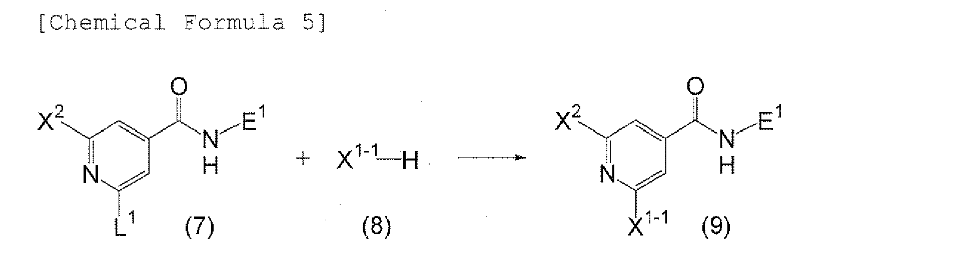 wherein E¹ and X² are as defined above, L¹ represents a fluorine atom or a chlorine atom, X¹⁻¹ represents a C1-C4 alkoxy group, a C3-C4 alkenyloxy group, a C3-C4 alkynyloxy group or an NR¹R² group, and R¹ and R² are as defined above.
The reaction is usually carried out in the presence of a solvent.
Examples of the solvent used for the reaction include ethers such as THF, ethylene glycol dimethyl ether and MTBE; aliphatic hydrocarbons such as hexane, heptane and octane; aromatic hydrocarbons such as toluene and xylene; halogenated hydrocarbons such as chlorobenzene; ketones such as acetone and isobutyl methyl ketone; nitriles such as acetonitrile; acid amides such as DMF; sulfoxides such as DMSO; alcohols such methanol, ethanol, 1-methylethanol and 1,1-dimethylethanol; water; and their mixtures.
Examples of the base used for the reaction include alkali metal hydride such as sodium hydride; alkali metal carbonates such as sodium carbonate and potassium carbonate; tertiary amines such as triethylamine and diisopropylethylamine; and alkali metal alkoxides such as potassium 1,1-dimethyl ethoxide.
The used amount of the compound (8) is usually 1 mol to an excess amount per 1 mol of the compound (7), and the used amount of the base is usually 1 to 10 mol per 1 mol of the compound (7).
After completion of the reaction, the compound (9) can be isolated by post-treatment such as extraction of the reaction mixture with an organic solvent, and drying and concentration of an organic layer. The isolated compound (9) can be further purified by chromatography, recrystallization and so on.
When X¹⁻¹ is an NR¹R² group, the compound (8) can also be used as a solvent.

### (Production Process 5)

Among amide compounds represented by formula (1), a compound represented by a formula (13) can be produced by the method shown in the following scheme: 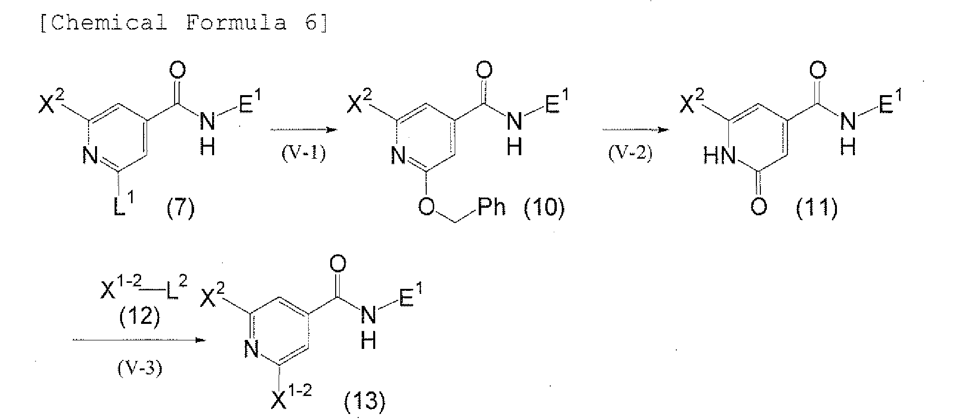 wherein E¹, L¹ and X² are as defined above, L² represents a chlorine atom, a bromine atom, an iodine atom or a methanesulfonyloxy group, X¹⁻² represents a C1-C4 alkoxy group, a C3-C4 alkenyloxy group or a C3-C4 alkynyloxy group, and Ph represents a phenyl group. Step (V-1)
According to the method described in Production Process 4, the compound (10) can be produced by reacting the compound (7) with benzyl alcohol in the presence of a base.

### Step (V-2)

The compound (11) can be produced by reacting the compound (10) with hydrogen in the presence of palladium carbon.
The reaction is usually carried out in the presence of a solvent.
Examples of the solvent used for the reaction include aliphatic hydrocarbons such as hexane, heptane and octane; aromatic hydrocarbons such as toluene and xylene; alcohols such as methanol and ethanol; esters such as ethyl acetate; ethers such as THF and MTBE; water; and their mixtures.
The used amount of palladium carbon is usually 0.01 to 0.1 mol per 1 mol of the compound (10), and the used amount of hydrogen is usually 1 to 2 mol per 1 mol of the compound (10).
The reaction temperature is usually within a range from 0 to 50°C, and the reaction time is usually within a range from 0.1 to 24 hours.
The pressure of hydrogen used for the reaction is usually from a normal pressure to 10 atm.
After completion of the reaction, the compound (11) can be isolated by post-treatment such as filtration and concentration of the reaction mixture. The isolated compound (11) can be further purified by chromatography, recrystallization and so on.
Alternatively, the compound (11) can also be produced by reacting the compound (7) with an alkali metal hydroxide such as sodium hydroxide without going through the compound (10).

### Step (V-3)

The compound (13) can be produced by reacting the compound (11) with the compound (12) in the presence of a base.
The reaction is usually carried out in the presence of a solvent.
Examples of the solvent used for the reaction include hydrocarbons such as toluene and hexane; ketones such as acetone and methyl isobutyl ketone; acid amides such as DMF; and sulfoxides such as DMSO.
Examples of the base used for the reaction include alkali metal carbonates such as sodium carbonate, potassium carbonate and cesium carbonate; and alkali metal hydrides such as sodium hydride.
The used amount of the compound (12) is usually 1 to 3 mol per 1 mol of the compound (11), and the used amount of the base is usually 1 to 3 mol per 1 mol of the compound (11).
The reaction temperature is usually within a range from 0 to 140°C, and the reaction time is usually within a range from 0.5 to 24 hours.
After completion of the reaction, the compound (13) can be isolated by post-treatment such as extraction of the reaction mixture with an organic solvent, and drying and concentration of an organic layer. The isolated compound (13) can be further purified by chromatography, recrystallization and so on.

### (Production Process 6)

Among amide compounds represented by formula (1), a compound represented by a formula (15) can be produced by the method shown in the following scheme: 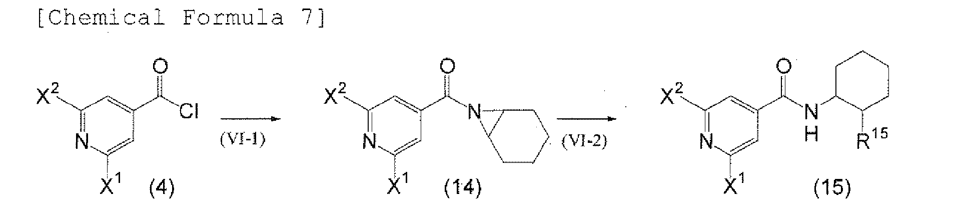 wherein X¹ and X² are as defined above, and R¹⁵ represents a fluorine atom, a chlorine atom, a bromine atom, an iodine atom, a cyano group, a C1-C3 alkylthio group, a C1-C6 alkoxy group or a phenoxy group.

### Step (VI-I)

According to the method described in Production Process 2, the compound (14) can be produced by reacting the compound (4) with 7-azabicyclo[4.1.0]heptane in the presence of a base.

### Step (VI-2)

The compound (15) can be produced by reacting the compound (14) with a reagent shown below.
Examples of the reagent used for the reaction are as follows.
When R¹⁵ is a fluorine atom, examples of the reagent include fluorinating agents, such as alkali metal fluorides such as potassium fluoride and lithium fluoride; alkali earth metal fluorides such as calcium fluoride; quaternary ammonium fluorides such as tetrabutylammonium fluoride; and hydrogen fluoride.
When R¹⁵ is a chlorine atom, examples of the reagent include chlorinating agents, such as alkali metal chlorides such as sodium chloride and lithium chloride; alkaline earth metal chlorides such as magnesium chloride; metal chlorides such as aluminum chloride and zinc(II) chloride; quaternary ammonium chlorides such as tetrabutylammonium chloride; organic silicon chlorides such as trimethylsilyl chloride; sulfur compounds such as thionyl chloride; phosphorus compounds such as phosphorus oxychloride, phosphorus trichloride and phosphorus pentachloride; and hydrogen chloride.
When R¹⁵ is a bromine atom, examples of the reagent include brominating agents, such as alkali metal bromides such as sodium bromide; alkaline earth metal bromides such as magnesium bromide; metal bromides such as zinc(II) bromide; quaternary ammonium bromides such as tetrabutylammonium bromide; organic silicon bromides such as trimethylsilyl bromide; phosphorus compounds such as phosphorus tribromide; and hydrogen bromide.
When R¹⁵ is an iodine atom, examples of the reagent include iodinating agents, such as alkali metal iodides such as potassium iodide; alkaline earth metal iodides such as magnesium iodide; metal iodides such as zinc(II) iodide; quaternary ammonium iodides such as tetrabutylammonium iodide; and organic silicon compounds such as trimethylsilyl iodide; and hydrogen iodide.
When R¹⁵ is a cyano group, examples of the reagent include cyanidating agents, such as cyanides such as potassium cyanide and sodium cyanide; and organic silicon compounds such as trimethylsilyl cyanide.
When R¹⁵ is a C1-C3 alkylthio group, examples of the reagent include a C1-C3 alkylmercaptan.
When R¹⁵ is a C1-C6 alkoxy group, examples of the reagent include a C1-C6 alcohol.
When R¹⁵ is a phenoxy group, examples of the reagent include phenol.

The reaction is usually carried out in the presence of a solvent.
Examples of the solvent used for the reaction include ethers such as THF, ethylene glycol dimethyl ether and MTBE; aromatic hydrocarbons such as toluene and xylene; halogenated hydrocarbons such as chlorobenzene and chloroform; esters such as butyl acetate and ethyl acetate; nitriles such as acetonitrile; acid amides such as DMF; water; and their mixtures.
The used amount of the reagent is usually 1 to 5 mol per 1 mol of the compound (14).
The reaction temperature is usually within a range from -20 to 150°C, and the reaction time is usually within a range from 0.1 to 24 hours.
If necessary, the reaction can also be carried out in the presence of an additive. Examples of the additive include phosphorus compounds such as tributyl phosphine.
The above-mentioned C1-C3 alkyl mercaptan, C1-C6 alcohol or phenol is reacted with an alkali metal hydride such as sodium hydride and so on to prepare an alkali metal, and then the alkali metal can also be used for the reaction.
After completion of the reaction, the compound (15) can be isolated by post-treatment such as extraction of the reaction mixture with an organic solvent, and drying and concentration of an organic layer. The isolated compound (15) can be further purified by chromatography, recrystallization and so on.
Among the compounds (15), the amide compound wherein R¹⁵ is a chlorine atom can be also synthesized by reacting the compound (4) with 7-azabicyclo[4.1.0]heptane and then reacting the reaction product with the above-described chlorinating agent.

Some of intermediates used for the production of the compound of the present invention are commercially available or are compounds disclosed in known literatures and so on. Such intermediates can be produced, for example, by the following methods.

### (Intermediate Production Process 1)

The compound (4) or a hydrochloride thereof can be produced by reacting the compound (3) with thionyl chloride: 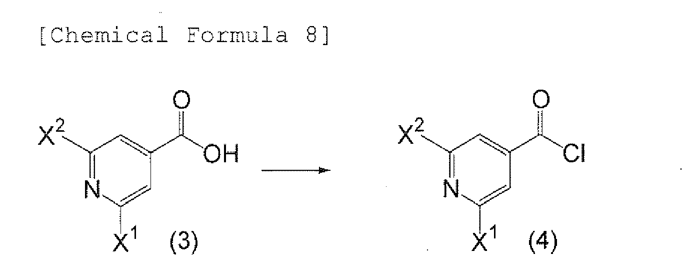 wherein X¹ and X² are as defined above.
The reaction is usually carried out in the presence of a solvent.
Examples of the solvent used for the reaction include aliphatic hydrocarbons such as hexane, heptane and octane; aromatic hydrocarbons such as toluene and xylene; nitriles such as acetonitrile; halogenated hydrocarbons such as chlorobenzene; acid amides such as DMF; and their mixtures.
The used amount of thionyl chloride is usually 1 to 5 mol per 1 mol of the compound (3).
The reaction temperature is usually within a range from 20 to 150°C, and the reaction time is usually within a range from 0.1 to 24 hours.
After completion of the reaction, the compound (4) can be isolated by post-treatment such as concentration of the reaction mixture. The isolated compound (4) can be further purified by recrystallization and so on.

The compound of the present invention exhibits the effect of controlling plant diseases.
Plant diseases against which the compound of the present invention exerts an excellent effect include plant diseases caused by fungi, bacteria and viruses. Specific examples of the fungi include *Erysiphe spp*. such as wheat powdery mildew (*Erysiphe graminis*), *Uncinula spp.* such as grape powdery mildew (*Unainula necator*), *Padosphaera spp.* such as apple powdery mildew (*Podosphaera leuectricha*), *Sphaerotheca spp*. such as cucumber powdery mildew (*Sphaerotheca cucurbitae*), *Oidiopsis spp*. such as tomato powdery mildew (*Oidiopsis sicula*), *Magnaporthe spp*. such as rice blast (*Magnaporthe oryzae*), *Cochliobolus spp*. such as rice brown spot (*Cochliobolus miyabeanus*), *Mycosphaerella spp*. such as wheat leaf blotch (*Mycosphaerella graminicola*), *Pyrenophora spp*. such as barley net blotch (*Pyrenophora teres*), *Stagonospora* spp. such as wheat Glume blotch (*Stagonospora nodorum*), *Rhynchosporium spp.* such as barley scald (*Rhynchasparium secalis*), *Pseudocercosporella spp.* such as wheat eyespot (*Pseudocercosporella herpotrichoides*), Gaeumannomyces *spp.* such as wheat take-all (*Gaeumannamyces graminis*), Fusarium *spp.* such as wheat Fusarium head blight (*Fusarium* spp.), *Microdochium spp*. such as wheat snow mold (*Microdochium nivale*), *Ventures spp.* such as apple scab (*Venturia inaequalis*), *Elsinoe spp.* such as grape anthracnose (*Elsinoe ampelina*), *Botrytis spp*. such as cucumber gray mold (*Botrytis cinerea*), *Monilinia spp.* such as peach brown rot (*Monilinia fructicola*), *Phoma spp.* such as rape stem canker (*Phoma lingam*), *Cladosporium spp.* such as tomato leaf mold (*Cladosporium fulvum*), *Cercospora spp.* such as sugarbeet brown spot (*Cercospora beticola*), *Cercosporidium spp.* such as peanut late leaf spot (*Cercosporidium personatum*), *Colletotrichum spp*. such as strawberry anthracnose (*Colletotrichum fragariae*), *Sclerotinia spp.* such as cucumber stem rot (*Scierotinia scierotiorum*), *Alternaria spp*. such as apple necrotic leaf spot (*Alternaria mali*), *Verticillium spp.* such as eggplant verticillium wilt (*Verticillium dahliae*), *Rhizoctonia spp.* such as rice sheath blight (*Rhizoctonia solani*), *Puccinia spp.* such as wheat leaf rust (*Puccinia recondita*), genus *Phakopsora* such as soybean rust (*Phakopsora paahyrhizi*), *Tilletia spp.* such as wheat bunt (*Tilletia caries*), *Ustilago spp.* such as barley loose smut (*Ustilago nuda*), *Sclerotium spp.* such as peanut southern blight (*Sclerotium rolfsii*), *Phytophthora spp*. such as potato late blight (*Phytophthora infestans*), *Pseudoperonospora spp.* such as cucumber downy mildew (*Pseudoperonospora cubensis*), *Peronospara spp*. such as Chinese cabbage downy mildew (*Peronospora parasitica*), *Plasmopara spp*. such as grape downy mildew (*Plamospara viticola*), Bremia spp. such as lettuce downy mildew (*Bremia lactucae*), *Sclerophthora spp.* such as rice downy mildew (*Sclerophthora macrospora*), *Pythium spp*. such as cucumber seedling damping-off (Pythium *ultimum*), and *Plasmodiophora spp*. such as rapeseed clubroot (*Plasmodiophora brassscae*), *Aphanomyces spp*. such as sugar beet black root (*Aphanomyces cochlioides*), *Thielaviopsis spp*. such as cotton black root rot (*Thielaviopsis basicola*), *Diplodia spp*. such as corn stem rot (*Diplodia maydis*), *Aspergillus* sp., *Penicillium* sp., *Trichoderma* sp.and *Rhizopus* sp.

Examples of the bacteria include *Burkholderia spp.* such as bacterial rice seedling blight (*Burkholderia plantarii*), *Pseudomonas spp*. such as bacterial cucumber leaf spot (Pseudomonas *syringae pv. Lachrymans), Ralstonia spp*. such as eggplant wilting (*Ralstonia solanacearum*), *Xanthomonas spp*. such as Asiatic citrus canker (*Xanthomonas citiri*), and *Erwinia spp*. such as Chinese cabbage bacterial soft rot (*Erwinia carotovora*).

Examples of the viruses include Tobacco mosaic virus and Cucumber mosaic virus. Examples of fungi responsible for viral diseases which mediate the viral diseases include *Polymyxa* sp., and *Olpidium* sp. The sterilizing spectrum of the compound of the present invention also includes fungi, bacteria and viruses other than the above-mentioned fungi, bacteria and viruses.

A plant disease control composition containing the compound of the present invention is also one aspect of the present invention.
The control composition of the present invention can be formulated by mixing the compound of the present invention with an inert carrier such as a solid carrier or a liquid carrier and, if necessary, a surfactant and other auxiliary agents for formulation. The control composition of the present invention can be formulated into an emulsifiable concentrate, a wettable powder, a granular wettable powder, a flowable formulation, a dust, a granule, and so on.
The control composition of the present invention contains usually 0.1 to 90% by weight of the compound of the present invention.

Examples of the solid carrier used in formulation include fine powders or particles of minerals such as kaolin clay, attapulgite clay, bentonite, montmorillonite, acid clay, pyrophyllite, talc, diatomaceous earth, and calcite; natural organic substances such as corncob powder, and walnut shell flour; synthetic organic substances such as urea; salts such as calcium carbonate, and ammonium sulfate; and synthetic inorganic substances such as synthetic hydrated silicon oxide.

Examples of the liquid carrier used in formulation include aromatic hydrocarbons such as xylene, alkylbenzene and methylnaphthalene; alcohols such as 2-propanol, ethylene glycol, propylene glycol and cellosolve; ketones such as acetone, cyclohexanone and isophorone; vegetable oils such as soybean oil and cotton seed oil; petroleum aliphatic hydrocarbons; esters; and dimethyl sulfoxide, acetonitrile and water.

Examples of the surfactant used in formulation include anionic surfactants such as alkyl sulfate, alkyl aryl sulfonate, dialkyl sulfosuccinate, polyoxyethylene alkyl aryl ether phosphate, ligninsulfonate and a naphthalenesulfonate formaldehyde polycondensate; and nonionic surfactants such as polyoxyethylene alkyl aryl ether, polyoxyethylene alkyl polyoxypropylene block copolymers and sorbitan fatty acid esters.

Examples of the auxiliary agent for formulation include water-soluble polymers such as polyvinyl alcohol and polyvinyl pyrrolidone; polysaccharides such as gum Arabic, alginic acid and a salt thereof, CMC(carboxymethyl cellulose) and xanthan gum; inorganic substances such as aluminum magnesium silicate, and alumina sol; preservatives; colorants; PAP (acidic isopropyl phosphate); and stabilizers such as BHT (dibutylhydroxytoluene).

The compound of the present invention can be used in treatment of plants, such as foliage treatment, to protect the plants from plant diseases, and also can be used in soil treatment to protect plants growing in the soil from plant diseases.
A method for controlling plant diseases which comprises applying an effective amount of the compound of the present invention to plants or soil is also one aspect of the present invention.
In the above-described treatment and application, the control composition of the present invention can be used as the compound of the present invention.

The application amount of the control agent of the present invention varies depending upon a weather condition, a formulation form, application time, an application method, a place to be applied, a target disease, a target crop, and so on. The application amount is usually within a range of from 1 to 500 g, preferably from 2 to 200 g per 10 ares of the compound of the present invention contained in the control agent of the present invention.
When the control agent of the present invention is in the form of a liquid formulation such as an emulsifiable concentrate, a wettable powder or a suspension, the formulation is usually used after dilution with water. In this case, the concentration of the compound of the present invention in a diluted solution is usually within a range from 0.0005 to 2% by weight, preferably from 0.005 to 1% by weight.
When the control agent of the present invention is in the form of a solid formulation such as a dust or a granule, the formulation is usually used for a treatment without being diluted.
In the case of seed treatment, the control agent of the present invention is usually applied in an amount within a range from 0.001 to 100 g, preferably from 0.01 to 50 g of the compound of the present invention per 1 kg of seeds.

The plant disease control method of the present invention is usually carried out by applying an effective amount of the compound of the present invention to a plant in which onset of diseases is presumed, or soil where the plant is grown, and/or applying an effective amount of the compound of the present invention to a plant in which onset of diseases has been confirmed, or the soil where the plant is grown.

The compound of the present invention can be used as a control agent for plant diseases in crop lands such as upland fields, paddy fields, lawn, and orchards. Specifically, the plant disease control agent can control plant diseases in crop lands where the following "crops" and so on are cultivated.

Field crops: corn, rice, wheat, barley, rye, oat, sorghum, cotton, soybean, peanut, buckwheat, beet, rape, sunflower, sugarcane, tobacco, etc.;
Vegetables: solanaceae (e.g. eggplant, tomato, green pepper, pepper and potato), Cucurbitaceae (e.g. cucumber, pumpkin, zucchini, watermelon and melon), Cruciferae (e.g. Japanese radish, turnip, horseradish, kohlrabi, Chinese cabbage, cabbage, leaf mustard, broccoli and cauliflower), Compositae (e.g. edible burdock, garland chrysanthemum, globe artichoke and lettuce), Liliacede (e.g., Welsh onion, onion, garlic and asparagus), Umbelliferae (e.g. carrot, parsley, celery and pastinaca), Chenopodiaceae (e.g. spinach and chard), Lamiaceae (e.g. perilla, mint and basil), strawberry, sweet potato, Chinese yam, taro, etc.;
Flowers and ornament plants;
Ornamental foliage plants;
Fruit trees: pomaceous fruits (e.g. apple, pear, Japanese pear, Chinese quince and quince), stone fruits (e.g. peach, plum, nectarine, Japanese apricot, cherry, apricot and prune), citrus fruits (e.g. satsuma mandarin, orange, lemon, lime and grapefruit), nut trees (e.g. chestnut, walnut, hazel, almond, pistachio, cashew nut and macadamia nut), berries (blueberry, cranberry, blackberry and raspberry), grape, Japanese persimmon, olive, loquat, banana, coffee, date palm, coconut palm, etc.;
Trees other than fruit trees: tea, mulberry, flowering trees and shrubs, street trees (e.g. Japanese ash, birch, flowering dogwood, blue gum, ginkgo, lilac, maple, oak, poplar, Chinese redbud, Formosa sweet gum, plane tree, zelkova, Japanese arborvitae, fir, Japanese hemlock, needle juniper, pine, Japanese spruce and Japanese yew), etc.

The above-described "crops" include crops having resistance to herbicides such as HPPD inhibitors (e.g. isoxaflutole), ALS inhibitors (e.g. imazethapyr and thifensulfuron-methyl), EPSP synthetase inhibitors, glutamine synthetase inhibitors, bromoxynil, and dicamba, which has been imparted by a classic breeding method or a genetic recombination technology.

Examples of the "crops" having the resistance imparted by a classic breeding method include Clearfield® canola resistant to imidazolinone herbicides (e.g. imazethapyr) and STS soybean resistant to sulfonylurea ALS inhibition type herbicides (e.g. thifensulfuron-methyl).
Examples of the "crops" having the resistance imparted by a genetic recombination technology include corn, soybean, cotton and rapeseed cultivars resistant to glyphosate and glufosinate, which are already on the market under the trade names of RoundupReady®, RoundupReady2® and LibertyLink®.

The above-described "crops" include genetically modified plants which have been enabled to synthesize a selective toxin and so on by using a genetic recombination technology.
Examples of the selective toxin produced in such genetically modified plants include insecticidal proteins derived from *Bacillus spp*., such as insecticidal proteins derived from *Bacillus* cereus and *Bacillus popilliae*; and insecticidal proteins such as δ-endotoxins (e.g. Cry1Ab, Cry1Ac, Cry1F, Cry1Fa2, Cry2Ab, Cry3A, Cry3Bb1 and Cry9C), VIP 1, VIP 2, VIP 3, VIP 3A, etc., which are derived from *Bacillus thuringiensis*. Further examples of the selective toxin include toxins derived from nematodes; toxins produced by animals, such as scorpion toxin, spider toxin, bee toxin, and insect-specific neurotoxins; filamentous fungi toxins; plant lectins; agglutinin; protease inhibitors such as trypsin inhibitors, patatin, cystatin, and papain inhibitors; ribosome-inactivating proteins (RIPs) such as ricin, corn-RIP, abrin, rufin, sapolin, and briodin; steroid metabolic enzymes such as 3-hydroxysteroid oxidase, ecdysteroid-UDP-glucosyltransferase, and cholesterol oxidase; ecdysone inhibitors; HMG-COA reductase; ion channel inhibitors such as sodium channel inhibitors, and calcium channel inhibitors; juvenile hormone esterase; diuretic hormone receptors; stilbene synthetase; bibenzyl synthetase; chitinase; and glucanase.

The toxins produced in such genetically modified crops also include hybrid toxins, partly deficient toxins and modified toxins of insecticidal proteins such as δ-endotoxin proteins (e.g., CrylAb, CrylAc, CrylF, CrylFa2, Cry2Ab, Cry3A, Cry3Bbl and Cry9C), VIP1, VIP2, VIP3, and VIP3A.
The hybrid toxins can be produced by a novel combination of different domains of such proteins, for example, using a recombinant technique. As the partly deficient toxin, Cry1Ab deficient in a part of the amino acid sequence is known. In the modified toxins, one or more amino acids of a natural toxin have been replaced.
Examples of such toxins and genetically modified plants capable of synthesizing such toxins are described in EP-A-0 374 753, WO 93/07278, WO 95/34656, EP-A-0 427 529, EP-A-451 878, and WO 03/052073.
The toxins contained in such genetically modified plants impart resistance to insect pests of, in particular, Coleoptera, Diptera and Lepidoptera to the plants.

Genetically modified plants containing one or more insecticidal insect-resistant genes and capable of producing one or more toxins have already been known, and some of them are on the market.
Examples of such genetically modified plants include YieldGard® (a corn cultivar capable of producing Cry1Ab toxin), YieldGard Rootworm® (a corn cultivar capable of producing Cry3Bb1 toxin), YieldGard Plus® (a corn cultivar capable of producing Cry1Ab and Cry3Bb1 toxins), Herculex I® (a corn cultivar capable of producing phosphinotrysin N-acetyltransferase (PAT) for imparting resistance to Cry1Fa2 toxin and Glyfosinate), NuCOTN33B (a cotton cultivar capable of producing Cry1Ac toxin), Bollgard I® (a cotton cultivar capable of producing Cry1Ac toxin), Boilgard II® (a cotton cultivar capable of producing Cry1Ac and Cry2Ab toxins), VIPCOT® (a cotton cultivar capable of producing VIP toxin), NewLeaf® (a potato cultivar capable of producing Cry3A toxin), NatureGard®, Agrisure® GT Advantage (GA21 glyphosate resistant properties), Agrisure® CB Advantage (Bt11 corn borer (CB) properties), and Protecta®.

The above-described "crops" also include crops having ability to produce an anti-pathogenic substance having a selective action which has been imparted by a genetic recombination technology.
As examples of the anti-pathogenic substance produced in such genetically modified plants, PR proteins (PRPs) and so on are known. Such anti-pathogenic substances and genetically modified plants capable of producing them are described in EP-A-0 392 225, WO 95/33818, EP-A-0 353 191, etc.
Examples of such anti-pathogenic substances produced in the genetically modified plants include ion channel inhibitors such as sodium channel inhibitors, and calcium channel inhibitors (for example, KP1, KP4, and KP6 toxins produced by viruses are known); stilbene synthase; bibenzyl synthase; chitinase; glucanase; and anti-pathogenic substances produced by microorganisms, such as peptide antibiotics, antibiotics having a heterocyclic ring, and protein factors concerned in resistance to plant diseases (which are called plant-disease-resistant genes and are described in WO 03/000906).
The above-described plants also include plants having two or more kinds of traits relating to the above-described herbicide resistance, pest insect resistance or disease resistance which have been imparted by using a classic breeding method or a genetic recombination technology, and plants having two or more kinds of properties of parent plants which have been imparted by cross combination of genetically modified plants having the same or different properties.

The compound of the present invention can be used in admixture with other active ingredients such as fungicides, insecticides, acaricides, nematocides, herbicides, plant growth regulators, fertilizers or soil conditioners. The compound of the present invention can be used simultaneously with other active ingredients without mixing. The control composition of the present invention can be used in admixture with other active ingredients, or can be used simultaneously with other active ingredients without mixing.

Examples of the fungicides include:
(1) azole fungicides such as propiconazole, prothioconazole, triadimenol, prochloraz, penconazole, tebuconazole, flusilazole, diniconazole, bromuconazole, epoxiconazole, difenoconazole, cyproconazole, metconazole, triflumizole, tetraconazole, microbutanil, fenbuconazole, hexaconazole, fluquinconazole, triticonazols, bitertanol, imazalil, flutriafol, simeconazole, and ipconazole;

(2) amine fungicides such as fenpropimorph, tridemorph, fenpropidin, and spiroxamine;

(3) benzimidazole fungicides such as carbendazim, benomyl, thiabendazole, and thiophanate-Methyl;

(4) dicarboxyimide fungicides such as procymidone, iprodione, and vinclozolin;

(5) anilinopyrimidine fungicides such as cyprodinil, pyrimethanil, and mepanipyrim;

(6) phenylpyrrole fungicides such as fenpiclonil and fludioxonil;

(7) strobilurin fungicides such as kresoxim-methyl, azoxystrobin, trifloxystrobin, fluoxastrobin, picoxystrobin, pyraclostrobin, dimoxystrobin, pyribencarb, metominostrobin, oryzastrobin, and enestrabin;

(8) phenylamide fungicides such as metalaxyl, metalaxyl-M or mefenoxam, benalaxyl, and benalaxyl-M or kiralaxyl;

(9) carboxamide fungicides such as dimethomorph, iprovalicarb, benthiavalicarb-isopropyl, mandipropamid, and valiphenal;

(10) carboxamide fungicids such as carboxin, mepronil, flutolanil, thifluzamide, furametpyr, boscalid, penthiopyrad, fluopyram, and bixafen;

(11) other fungicides or plant disease control agents, such as diethofencarb; thiuram; fluazinam; mancozeb; chlorothalonil; captan; dichlofluanide; folpet; quinoxyfen; phenhexamid; famoxadone; fenamidon; zoxamide; ethaboxam; amisulbrom; cyazofamid; metrafenone; cyflufenamid; proquinazid; flusulfamide; fluopioolide; fosetyl; cymoxanil; pencycuron; tolclofos-methyl; carpropamid; diclocymet; fenoxanil; tricyclazole; pyroquilon; probenazole; isotianil; tiadinil; tebufloquin, diclomezine; kasugamycin; ferimzone; fthalide; validamycin; hydoroxyisoxazole; iminoctadine acetate; isoprothiolane; oxolinic acid; oxytetracycline; streptomycin; basic copper chloride; cupric hydroxide; basic copper sulfate; organic copper; and sulfur.

Examples of the insecticides include:
(1) organic phosphorous compounds such as acephate, aluminium phosphide, butathiofos, cadusafos, chlorethoxyfos, chlorfenvinphos, chlorpyrifos, chlorpyrifos-methyl, cyanophos (CYAP), diazinon, DCIP (dichlorodiisopropyl ether), dichlofenthion (ECP), dichlorvos (DDVP), dimethoate, dimethylvinphos, disulfoton, EPN, ethion, ethoprophos, etrimfos, fenthion (MPP), fenitrothion (MEP), fosthiazate, formothion, Hydrogen phosphide, isofenphos, isoxathion, malathion, mesulfenfos, methidathion (DMTP), monocrotophos, naled (BRP), oxydeprofos (ESP), parathion, phosalone, phosmet (PMP), pirimiphos-methyl, pyridafenthion, quinalphos, phenthoate (PAP), profenofos, propaphos, prothiofos, pyraclorfos, salithion, sulprofos, tebupirimfos, temephos, tetrachlorvinphos, terbufas, thiometon, trichlorphon (DEP), vamidothion, phorate, and cadusafos;

(2) carbamate compounds such as alanycarb, bendiocarb, benfuracarb, BPMC, carbaryl, carbofuran, carbosulfan, cloethocarb, ethiofencarb, fenobucarb, fenothiocarb, fenoxycarb, furathiocarb, isoprocarb (MIPC), metolcarb, methomyl, methiocarb, NARC, oxamyl, pirimicarb, propoxur (PHC), XMC, thiodicarb, xylylcarb, and aldicarb;

(3) synthesis pyrethroid compounds such as acrinathrin, allethrin, benfluthrin, beta-cyfluthrin, bifenthrin, cycloprothrin, cyfluthrin, cyhalothrin, cypermethrin, deltamethrin, esfenvalerate, ethofenprox, fenpropathrin, fenvalerate, flucythrinate, flufenoprox, flumethrin, fluvalinate, halfenprox, imiprothrin, permethrin, , prallethrin, pyrethrin, resmethrin, sigma-cypermethrin, silafluofen, tefluthrin, tralomethrin, transfluthrin, tetramethrin, phenothrin, cyphenothrin, alpha-cypermethrin, zeta-cypermethrin, lambda-cyhalothrin, furamethrin, taufluvalinate, 2,3,5,6-tetrafluoro-4-(methoxymethyl)benzyl (EZ)-(1RS, 3RS;1RS, 3SR)-2,2-dimethyl-3-prop-1-enylcyclopropanecarboxylate, 2,3,5,6-tetrafluoro-4-methylbenzyl (EZ)-(1RS, 3RS;1RS, 35R)-2,2-dimethyl-3-prop-1-enylcyclopropanecarboxylate, and 2,3,5,6-tetrafluoro-4-(methoxymethyl)benzyl (1RS, 3RS;1RS, 3SR)-2,2-dimethyl-3-(2-methyl-1-propenyl)cyclopropanecarboxylate;

(4) nereistoxin compound such as cartap, bensultap, thiocyclam, monosultap, and bisultap;

(5) neonicotinoid compounds such as imidacloprid, nitenpyram, acetamiprid, thiamethoxam, thiacloprid, dinotefuran, and clothianidin;

(6) benzoylurea compounds such as chlorfluazuron, bistrifluron, diafenthiuron, diflubenzuron, fluazuron, flucycloxuron, flufenoxuron, hexaflumuron, lufenuron, novaluron, noviflumuron, teflubenzuron, triflumuron, and triazuron;

(7) phenylpyrazole compounds such as acetoprole, ethiprole, fipronil, vaniliprole, pyriprole, and pyrafluprole;

(8) Bt toxin insecticides such as live spores and crystal toxins originated from Bacillus thuringiesis and a mixture thereof;

(9) hydrazine compounds such as chromafenozide, halofenozide, methoxyfenozide, and tebufenozide;

(10) organic chlorine compounds such as aldrin, dieldrin, dienochlor, endosulfan, and methoxychlor;

(11) natural insecticides such as machine oil and nicotine-sulfate; and

(12) other insecticides such as avermeotin-B, bromopropylate, buprofezin, chlorphenapyr, cyromazine, D-D, 1,3-Dichloropropene, emamectin-benzoate, fenazaquin, flupyrazofos, hydroprene, methoprene, indoxacarb, metoxadiazone, milbemycin-A, pymetrozine, pyridalyl, pyriproxyfen, spinosad, sulfluramid, tolfenpyrad, triazamate, flubendiamide, lepimectin, Arsenic acid, benclothiaz, Calcium cyanamide, Calcium polysulfide, chlordane, DOT, DSP, flufenerim, flonicamid, flurimfen, formetanate, metam-ammonium, metam-sodium, Methyl bromide, nidinotefuran, Potassium oleate, protrifenbute, spiromesifen, Sulfur, metaflumizone, spirotetramat, pyrifluquinazone, spinetoram, and chlorantraniliprole.

Examples of the acaricides (acaricidal active ingredients) include acequinocyl, amitraz, benzoximate, bifenazate, bromopropylate, chinomethionat, chlorobenzilate, CPCBS, chlorfenson, clofentezine, cyflumetofen, Kelthane (dicofol), etoxazole, fenbutatin oxide, fenothiocarb, fenpyroximate, fluacrypyrim, fluproxyfen, hexythiazox, propargite (BPPS), polynactins, pyridaben, Pyrimidifen, tebufenpyrad, tetradifon, spirodiclofen, spiromesafen, spirotetramat, amidoflumet, and cyenopyrafen.

Examples of the nematocides (nematocidal active ingredients) include DCIP, fosthiazate, levamisol hydrochloride, methylisothiocyanate, morantel tartarate, and imicyafos.

Examples of the phytotoxicity-reducing agents (active ingredients of phytotoxicity reduction) include 1, 8-naphthalic anhydride, cyometrinil, oxabetrinil, fluxofenim, flurazole, benoxacor, dichlormid, furilazole, fenclorim, daimuron, cumyluron, dimepiperate, cloquintocet-mexyl, fenchlorazole-ethyl, mefenpyr-diethyl, and isoxadifen-ethyl.

Examples of the plant growth regulators (plant growth active ingredients) include ethephon, chlormequat-chloride, and mepiquat-chloride.

High "crop growth improving effect" can be obtained effectively and labor-savingly by treating crops that have been provided with herbicide resistance in any way with the agricultural composition of the present invention, and at the same time or different time, treating the crops with a certain herbicide. As used herein, the "crop growth improving effect" means that control of insect damage, disease damage and weed damage of a crop results in an increase of the crop yield.
Specifically, a crop provided with imidazolinone herbicide resistance, for example, Clearfield® canola can be treated with the agricultural composition of the present invention and an imidazolinone herbicide such as imazapyr at the same or different time to improve the growth of Clearfield canola. Also, a crop provided with glyphosate resistance, for example, Roundup Ready® Cotton or Roundup Ready2® soybean can be treated with the agricultural composition of the present invention and glyphosate at the same or different time to improve the growth of Roundup Ready corn or Roundup Ready2 soybean. Also, a crop provided with glufosinate resistance, for example, LibertyLink® corn is treated with the agricultural composition of the present invention and glufosinate at the same or different time to improve the growth of LibertyLink cotton.

### Examples

Hereinafter, the present invention will be explained in more detail by way of Production Examples, Formulation Examples, Test Examples and so on, which the present invention is not limited to.
First, Production Examples of the present compound are shown.

### Production Example 1

To 30 ml of toluene were added 3.0 g of 2-chloroisonicotinic acid, 3.6 g of thionyl chloride and 3 drops of N,N-dimethyl formamide (DMF), and then the mixture was heated under reflux for 2 hours. The reaction mixture was cooled to around room temperature, and then concentrated under reduced pressure, so that 3.5 g of 2-chloroisonicotinyl chloride was obtained.
To 20 ml of tetrahydrofuran (THF) were added 3.5 g of 2-chloroisonicotinyl chloride, 2.4 g of cyclohexylmetrylamine and 2.5 g of triethylamine, followed by stirring at room temperature for 30 minutes. To the reaction mixture, ethyl acetate was added, followed by filtration through Celite®. The resultant filtrate was concentrated under reduced pressure. The residue was washed with a mixed solvent of tert-butyl methyl ether (MTBE) and hexane, so that 4.2 g of N-(cyclohexylmethyl)-2-chloroisonicotinamide was obtained. N-(cyclohexylmethyl)-2-chloroisonicotinamide 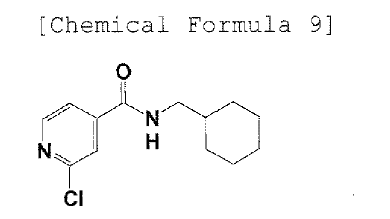 ¹H-NMR (CDCl₃) δ: 0.93-1.32 (5H, m), 1.53-1.80 (6H, m), 3.31 (2H, dd, J = 6.5, 6.4 Hz), 6.25 (1H, br s), 7.52 (1H, dd, J = 5.1, 1.5 Hz), 7.63-7.64 (1H m), 8.50 (1H, d, J = 5.1 Hz).

To 10 ml of 1,4-dioxane were added 0.42 g of N-(cyclohexylmethyl)-2-chloroisonicotinamide and 1.0 g of a 28% solution of sodium ethoxide in methanol, followed by heating at 80°C with stirring for 4 hours. The reaction mixture was cooled to around room temperature, poured into water, and then extracted with ethyl acetate. The organic layer was dried over magnesium sulfate and concentrated under reduced pressure. The residue was subjected to silica gel column chromatography, so that 0.10 g of N-(cyclohexylmethyl)-2-methoxyisonicotinamide (hereinafter, referred to as the present compound 1) was obtained. Present compound 1 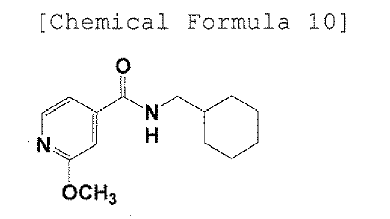 ¹H-NMR (CDCl₃) δ: 0.94-1.31 (5H, m), 1.52-1.80 (6H, m), 3.29 (2H, dd, J = 6.2, 6.3Hz), 3.97 (3H, s), 6.15 (1H br s), 7.02-7.03 (1H, m), 7.16 (1H, dd, J = 5.3, 1.3Hz), 8.25 (1H, d, J = 5.3Hz).

### Production Example 2

To a solution of 3.6 g of N-(cyclohexylmethyl)-2-chloroisonicotinamide and 3.4 g of benzyl alcohol in 30 ml of DMF was added 1.25 g of 60% sodium hydride (oily), and then the mixture was heated under reflux for 2 hours. The reaction mixture was cooled to about room temperature and then poured into water. To the mixture was added an aqueous 10% citric acid solution, followed by extraction with ethyl acetate. The organic layer was dried over magnesium sulfate and then concentrated under reduced pressure. The residue was washed with MTBE and then hexane, so that 1.6 g of N-(cyclohexylmethyl)-2-benzyloxyisonicotinamide was obtained. N-(cyclohexylmethyl)-2-benzyloxyisonicotinamide 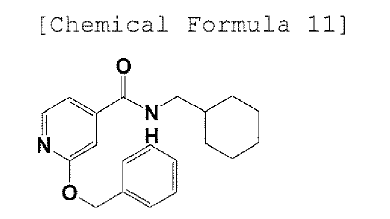 ¹H-NMR (CDCl₃) δ: 0.93-1.31 (5H, m), 1.51-1.79 (6H, m), 3.28 (2H, dd, J = 6.6, 6.2Hz), 5.41 (2H, s), 6.16 (1H, br s), 7.08 (1H, dd, J = 1.6, 0.7Hz), 7.19 (1H, dd, J = 5.3, 1.6Hz), 7.29-7.47 (5H, m), 8.25 (1H, dd, J = 5.3, 0.7Hz).

To 30 ml of methanol were added 1.6 g of N-(cyclohexylmethyl)-2-benzyloxyisonicotinamide and 50 mg of palladium carbon, and the mixture was reacted in a hydrogen atmosphere under a normal pressure. The reaction mixture was filtered through Celite® and the filtrate was concentrated under reduced pressure. The residue was washed with MTBE and then hexane, so that 1.3 g of N-(cyclohexylmethyl)-1,2-dihydro-2-oxoisonicotinamide was obtained.
N-(cyclohexylmethyl)-1,2-dihydro-2-oxoisonicotinamide 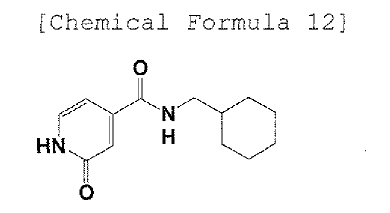 ¹H-NMR (CDCl₃) δ: 0.85-1.33 (5H, m), 1.49-1.80 (6H, m), 3.28 (2H, dd, J = 6.4, 6.3Hz), 6.14-6.19 (1H, m), 6.63 (1H, dd, J = 6.8, 1.2Hz), 6.81-6.82 (1H, m), 7.41 (1H, d, J = 6.8Hz), 12.41 (1H, br s).

To DMF are added N-(cyclohexylmethyl)-1,2-dihydro-2-oxoisonicotinamide, ethyl iodide and cesium carbonate, followed by stirring at room temperature. To the reaction solution is added water, followed by extraction with ethyl acetate. The organic layer is dried over magnesium sulfate and then concentrated under reduced pressure. The residue was subjected to silica gel column chromatography, so that N-(cyclohexylmethyl)-2-ethoxyisonicotinamide (hereinafter, referred to as the present compound 2) was obtained. Present compound 2

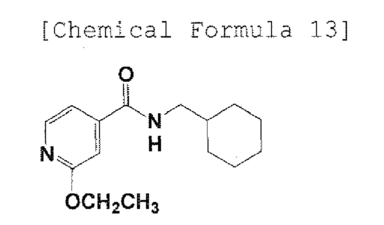

### Production Example 3

To 30 ml of a 15% solution of sodium thiomethoxide in water was added 3.3 g of N-(cyclohexylmethyl)-2-chloroisonicotinic acid amid, and the mixture was heated under reflux for 3 hours. The reaction mixture was cooled to about room temperature, and water was added thereto, followed by extraction with ethyl acetate. The organic layer was dried over magnesium sulfate and then concentrated under reduced pressure. The residue was subjected to silica gel column chromatography, so that 2.6 g of N-(cyclohexylmethyl)-2-methylthioisonicotinamide was obtained.
N-(cyclohexylmethyl)-2-methylthioisonicotinamide 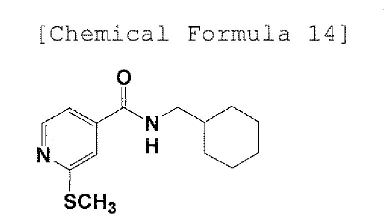 ¹H-NMR (CDCl₃) δ: 0.92-1.22 (5H, m), 1.51-1.79 (6H, m), 2.58 (3H, s), 3.28 (2H, dd, J = 6.4, 6.3Hz), 6.37 (1H, br s), 7.23 (1H, dd, J = 5.2, 1.5Hz), 7.48 (1H, dd, J = 1.5, 0.7Hz), 8.50 (1H, dd, J = 5.2, 0.7Hz).

To 20 ml of chloroform were added 2.3 g of N-(cyclohexylmethyl)-2-methylthioisonicotinamide and 4.0 g of 3-chloroperbenzoic acid (purity: 65%), followed by stirring at room temperature for 8 hours. To the reaction mixture, water and an aqueous saturated sodium thiosulfate solution were added, followed by extraction with chloroform. The organic layer was washed with an aqueous sodium hydrogen carbonate solution, dried over magnesium sulfate and then concentrated under reduced pressure. The residue was subjected to silica gel column chromatography, so that 1.5 g of N-(cyclohexylmethyl)-2-methylsulfonyl isonicotinamide was obtained.
N-(cyclohexylmethyl)-2-methylsulfonyl isonicotinamide 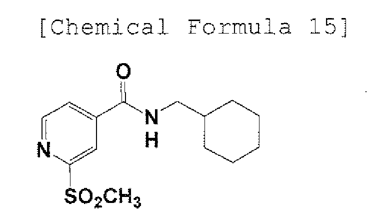 ¹H-NMR (CDCl₃) δ: 0.95-1.32 (5H, m), 1.56-1.81 (6H, m), 3.27 (3H, s), 3.34 (2H, dd, J = 6.4, 6.3Hz), 6.47 (1H, br s), 7.99 (1H, dd, J = 5.0, 1.7Hz), 8.28 (1H, dd, J = 1.7, 0.7Hz), 8.85 (1H, dd, J = 5.0, 0.7Hz).

To 3 ml of DMF were added 0.30 g of N-(cyclohexylmethyl)-2-methylsulfonyl isonicotinamide, 0.13 g of propargyl alcohol and 80 mg of 60% sodium hydride (oily), followed by stirring at room temperature for 4 hours. To the reaction mixture, an aqueous 10% citric acid solution was added, followed by extraction with ethyl acetate. The organic layer was dried over magnesium sulfate and then concentrated under reduced pressure. The residue was subjected to silica gel column chromatography, so that 0.24 g of N-(cyclohexylmethyl)-2-(2-propynyloxy)isonicotinamide (hereinafter, referred to as the present compound 3) was obtained.
Present compound 3 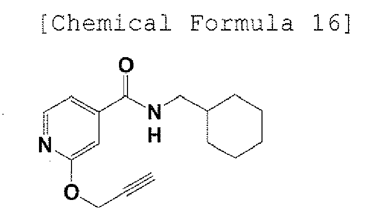 ¹H-NMR (CDCl₃) δ: 0.93-1.32 (5H, m), 1.51-1.79 (6H, m), 2.48 (1H, t, J = 2.4Hz), 3.29 (2H, dd, J = 6.5, 6.4Hz), 5.01 (2H, d, J = 2.4Hz), 6.21 (1H, br s), 7.07-7.09 (1H, m), 7.23 (1H, dd, J = 5.3, 1.4Hz), 8.26 (1H, d, J = 5.3Hz).

### Production Example 4

To 3 ml of thionyl chloride were added 0.25 g of 2-fluoroisonicotinic acid and one drop of DMF, and the mixture was heated under reflux for 1 hour. The reaction solution was cooled to about room temperature and then concentrated under a normal pressure. To the residue were added 5 ml of THF, 0.30 g of cyclohexylmethylamine and 1.0 g of triethylamine. The mixture was stirred at room temperature for 2 hours and then concentrated. The residue was subjected to silica gel column chromatography, so that 0.29 g of N-(cyclohexylmethyl)-2-fluoroisonicotinamide (hereinafter, referred to as the present compound 4) was obtained.
Present compound 4 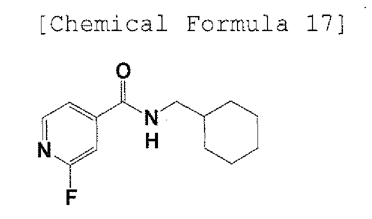 ¹H-NMR (CDCl₃) δ: 0.92-1.30 (5H, m), 1.53-1.80 (6H, m), 3.30 (2H, dd, J = 6.4, 6.3Hz), 6.62 (1H br s), 7.28-7.29 (1H m), 7.50 (1H, ddd, J = 5.3, 1.7, 1.6Hz), 8.31 (1H, d, J = 5.3Hz).

### Production Example 5

To 50 ml of toluene were added 2.5 g of 2,6-dimethoxyisonicotinic acid, 2.5 g of thionyl chloride and one drop of DMF, and the mixture was heated under reflux for 3 hours. The reaction solution was concentrated under reduced pressure, so that 2,6-dimethoxyisonicotinyl chloride was obtained. Then, the 2,6-dimethoxyisonicotinyl chloride, 2.0 g of cyclohexylmethylamine and 2.0 g of triethylamine were added to 30 ml of THF, followed by stirring at room temperature for 4 hours. The reaction mixture was concentrated under reduced pressure. The residue was subjected to silica gel column chromatography, so that 1.7 g of N-(cyclohexylmethyl)-2,6-dimethoxyisonicotinamide (hereinafter, referred to as the present compound 5) was obtained.
Present compound 5 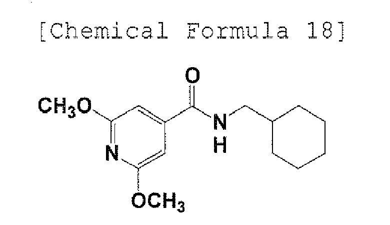 ¹H-NMR (CDCl₃) δ: 0.92-1.30 (5H, m), 1.50-1.78 (6H, m), 3.26 (2H, dd, J = 6.5, 6.4Hz), 3.93 (6H, s), 6.26 (1H, br s), 6.59 (2H, s).

### Production Example 6

According to Production Example 4,using (1S)-1,2-dimethylpropylamine in place of cyclohexylmethylamine, N-((1S)-1,2-dimethylpropyl)-2-fluoroisonicotinamide (hereinafter, referred to as the present compound 6) was obtained.

### Present compound 6

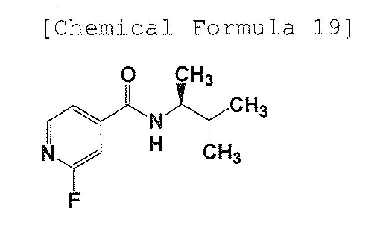

¹H-NMR (CDCl₃) δ: 0.96 (3H, d, J = 3.6Hz), 0.98 (3H, d, J = 3.6Hz), 1.20 (3H, d, J = 6.8Hz), 1.77-1.89 (1H, m), 4.02-4.15 (1H, m), 5.94-6.04 (1H, m), 7.25-7.26 (1H, m), 7.47 (1H, ddd, J = 5.3, 1.5, 1.5Hz), 8.33 (1H, d, J = 5.3Hz).

### Production Example 7

According to Production Example 4, using (1S)-1-cyclohexylethylamine in place of cyclohexylmethylamine, N-((1S)-1-cyclohexylethyl)-2-fluoroisonicotinamide (hereinafter, referred to as the present compound 7) was obtained.

### Present compound 7

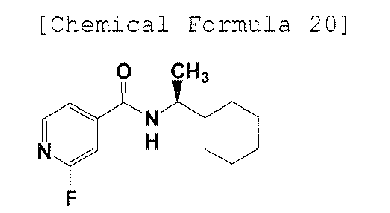

¹H-NMR (CDCl₃) δ: 0.97-1.31 (8H, m), 1.39-1.50 (1H, m), 1.64-1.83 (5H, m), 4.01-4.11 (1H, m), 6.03 (1H, d, J = 7.7Hz), 7.25-7.26 (1H, m), 7.47 (1H, ddd, J = 5.1, 1.6, 1.5Hz), 8.32 (1H,d,J = 5.1Hz).

### Production Example 8

According to Production Example 4, using 2-methylcyclohexylamine in place of cyclohexylmethylamine, N-(2-methylcyclohexyl)-2-fluoroisonicotinamide (hereinafter, referred to as the present compound 8) was obtained. Present compound 8 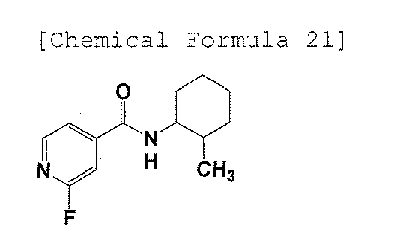 ¹H-NMR (CDCl₃) δ: 0.93-0.99 (3.0H, m), 1.08-1.84 (8.0H, m), 1.93-2.08 (1.0H, m), 3.64-3.74 (0.7H, m), 4.22-4.29 (0.3H, m), 6.07-6.28 (1.0H, m), 7.24-7.28 (1.0H, m), 7.45-7.51 (1.0H, m), 8.30-8.34 (1.0H, m).

### Production Example 9

To 5 ml of THF were added 0.22 g of (1S)-1,2-dimethylpropylamine and 0.30 g of triethylamine. To the mixture, a mixed solution of 0.44 g of 2-chloroisonicotinyl chloride and 3 ml of THF was added under ice cooling, followed by stirring for 30 minutes. To the reaction mixture was added water, followed by extraction with ethyl acetate. The organic layer was dried over magnesium sulfate and then concentrated under reduced pressure, so that 0.53 g of N-((1S)-1,2-dimethylpropyl)-2-chloroisonicotinamide was obtained. N-((1S)-1,2-dimethylpropyl)-2-chloroisonicotinamide 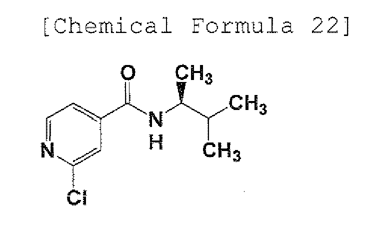 ¹H-NMR (CDCl₃) δ: 0.97 (3H, d, J = 6.8Hz), 0.98 (3H, d, J = 6.8Hz), 1.20 (3H, d, J = 6.8Hz), 1.78-1.88 (1H, m), 4.02-4.12 (1H, m), 5.94 (1H, d, J = 8.0Hz), 7.51 (1H, dd, J = 5.1,1.7Hz), 7.62 (1H, dd, J = 1.4, 0.7Hz), 8.50 (1H, dd, J = 5.1, 0.7Hz).

To 3 ml of methanol were added 0.32 g of N-((1S)-1,2-dimethylpropyl)-2-chloroisonicotinamide and 0.68 g of 28% sodium methoxide, and the mixture was heated under reflux for 16 hours. The reaction mixture was cooled to about room temperature and then concentrated under reduced pressure. To the residue was added water, followed by extraction with ethyl acetate. The organic layer was dried over sodium sulfate and then concentrated under reduced pressure, so that 0.22 g of N-((1S)-1,2-dimethylpropyl)-2-methoxyisonicotinamide (hereinafter, referred to as the present compound 9) was obtained.

### Present compound 9

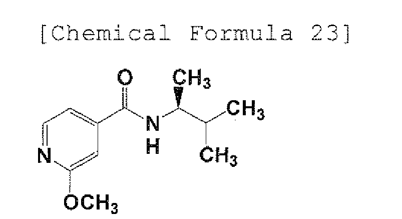

¹H-NMR (CDCl₃) δ: 0.96 (3H, d, J = 6.8Hz), 0.96 (3H, d, J = 6.8Hz), 1.18 (3H, d, J = 6.8Hz), 1.76-1.86 (1H, m), 3.97 (3H, s), 4.01-4.11 (1H, m), 5.91 (1H, d, J = 6.8Hz), 7.02 (1H, dd, J = 1.5, 0.7Hz), 7.15 (1H, dd, J = 5.4, 1.5Hz), 8.25 (1H, dd, J = 5.4, 0.7Hz).

### Production Example 10

According to Production Example 9, using (1S)-1-cyclohexylethylamine in place of (1S)-1,2-dimethylpropylamine, N-((1S)-1-cyclohexylethyl)-2-chloroisonicotinamide was obtained, and then N-((1S)-1-cyclohexylethyl)-2-methoxyisonicotinamide (hereinafter, referred to as the present compound 10) was obtained. N-((1S)-1-cyclohexylethyl)-2-chloroisonicotinamide 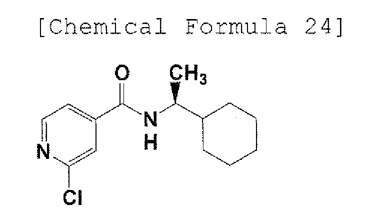 ¹H-NMR (CDCl₃) δ: 1.20-1.80 (11H, m), 1.21 (3H, d, J = 6.8Hz), 4.00-4.12 (1H, m), 5.83-5.97 (1H, m), 7.51 (1H, dd, J = 5.1, 1.5Hz), 7.61-7.62 (1H, m), 8.50 (1H, dd, J = 5.1, 0.5Hz).

### Present compound 10

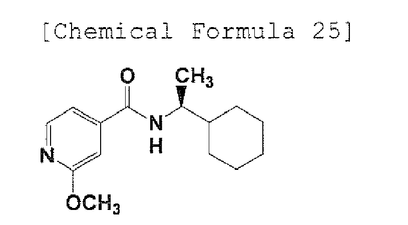

¹H-NMR (CDCl₃) δ: 1.01-1.75 (11H, m), 1.19 (3H, d, J = 7.0Hz), 3.97 (3H, s), 4.05 (1H, d, J = 8.9Hz), 5.90 (1H, d, J = 8.0Hz), 7.01-7.02 (1H, m), 7.16 (1H, dd, J = 5.3, 1.4Hz), 8.25 (1H, d, J = 5.1Hz).

### Production Example 11

According to Production Example 9, using 1,1,2-trimethylpropylamine in place of (1S)-1,2-dimethylpropylamine, N-(1,1,2-trimethylpropyl)-2-chloroisonicotinamide was obtained, and then N-(1,1,2-trimethylpropyl)-2-methoxyisonicotinamide (hereinafter, referred to as the present compound 11) was obtained. N-(1,1,2-trimethylpropyl)-2-chloroisonicotinamide 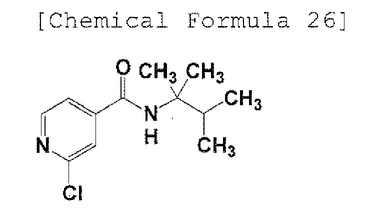 ¹H-NMR (CDCl₃) δ: 0.95 (6H, d, J = 6.8Hz), 1.40 (6H, s), 2.37-2.44 (1H, m), 5.83 (1H, br s), 7.46 (1H, dd, J = 5.1, 1.5Hz), 7.57 (1H, dd, J = 1.5, 0.7Hz), 8.48 (1H, d, J = 4.9Hz).

### Present compound 11

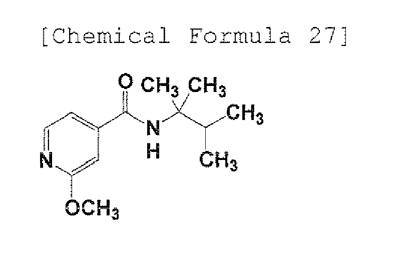

¹H-NMR (CDCl₃) δ: 0.94 (6H, d, J = 7.0Hz), 1.39 (6H, s), 2.34-2.44 (1H, m), 3.96 (3H, s), 5.82 (1H, br s), 6.97 (1H, dd, J = 1.4,0.7Hz), 7.12 (1H, dd, J = 5.3, 1.4Hz), 8.23 (1H, dd, J = 5.3,0.7Hz).

### Production Example 12

According to Production Example 9, using (1S)-1,2,2-trimethylpropylamine in place of (1S)-1,2-dimethylpropylamine, N-((1S)-1,2,2-trimethylpropyl)-2-chloroisonicotinamide was obtained, and then N-((1S)-1,2,2-trimethylpropyl)-2-methoxyisonicotinamide (hereinafter, referred to as the present compound 12) was obtained. N-((1S)-1,2,2-trimethylpropyl)-2-chloroisonicotinamide 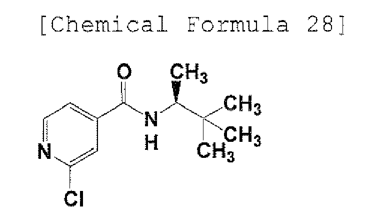 ¹H-NMR (CDCl₃) δ: 0.98 (9H,s), 1.18 (3H,d,J = 6.8Hz), 4.05-4.13 (1H, m), 5.92 (1H,d,J = 7.8Hz), 7.50 (1H,dd,J = 5.1,1.5Hz), 7.60-7.61 (1H, m), 8.51 (1H,d,J = 4.9Hz). Present compound 12 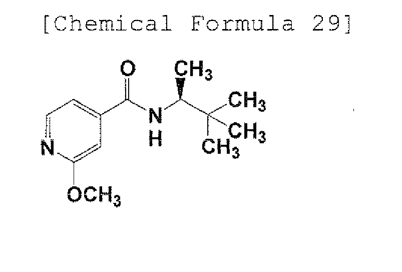 ¹H-NMR (CDCl₃) δ: 0.96 (9H, s), 1.16 (3H, d, J = 6.8Hz), 3.97 (3H, s), 4.03-4.12 (1H, m), 5.89 (1H, br s), 7.00-7.01 (1H, m), 7.15 (1H, dd, J = 5.4, 1.5Hz), 8.26 (1H, dd, J = 5.4, 0.7Hz).

### Production Example 13

According Production Example 9, using 2-methylcyclohexylamine in place of (1S)-1,2-dimethylpropylamine, N-(2-methylcyclohexyl)-2-chloroisonicotinamide was obtained, and then N-(2-methylcyclohexyl)-2-methoxyisonicotinamide (hereinafter, referred to as the present compound 13) was obtained. N-(2-methylcyclohexyl)-2-chloroisonicotinamide 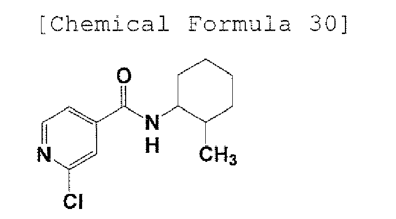 ¹H-NMR (CDCl₃) δ: 0.94 (0.9H, d, J = 6.8Hz), 0.98 (2.1H, d, J = 6.3Hz), 1.08-2.07 (9.0H, m), 3.63-3.73 (0.7H, m), 4.22-4.28 (0.3H, m), 5.99-6.21 (1.0H, m), 7.50-7.54 (1.0H, m), 7.61-7.64 (1.0H, m), 8.47-8.50 (1.0H, m).

### Present compound 13

### [Chemical Formula 31]

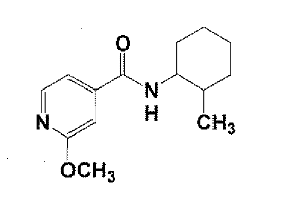

¹H-NMR (CDCl₃) δ: 0.93 (0.9H, d, J = 6.8Hz), 0.97 (2.1H, d, J = 6.6Hz), 1.13-2.04 (9.0H, m), 3.63-3.73 (0.7H, m), 3.96-3.98 (3.0H, m), 4.21-4.28 (0.3H, m), 5.84 (0.7H, d, J = 8.0Hz), 6.09 (0.3H, d, J = 7.6Hz), 7.02-7.03 (1.0H, m), 7.15-7.17 (1.0H, m), 8.24-8.27 (1.0H, m).

### Production Example 14

According to Production Example 9, using 1,2-dimethylpropylamine in place of (1S)-1,2-dimethylpropylamine, N-(1,2-dimethylpropyl)-2-chloroisonicotinamide was obtained, and then N-(1,2-dimethylpropyl)-2-methoxyisonicotinamide (hereinafter, referred to as the present compound 14) was obtained. N-(1,2-dimethylpropyl)-2-chloroisonicotinamide 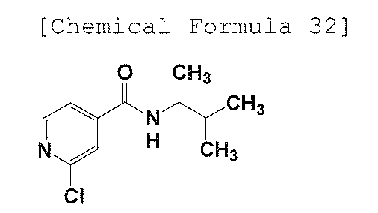 ¹H-NMR (CDCl₃) δ: 0.96 (3H, d, J = 3.7Hz), 0.98 (3H, d, J = 3.7Hz), 1.20 (3H, d, J = 6.8Hz), 1.76-1.89 (1H, m), 4.02-4.11 (1H, m), 5.90-6.01 (1H, m), 7.51 (1H, dd, J = 5.1,1.5Hz), 7.62 (1H, dd, J = 1.5, 0.7Hz), 8.50 (1H, d, J = 5.1Hz).

### Present compound 14

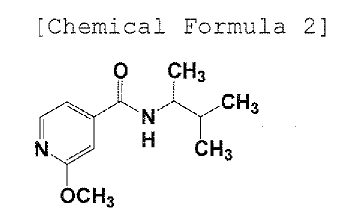

¹H-NMR (CDCl₃) δ: 0.95 (3H, d, J = 6.8Hz), 0.96 (3H, d, J = 6.8Hz), 1.18 (3H, d, J = 6.8Hz), 1.76-1.86 (1H, m), 3.97 (3H,s), 4.03-4.10 (1H, m), 5.93 (1H, d, J = 6.6Hz), 7.02 (1H, dd, J = 1.5, 0.7Hz), 7.15 (1H, dd, J = 5.4, 1.5Hz), 8.25 (1H, dd, J = 5.1, 0.7Hz).

### Production Example 15

According to Production Example 4, using 2-methylcyclopentylamine hydrochloride in place of cyclohexylmethylamine, N-(2-methylcyclopentyl)-2-fluoroisonicotinamide (hereinafter, referred to as the present compound 15) was obtained.

### Present compound 15

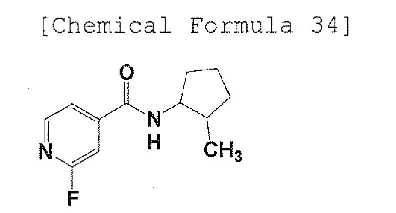

¹H-NMR (CDCl₃) δ: 0.95 (0.9H, d, J = 7.0Hz), 1.09 (2.1H, d, J = 6.8Hz), 1.29-2.28 (7.0H, m), 3.93-4.01 (0.7H, m), 4.41-4.48 (0.3H, m), 6.02-6.06 (1.0H, br m), 7.25-7.27 (1.0H, m), 7.45-7.49 (1.0H, m), 8.32-8.34 (1.0H, m).

### Production Example 16

According to Production Example 4, using cyclopentylamine in place of cyclohexylmethylamine, N-(cyclopentylmethyl)-2-fluoroisonicotinamide (hereinafter, referred to as the present compound 16) was obtained. Present compound 16 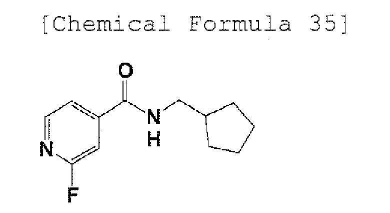 ¹H-NMR (CDCl₃) δ: 1.22-1.32 (3H, m), 1.55-1.86 (5H, m), 2.12-2.21 (1H, m), 3.41 (2H, dd, J = 7.2, 5.8Hz), 6.25 (1H, br s), 7.26-7.27 (1H, m), 7.47-7.49 (1H, m), 8.33 (1H, d,J = 5.1Hz).

### Production Example 17

According to Production Example 4, using 1-cyclopentylethylamine in place of cyclohexylmethylamine, N-(1-cyclopentylethyl)-2-fluoroisonicotinamide (hereinafter, referred to as the present compound 17) was obtained. Present compound 17 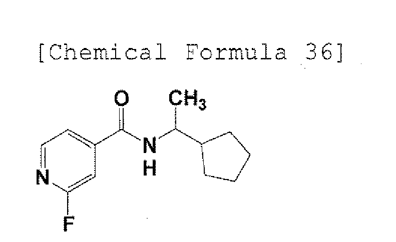 ¹H-NMR (CDCl₃) δ: 1.25 (3H, d, J = 6.5Hz), 1.27-1.38 (2H, m), 1.55-1.85 (6H, m), 1.88-1.97 (1H, m), 4.03-4.12 (1H, m), 5.92-5.95 (1H, m), 7.24-7.25 (1H, m), 7.45-7.47 (1H, m), 8.33 (1H, d, J = 5.1Hz).

### Production Example 18

According to Production Example 4, using cyclohexylamine in place of cyclohexylmethylamine, N-(cyclohexyl)-2-fluoroisonicotinamide (hereinafter, referred to as the present compound 18) was obtained.

### Present compound 18

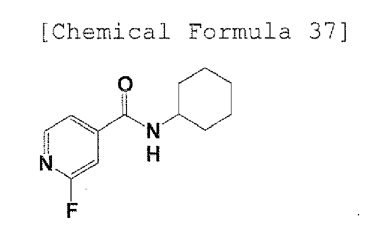

¹H-NMR (CDCl₃) δ: 1.20-1.30 (3H, m), 1.37-1.49 (2H, m), 1.66-1.81 (3H, m), 2.02-2.06 (2H, m), 3.93-9.02 (1H, m), 5.95-5.97 (1H, m), 7.25-7.26 (1H, m), 7.45-7.47 (1H, m), 8.33 (1H, d, J = 5.1Hz).

### Production Example 19

According to Production Example 4, using 2-cyclohexylethylamine in place of cyclohexylmethylamine, N-(2-cyclohexylethyl)-2-fluoroisonicotinamide (hereinafter, referred to as the present compound 19) was obtained. Present compound 19 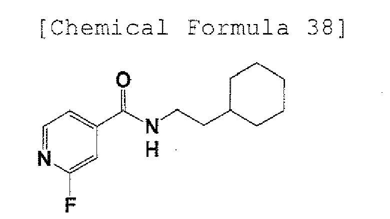 ¹H-NMR (CDCl₃) δ: 0.89-1.01 (2H, m), 1.09-1.40 (4H, m), 1.49-1.54 (2H, m), 1.62-1.77 (5H, m), 3.45-3.51 (2H, m), 6.38 (1H, br s), 7.27-7.28 (1H, m), 7.48 (1H, ddd, J = 5.3, 1.7, 1.6Hz), 8.32 (1H, ddd, J = 5.3, 0.8, 0.7Hz).

### Production Example 20

According to Production Example 4, using 3-cyclohexylpropylamine in place of cyclohexylmethylamine, N-(3-cyclohexylpropyl)-2-fluoroisonicotinamide (hereinafter, referred to as the present compound 20) was obtained. Present compound 20 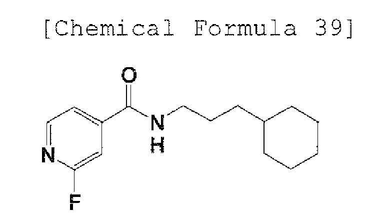 ¹H-NMR (CDCl₃) δ: 0.83-0.95 (2H, m), 1.08-1.29 (6H, m), 1.59-1.74 (7H, m), 3.41-3.97 (2H, m), 6.25 (1H, br s), 7.26-7.27 (1H, m), 7.47 (1H, ddd, J = 5.1, 1.7, 1.6Hz), 8.33 (1H, d, J = 5.1Hz).

### Production Example 21

According to Production Example 4, using (1-hydroxycyclohexyl)methylamine hydrochloride in place of cyclohexylmethylamine, N-(1-hydroxycyclohexyl)methyl-2-methoxyisonicotinamide (hereinafter, referred to as the present compound 21) was obtained.

### Present compound 21

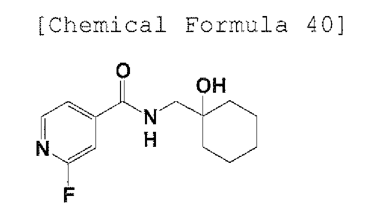

¹H-NMR (CDCl₃) δ: 1.31-1.60 (10H, m), 2.32 (1H,s), 3.49 (2H, d, J = 5.9Hz), 6.87-6.89 (1H, m), 7.31-7.31 (1H, m), 7.51-7.53 (1H, m), 8.31 (1H, d, J = 5.1Hz).

### Production Example 22

To 5 ml of toluene were added 1.0 g of 2,6-difluoroisonicotinic acid, 1.3 ml of thionyl chloride and one drop of DMF, and the mixture was heated under reflux for 4 hours. The reaction mixture wasvcooled to about room temperature and then concentrated under reduced pressure, so that 6 ml of a solution of 2,6-difluoroisonicotinyl chloride in toluene was obtained.
To 3 ml of ethyl acetate were added 1 ml of the solution of 2,6-difluoroisonicotinyl chloride in toluene obtained by the above step, 0.16 g of cyclohexylmethylamine and 0.2 ml of triethylamine, followed by stirring at room temperature for 3 hours. Then, the reaction mixture was concentrated under reduced pressure. The residue was subjected to silica gel column chromatography, so that 0.22 g of N-(cyclohexylmethyl)-2,6-diflaoroisonicotinamide (hereinafter, referred to as the present compound 22) was obtained.

### Present compound 22

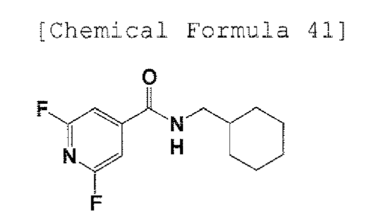

¹H-NMR (CDCl₃) δ: 0.95-1.03 (2H, m), 1.15-1.30 (3H, m), 1.54-1.77 (6H, m), 3.30 (2H, dd, J = 6.8, 6.0Hz), 6.44-6.46 (1H, m), 7.16 (2H, s).

### Production Example 23

According to Production Example 22, using (1S)-1-cyclohexylethylamine in place of cyclohexylmethylamine, N-((1S)-1-cyclohexylethyl)-2,6-difluoroisonicotinamide (hereinafter, referred to as the present compound 23) was obtained.

### Present compound 23

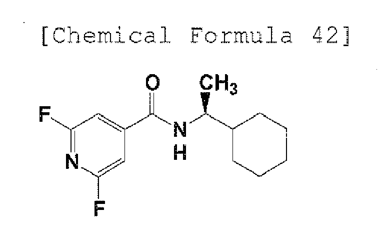

¹H-NMR (CDCl₃) δ: 1.01-1.27 (8H, m), 1.40-1.46 (1H, m), 1.67-1.80 (5H, m), 4.01-4.10 (1H, m), 5.90-5.92 (1H, m), 7.13 (2H, s).

### Production Example 24

According to Production Example 22, using 2-methylcyclohexylamine in place of cyclohexylmethylamine, N-(2-methylcyclohexyl)-2,6-difluoroisonicotinamide (hereinafter, referred to as the present compound 24) was obtained.

### Present compound 24

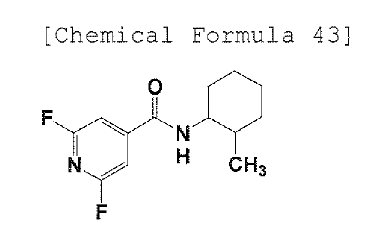

¹H-NMR (CDCl₃) δ: 0.93-0.99 (3.0H, m), 1.02-2.05 (9.0H, m), 3.63-3.72 (0.7H, m), 4.21-4.27 (0.3H, m), 6.24-6.27 (1.0H, m), 7.13 (0.6H, s), 7.17 (1.4H, s).

### Production Example 25

According to Production Example 22, using 1,2-dimethylpropylamine in place of cyclohexylmethylamine, N-(1,2-dimethylpropyl)-2,6-difluorcisonicotinamide (hereinafter, referred to as the present compound 25) was obtained.

### Present compound 25

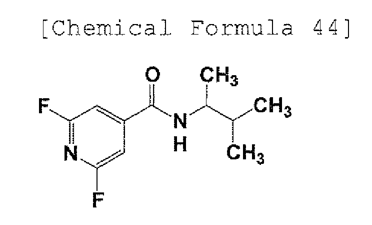

¹H-NMR (CDCl₃) δ: 0.96 (3H, d, J = 3.4Hz), 0.98 (3H, d, J = 3.4Hz), 1.20 (3H, d, J = 6.8Hz), 1.78-1.87 (1H, m), 4.01-4.09 (1H, m), 6.10 (1H, d, J = 6.6Hz), 7.14 (2H,s).

### Production Example 26

According to Production Example 22, using cyclohexylamine in place of cyclohexylmethylamine, N-(cyclohexyl)-2,6-difluoroisonicotinamide (hereinafter, referred to as the present compound 26) was obtained. Present compound 26 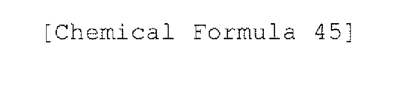 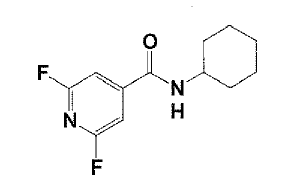 ¹H-NMR (CDCl₃) δ: 1.14-1.46 (5H, m), 1.66-1.80 (3H, m), 1.99-2.05 (2H, m), 3.89-3.99 (1H, m), 6.31-6.33 (1H, m), 7.15 (2H,s).

### Production Example 27

According to Production Example 22, using (1S)-1,2-dimethylpropylamine in place of cyclohexylmethylamine, N-((1S)-1,2-dimethylpropyl)-2,6-difluoroisonicotinamide (hereinafter, referred to as the present compound 27) was obtained.

### Present compound 27

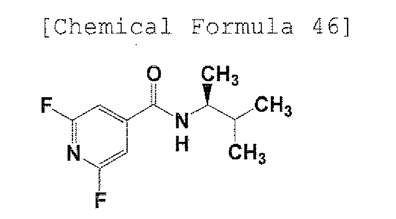

¹H-NMR (CDCl₃) δ: 0.96 (3H, d, J = 3.2Hz), 0.98 (3H, d, J = 3.4Hz), 1.20 (3H, d, J = 6.8Hz), 1.78-1.87 (1H, m), 4.01-4.08 (1H, m), 6.08 (1H, d, J = 7.6Hz), 7.14 (2H, s).

### Production Example 28

According to Production Example 22, using 2-methylcyclopentylamine hydrochloride in place of cyclohexylmethylamine, N-(2-methylcyclcpentyl)-2,6-difluoroisonicotinamide (hereinafter, referred to as the present compound 28) was obtained.

### Present compound 28

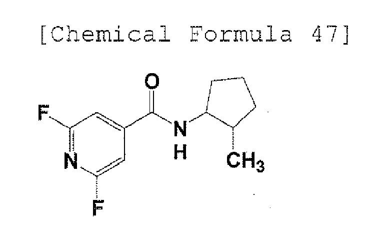

¹H-NMR (CDCl₃) δ: 0.95 (1.2H, d, J = 7.0Hz), 1.08 (1.8H, d, J = 6.5Hz), 1.24-1.96 (6.0H, m), 2.04-2.30 (1.0H, m), 3.91-4.00 (0.6H, m), 4.39-4.46 (0.4H, m), 6.16-6.26 (1.0H, m), 7.14 (0.8H, s), 7.16 (1.2H, s).

### Production Example 29

According to Production Example 22, using (1S)-1,2,2-trimethylpropylamine in place of cyclohexylmethylamine, N-((1S)-1,2,2-trimethylpropyl)-2,6-difluoroisonicotinamide (hereinafter, referred to as the present compound 29) was obtained.

### Present compound 29

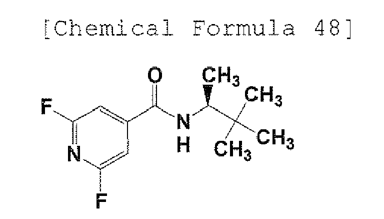

¹H-NMR (CDCl₃) δ: 0.97 (9H, s), 1.18 (3H, d, J = 6.8Hz), 4.04-4.13 (1H, m), 6.02 (1H, d, J = 8.2Hz), 7.12 (2H,s).

### Production Example 30

According to Production Example 4, using cyclobutylmethylamine in place of cyclohexylmethylamine, N-(cyclobutylmethyl)-2-fluoroisonicotinamide (hereinafter, referred to as the present compound 30) was obtained. Present compound 30 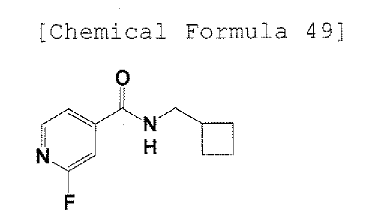 ¹H-NMR (CDCl₃) δ: 1.69-1.79 (2H, m), 1.88-2.00 (2H, m), 2.06-2.7.4 (2H, m), 2.53-2.64 (1H, m), 3.48 (2H, dd, J = 7.2, 6.0Hz), 6.60 (1H, br s), 7.27-7.29 (1H, m), 7.48-7.50 (1H, m), 8.30 (1H, dd, J = 5.2, 0.6Hz).

### Production Example 31

According to Production Example 4, using 1-cyclobutylethylamine hydrochloride in place of cyclohexylmethylamine, N-(1-cyclobutylethyl)-2-fluoroisonicotinamide (hereinafter, referred to as the present compound 31) was obtained.

### Present compound 31

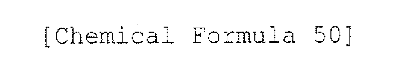

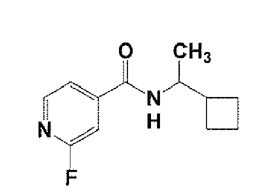

¹H-NMR (CDCl₃) δ: 1.14 (3H, d, J = 6.8Hz), 1.71-1.93 (4H, m), 1.95-2.09 (2H, m), 2.31-2.41 (1H, m), 4.10-4.19 (1H, m), 6.44 (1H, d,J = 8.0Hz), 7.27-7.28 (1H, m), 7.48-7.51 (1H, m), 8.28 (1H, d, J = 5.1Hz).

### Production Example 32

According to Production Example 4, using 1-cyclopropylethylamine in place of cyclohexylmethylamine, N-(1-cyclopropylethyl)-2-fluoroisonicotinamide (hereinafter, referred to as the present compound 32) was obtained. Present compound 32 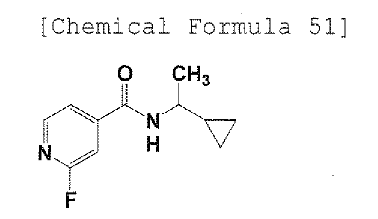 ¹H-NMR (CDCl₃) δ: 0.25-0.31 (1H, m), 0.36-0.60 (3H, m), 0.88-0.97 (1H, m), 1.31 (3H, d, J = 6.8Hz), 3.50-3.59 (1H, m), 6.88 (1H, d, J = 7.0Hz), 7.30-7.31 (1H, m), 7.52-7.54 (1H, m), 8.28 (1H, d, J = 5.1Hz).

### Production Example 33

According to Production Example 4, using cyclopropylmethylamine in place of cyclohexylmethylamine, N-(cyclopropylmethyl)-2-fluoroisonicotinamide (hereinafter, referred to as the present compound 33) was obtained.

### Present compound 33

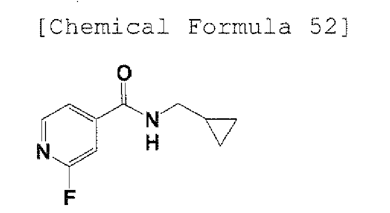

¹H-NMR (CDCl₃) δ: 0.26-0.30 (2H, m) 0.55-0.59 (2H, m), 1.01-1.13 (1H, m), 3.32 (2H, dd, J = 7.2,5.6Hz), 6.78 (1H, br s), 7.31-7.32 (1H, m), 7.52-7.54 (1H, m), 8.31 (1H, d, J = 5.3Hz).

### Production Example 34

According to Production Example 4, using 1-(1-hydroxycyclohexyl)ethylamine hydrochloride in place of cyclohexylmethyl amine, N-(1-(1-hydroxycyclohexyl)ethyl)-2-fluoroisonicotinamide (hereinafter, referred to as the present compound 34) was obtained.

### Present compound 34

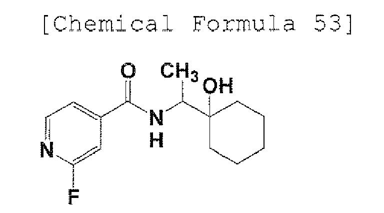

¹H-NMR (CDCl₃) δ: 1.20-1.67 (12H, m), 2.43-2.50 (2H, m), 4.14-4.22 (1H m), 6.91 (1H, d, J = 9.0Hz), 7.29-7.33 (1H, m), 7.53 3 (1H, ddd, J = 5.3, 1.7, 1.5Hz), 8.30 (1H, d, J = 5.3Hz).

### Production Example 35

According to Production Example 4, using 2-chlorocyclohexylamine hydrochloride in place of cyclohexylmethylamine, N-(2-chlorocyclohexyl)-2-fluoroisonicotinamide (hereinafter referred to as the present compound 35) was obtained.

### Present compound 35

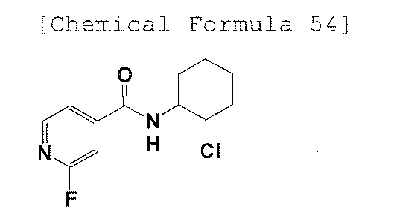

¹H-NMR (CDCl₃) δ: 1.18-1.50 (3H, m), 1.74-1.87 (3H, m), 2.28-2.35 (2H, m), 3.89 (1H, td, J = 10.7, 4.2Hz), 4.00-4.09 (1H, m), 6.39 (1H, d, J = 6.8Hz), 7.30-7.31 (1H, m), 7.51-7.53 (1H, m), 8.34 (1H, d, J = 5.3Hz).

### Production Example 36

According to Production Example 4, using 2-(1-methylethyl)cyclohexylamine in place of cyclohexylmethylamine, two isomers of N-(2-(1-methylethyl)cyclohexyl)-2-fluoroisonicotinamide (hereinafter, referred to as the present compounds 36 and 37, respectively) each showing spectra shown below.

### Present compound 36

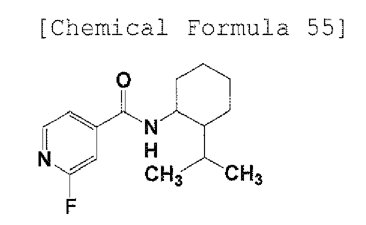

¹H-NMR (CDCl₃) δ: 0.85 (3H, d, J = 7.0Hz), 0.93 (3H, d, J = 6.8Hz), 1.08-2.11 (10H, m), 3.97 (1H, ddd, J = 20.4, 10.7, 4.0Hz), 5.84 (1H, d, J = 8.7Hz), 7.25-7.27 (1H, m), 7.45-7.49 (1H, m), 8.32-8.35 (1H, m).

### Present compound 37

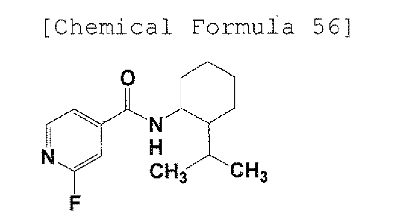

¹H-NMR (CDCl₃) δ: 0.76-2.10 (16H, m), 4.53-4.59 (1H, m) 6.22 (1H, d, J = 7.7Hz), 7.23-7.25 (1H, m), 7.44-7.48 (1H, m), 8.34 (1H, d, J = 5.1Hz).

### Production Example 37

To 1 ml of 1,4-dioxane were added 0.23 g of N-(cyclohexylmethyl)-2-fluoroisonicotinamide and 1 ml of 28% ammonia water, and the mixture was reacted in a microwave reaction apparatus (manufactured by CEM Co. under the trade name of Discover) at 18 kgf/cm² and 150°C for 50 minutes. The reaction mixture was cooled to about room temperature and then concentrated under reduced pressure. The residue was subjected to silica gel column chromatography, so that 0.22 g of N-(cyclohexylmethyl)-2-aminoisonicotonic acid amide (hereinafter, referred to as the present compound 38) was obtained.

### Present compound 38

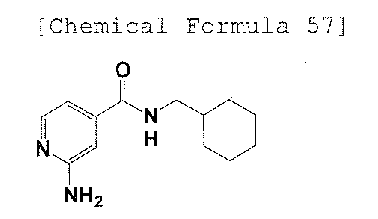

¹H-NMR (CDCl₃) δ: 0.94-1.04 (2H, m), 1.12-1.31 (3H, m), 1.52-1.78 (6H, m), 3.28 (2H, dd, J = 6.5, 6.3Hz), 4.59 (2H, s), 6.12-6.13 (1H, m), 6.83 (1H, dd, J = 5.3, 1.4Hz), 6.86-6.87 (1H, m), 8.15 (1H, dd, J = 5.3, 0.7Hz).

### Production Example 38

According to Production Example 37, using N-((1S)-1-cyclohexylethyl)-2-fluoroisonicotinamide in place of N-(cyclohexylmethyl)-2-fluoroisonicotinamide, N-((1S)-1-cyclohexylethyl)-2-aminoisonicotonic acid amide (hereinafter, referred to as the present compound 39) was obtained.

### Present compound 39

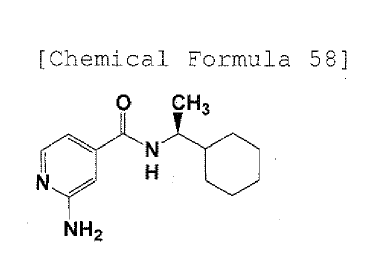

¹H-NMR (CDCl₃) δ: 0.98-1.26 (8H, m), 1.38-1.45 (1H, m), 1.66-1.80 (5H, m), 4.00-4.07 (1H, m), 4.62 (2H, s), 5.89 (1H, d, J = 7.6Hz), 6.83 (1H, dd, J = 5.2, 1.3Hz), 6.86-6.86 (1H, m), 8.14 (1H, d, J = 5.1Hz).

### Production Example 39

According to Production Example 37, using N-(2-methylcyclohexyl)-2-fluoroisonicotinamide in place of N-(cyclohexylmethyl)-2-fluoroisonicotinamide, N-(2-methylcyclohexyl)-2-aminoisonicotonic acid amide (hereinafter, referred to as the present compound 40) was obtained.

### Present compound 40

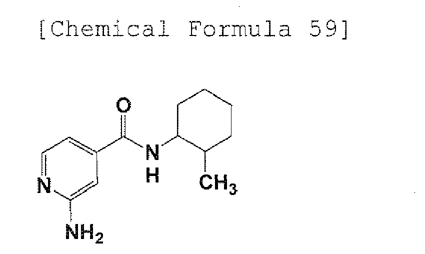

¹H-NMR (CDCl₃) δ: 0.93 (0.9H, d, J = 7.0Hz), 0.97 (2.1H, d, 1.66-1.80 (5H, m), 4.00-4.07 (1H, m), 4.62 (2H, s), 5.89 (1H, d, J = 7.6Hz), 6.83 (1H, dd, J = 5.2, 1.3Hz), 6.86-6.86 (1H, m), 8.14 (1H, d, J = 5.1Hz). 8.2Hz), 6.08 (0.3H, d, J = 7.2Hz), 6.82-6.87 (2.0H, m), 8.14-8.16 (1.0H, m).

### Production Example 40

To 4 ml of an aqueous 40% methylamine solution was added 0.30 g of N-(cyclohexylmethyl)-2-chloroisonicotinamide, and the mixture was reacted in a microwave reaction apparatus (manufactured by CEM Co. under the trade name of Discover) at 18 kgf/cm² and 150°C for 10 minutes. The reaction mixture was cooled to about room temperature and then concentrated. The residue was subjected to silica gel column chromatography, so that 0.29 g of N-(cyclohexylmethyl)-2-methylaminoisonicotonic acid amide (hereinafter, referred to as the present compound 41) was obtained.

### Present compound 41

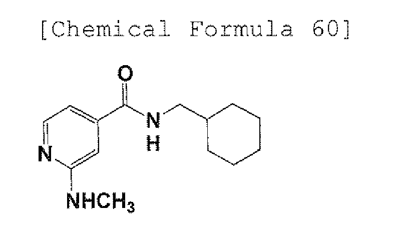

¹H-NMR (CDCl₃) δ: 0.93-1.32 (5H, m), 1.52-1.81 (6H, m), 2.96 (3H, d, J = 5.3Hz), 3.29 (2H, dd, J = 6.4, 6.3Hz), 4.67-4.75 (1H, br m), 6.17 (1H, br s), 6.73 (1H, dd, J = 5.2, 1.4Hz), 6.78 (1H, s), 8.15 (1H, d, J = 5.2Hz).

### Production Example 41

To 4 ml of an aqueous 40% methylamine solution was added 0.40 g of N-(2-methylcyclohexyl)-2-chloroisonicotinamide, and the mixture was reacted in a microwave reaction apparatus (manufactured by CEM Co. under the trade name of Discover) at 18 kgf/cm² and 150°C for 10 minutes. The reaction mixture was cooled to about room temperature and then concentrated under reduced pressure. The residue was subjected to silica gel column chromatography, so that 0.42 g of N-(2-methylcyclohexyl)-2-methylaminoisonicotonic acid amide (hereinafter, referred to as the present compound 42) was obtained.

### Present compound 42

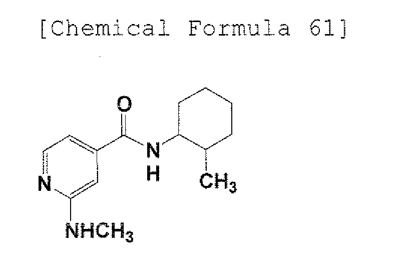

¹H-NMR (CDCl₃) δ: 0.93 (0.9H, d, J = 7.0Hz), 0.98 (2.1H, d, J = 6.5Hz), 1.09-2.06 (9.0H, m), 2.95-2.98 (3.0H, m), 3.63-3.73 (0.7H, m), 4.21-4.28 (0.3H, m), 4.70-4.77 (1.0H, m), 5.83-5.90 (0.7H, m), 6.08-6.16 (0.3H, m), 6.72-6.78 (2.0H, m), 8.14-8.18 (1.0H, m).

### Production Example 42

To 4 ml of an aqueous 50% dimethylamine solution was added 0.30 g of N-(cyclohexylmethyl)-2-chloroisonicotinamide, and the mixture was reacted in a microwave reaction apparatus (manufactured by CEM Co. under the trade name of Discover) at 18 kgf/cm² and 150°C for 10 minutes. The residue was subjected to silica gel column chromatography, so that 0.35 g of N-(cyclohexylmethyl)-2-dimethylaminoisonicotonic acid amide (hereinafter, referred to as the present compound 43) was obtained.

### Present compound 43

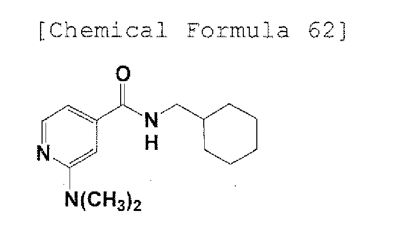

¹H-NMR (CDCl₃) δ: 0.93-1.31 (5H, m), 1.51-1.80 (6H, m), 3.10-3.14 (6H, m), 3.29 (2H, dd, J = 6.4, 6.3Hz), 6.20 (1H, br s), 6.67 (1H, d, J = 5.3Hz), 6.93 (1H, s), 8.21 (1H, dd, J = 5.3, 0.7Hz).

### Production Example 43

To 4 ml of an aqueous 50% dimethylamine solution was added 0.40 g of N-(2-methylcyolahexyl)-2-chloroisonicotinamide, and the mixture was reacted in a microwave reaction apparatus (manufactured by CEM Co. under the trade name of Discover) at 18 kgf/cm² and 150°C for 10 minutes. The reaction mixture was cooled to about room temperature and then concentrated under reduced pressure. The residue was subjected to silica gel column chromatography, so that 0.47 g of N-(2-methylcyclohexyl)-2-dimethylaminoisonicotonic acid amide (hereinafter, referred to as the present compound 44) was obtained.

### Present compound 44

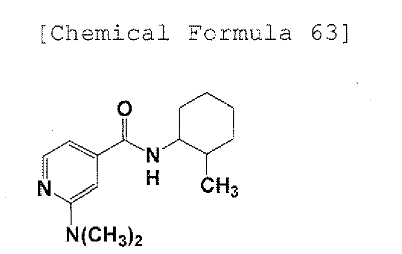

¹H-NMR (CDCl₃) δ: 0.93 (0.9H, d, J = 7.0Hz), 0.98 (2.1H, d, J = 6.5Hz), 1.09-2.08 (9.0H, m), 3.12-3.13 (6.0H m), 3.63-3.74 (0.7H, m), 4.22-4.28 (0.3H, m), 5.87 (0.7H, d, J = 8.2Hz), 6.12 (0.3H, d, J = 7.7Hz), 6.65-6.67 (1.0H, m) 6.91-6.94 (1.0H, m), 8.20-8.24 (1.0H, m).

### Production Example 44

To a mixture of 1 ml of tetrahydrofuran, 0.14 g of N-(cyclohexylmethyl)-2-aminoisonicotonic acid amide and 0.28 g of pyridine, 0.5 g of acetyl chloride was added dropwise under ice cooling. The mixture was stirred at room temperature for 12 hours. Then, the reaction mixture was concentrated under reduced pressure. The residue was subjected to silica gel column chromatography, so that 0.14 g of N-(cyclohexylmethyl)-2-(methylcarbonylamino)isonicotinamide (hereinafter, referred to as the present compound 45) was obtained.

### Present compound 45

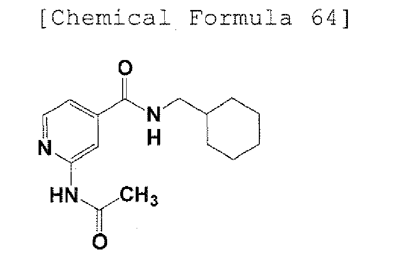

¹H-NMR (CDCl₃) δ: 0.94-1.03 (2H, m), 1.11-1.31 (3H, m), 1.54-1.79 (6H, m), 2.24 (3H,s), 3.30 (2H, dd, J = 6.5, 6.5Hz), (1H,s), 7.51 (1H, dd, J = 5.1, 1.4Hz), 8.28 (1H, br s), 8.37 (1H, d, J = 5.3Hz), 8.43 (1H,s).

### Production Example 45

To a mixture of 1 ml of tetrahydrofuran, 0.19 g of N-(cyclohexylmethyl)-2-aminonsonicotonic acid amide and 0.22 ml of triethylamine, 0.08 g of acetyl chloride was added dropwise under ice cooling, and the mixture was stirred at room temperature for 10 minutes. Then, ethyl acetate was added to the reaction mixture, followed by filtration through Gelite®. The filtrate was concentrated under reduced pressure and the residue was subjected to silica gel column chromatography, so that 0.11 g of N-(cyclohexylmethyl)-2-(di(methylcarbonyl)amino)isonicotinamide (hereinafter, referred to as the present compound 46) was obtained.

### Present compound 46

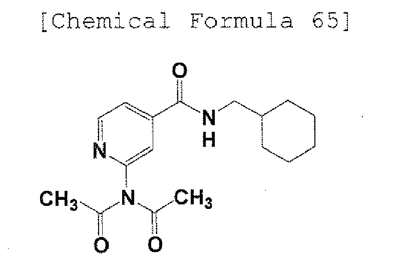

¹H-NMR (CDGl₃) δ: 0.94-1.04 (2H, m), 1.10-1.31 (3H, m), 1.52-1.63 (1H, m), 1.68-1.79 (5H, m), 2.30 (6H, s), 3.28 (2H, dd, J = 6.7, 6.2Hz), 6.44 (1H, br s), 7.54-7.54 (1H, m), 7.63 (1H, dd, J = 4.9, 1.5Hz), 8.66-8.67 (1H, m).

### Production Example 46

To ml of 1,4-dioxane were added 0.14 g of N-(cyclohexylmethyl)-2,6-difluoroisonicotinamide and 1 ml of 28% ammonia water, and the mixture was reacted in a microwave reaction apparatus (manufactured by CEM Co. under the trade name of Discover) at 18 kgf/cm² and 120°C for 5 minutes. The reaction mixture was cooled to about room temperature and then concentrated under reduced pressure. The residue was subjected to silica gel column chromatography, so that 0.10 g of N-(cyclohexylmethyl)-2-amino-6-fluoroisonicotinamide (hereinafter, referred to as the present compound 47) was obtained.

### Present compound 47

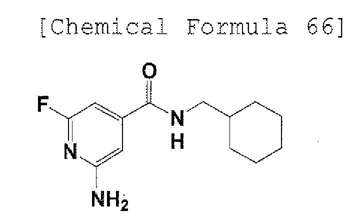

¹H-NMR (CDCl₃) δ: 0.95-1.04 (2H, m), 1.16-1.30 (3H, m), 1.52-1.60 (1H, m), 1.67-1.77 (5H, m), 3.28 (2H, dd, J = 6.5, 6.3Hz), 4.67 (2H,s), 6.05 (1H, br s), 6.41-6.42 (1H, m), 6.68-6.68 (1H, m).

### Production Example 47

According to Production Example 44, using N-((1S)-1-cyclohexylethyl)-2-aminoisonicotonic acid amide in place of N-(2-methylcyclohexyl)-2-aminoisonicotonic acid amide, N-(1-cyclohexylethyl)-2-(methylcarbonylamino)isonicotinamide (hereinafter, referred to as the present compound 48) was obtained.

### Present compound 48

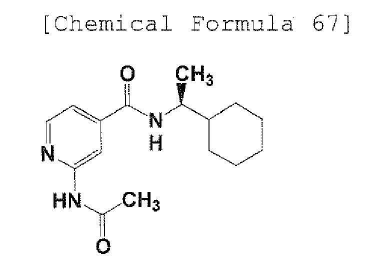

¹H-NMR (CDCl₃) δ: 0.72-1.79 (14H, m), 2.25 (3H,s), 4.01-4.09 (1H, m), 6.23 (1H, br s), 7.52-7.54 (1H, m), 8.04-8.06 (1H, m), 8.28 (1H, d, J = 5.1Hz), 8.50 (1H, s).

### Production Example 48

To DMF are added N-(cyclohexylmethyl)-1,2-dihydro-2-oxoisonicotinamide, methyl iodide and cesium carbonate, followed by stirring at room temperature. To the reaction mixture, water is added, followed by extraction with ethyl acetate. The organic layer is dried over magnesium sulfate and concentrated under reduced pressure. The residue is subjected to silica gel column chromatography, so that N-(cyclohexylmethyl)-2-methoxyisonicotinamide (present compound 1) was obtained.

### Production Example 49 9

To 1 ml of 1,4-dioxane were added 0.28 g of N-((1S)-1-cyclohexylethyl)-2,6-difluoroisonicotinamide and 1 ml of 28% ammonia water, and the mixture was reacted in a microwave reaction apparatus (manufactured by CEM Co. under the trade name of Discover) at 18 kgf/cm² and 120°C for 7 minutes. The reaction mixture was cooled to about room temperature and then concentrated under reduced pressure. A precipitated solid was collected by filtration, washed with water and then hexane, and dried, so that 0.16 g of N-((1S)-1-cyclohexylethyl)-2-amino-6-fluoroisonicotinamide (hereinafter, referred to as the present compound 49) was obtained.

### Present compound 49

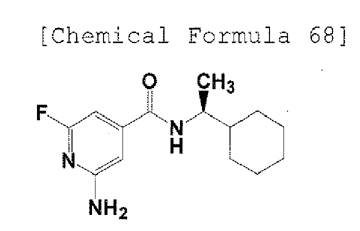

¹H-NMR (CDCl₃) δ: 0.99-1.29 (8H, m), 1.37-1.46 (1H, m), 1.63-1.79 (5H, m), 3.98-4.07 (1H, m), 4.70 (2H, br s), 5.84 (1H,d,J = 7.7Hz), 6.41 (1H,dd,J = 1.7,1.0Hz), 6.67 (1H,dd,J = 1.6,1.1Hz).

### Production Example 50

To 1 ml of 1,4-dioxane were added 0.23 g of N-(2-methylcyclohexyl)-2,6-difluoroisonicotinamide and 1 ml of 28% ammonia water, and the mixture was reacted in a microwave reaction apparatus (manufactured by CEM Co. under the trade name of Discover) at 18 kgf/cm² and 120°C for 7 minutes. The reaction mixture was cooled to about room temperature and then concentrated under reduced pressure. A precipitated solid was collected by filtration, washed with water and then hexane, and dried, so that 0.15 g of N-(2-methylcyclohexyl)-2-amino-6-fluoroisonicotinamide (hereinafter, referred to as the present compound 50) was obtained.

### Present compound 50

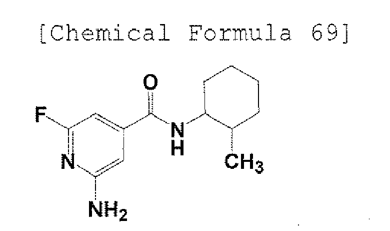

¹H-NMR (CDCl₃) δ: 0.93 (1.2H, d, J = 6.8Hz), 0.97 (1.8H, d, J = 6.3Hz), 1.12-1.79 (8.0H, m), 1.94-2.06 (1.0H, m), 3.65-3.70 (0.6H, m), 4.20-4.24 (0.4H, m), 4.68 (2.0H, br s), 5.75 (0.6H, d, J = 5.1Hz), 6.01 (0.4H, d, J = 6.8Hz), 6.41 (1.0H, br s), 6.67-6.68 (1.0H, m).

### Production Example 51

To 4 ml of DMF were added 0.30 g of 2-aminoisonicotinic acid, 0.30 g of 1-cyclobutylethylamine hydrochloride, 0.38 g of 1-hydroxybenzotriazole, 0.30 g of triethylamine and 0.54 g of WSC, followed by stirring at room temperature for 1 day. To the reaction mixture, water was added, followed by extraction with ethyl acetate. The organic layer was washed with water and then an aqueous sodium chloride solution, dried over magnesium sulfate, and then concentrated under reduced pressure. The residue was subjected to silica gel column chromatography, so that 0.22 g of N-(1-cyclobutylethyl)-2-aminoisonicotonic acid amide (hereinafter, referred to as the present compound 51) was obtained.

### Present compound 51

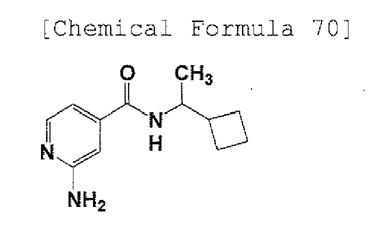

¹H-NMR (CDCl₃) δ: 1.13 (3H, d, J = 6.5Hz), 1.73-2.09 (6H, m), 2.28-2.39 (1H, m), 4.10-4.19 (1H, m), 4.58 (2H,s), 5.72-5.81 (1H, m), 6.81 (1H, dd, J = 5.2, 1.4Hz), 6.85-6.86 (1H, m), 8.14 1H, dd, J = 5.2, 0.6Hz).

### Production Example 52

To 4 ml of DMF were added 0.30 g of 2-aminoisonicotinic acid, 0.43 g of (1-hydroxycyclohexyl)methylamine hydrochloride, 0.38 g of 1-hydroxybenzotriazole, 0.30 g of triethylamine and 0.54 g of WSC, followed by stirring at room temperature for 1 day.
To the reaction solution, water was added, followed by extraction with ethyl acetate. The organic layer was washed with water and then an aqueous sodium chloride solution, dried over magnesium sulfate, and then concentrated under reduced pressure. The residue was subjected to silica gel column chromatography, so that 0.40 g of N-((1-hydroxycyclohexyl)methyl)-2-aminoisonicotonic acid amide (hereinafter referred to as the present compound 52) was obtained.

### Present compound 52

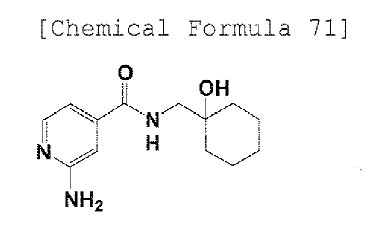

¹H-NMR (CDCl₃) δ: 1.48-1.62 (10H, m), 2.22 (1H, br s), 3.47 (2H, d, J = 5.8Hz), 4.63 (2H, br s), 6.60 (1H, br s), 6.86-6.87 (2H, m), 8.11-8.13 (1H, m).

Then, Formulation Examples are shown. The term "part(s)" represents part(s) by weight.

### Formulation Example 1

50 parts of any one of the present compounds 1 to 52, 3 parts of calcium ligninsulfonate, 2 parts of magnesium laurylsulfate and 45 parts of synthetic hydrated silicon oxide are pulverized and mixed well to give a wettable powder of each compound.

### Formulation Example 2

20 parts of any one of the present compounds 1 to 52 and 1.5 parts of sorbitan trioleate are mixed with 28.5 parts of an aqueous solution containing 2 parts of polyvinyl alcohol, and wet-pulverized finely. To the obtained mixture, 40 parts of an aqueous solution containing 0.05 part of xanthan gum and 0.1 part of aluminium magnesium silicate is added and further 10 parts of propylene glycol is added. The mixture was stirred and mixed to give a flowable of each compound.

### Formulation Example 3

2 parts of any one of the present compounds 1 to 52, 88 parts of kaolin clay and 10 parts of talc are pulverized and mixed well to give a dust of each compound.

### Formulation Example 4

5 parts of each of the present compounds 1 to 52, 14 parts of polyoxyethylenestyryl phenyl ether, 6 parts of calcium dodecylbenzenesulfonate and 75 parts of xylene are mixed well to give an emulsifiable concentrate of each compound.

### Formulation Example 5

2 parts of any one of the present compounds 1 to 52, 1 part of synthetic hydrated silicon oxide, 2 parts of calcium ligninsulfonate, 30 parts of bentonite and 65 parts of kaolin clay are pulverized and mixed well, and water is added thereto and kneeded well, granulated and dried to give granules of each compound.

### Formulation Example 6

10 parts of any one of the present compounds 1 to 52, 35 parts of white carbon containing 50 parts of a polyoxyethylenealkyl ether sulfate ammonium salt, and 55 parts of water are mixed and wet pulverized finely to give a formulation of each compound.

The following Test Examples show that the compound of the present invention is useful for controlling a plant disease.
In the following Test Examples, foliage application was accomplished by spraying each test solution over a different plant.
The controlling effect was evaluated by visually observing the area or lesion spots on each of test plants at the time of investigation and comparing the area of lesion spots on a plant treated with the present compound with that on an untreated plant.

### Test Example 1

### Test of preventive effect on wheat powdery mildew (Erysiphe graminis f. sp. tritici)

Each of plastic pots was filled with sandy loam and sown with wheat (cultivar; Shirogane), followed by growing in a greenhouse for 10 days.
Each of the present compounds 1, 3 to 8 and 12 to 37 was formulated into a flowable formulation according to Formulation Example 6. The flowable formulation was diluted to a predetermined concentration (500 ppm) with water, and foliage application of the dilution was carried out so that the dilution could adhere sufficiently to the surfaces of leaves of the grown wheat seedling.
The wheat which was applied by the dilution was air-dried and then inoculated by sprinkling with spores of *Erysiphe graminis f. sp. tritici*. After the inoculation, the plant was held in a greenhouse at 23°C for 7 days and the area of lesion spots was investigated.
As a result, it was found that the area of lesion spots on the plant treated with any one of the present compounds 8, 16, 17, 18, 21, 22, 24, 26, 28, 31 and 35 was 30% or less of that on an untreated plant.

### Test Example 2

### Test of preventive effect on wheat glue blotch

### (Stagonospora nodorum)

Each of plastic pots was filled with sandy loam and sown with wheat (cultivar; Shirogane), followed by growing in a greenhouse for 10 days.
Each of the present compounds 1. and 3 to 37 was formulated into a flowable formulation according to Formulation Example 6. The flowable formulation was diluted to a predetermined concentration (500 ppm) with water, and foliage application of the dilution was carried out so that the dilution could adhere sufficiently to the surfaces of leaves of the grown wheat seedling.
The wheat which was applied by the dilution was air-dried and then inoculated by spraying a water suspension of spores of *Stagonospora nodorum.*
After the inoculation, the plant was held under darkness and high humidity conditions at 18°C for 4 days and then under lighting conditions for 4 days, and then the area of lesion spots was investigated.
As a result, it was found that the area of lesion spots on the plant treated with any one of the present compounds 4, and 17 to 31 was 30% or less of that on an untreated plant.

### Test Example 3

### Test of preventive effect on wheat Fusarium blight

### (Fusarium culmorum)

Each of plastic pots was filled with sandy loam and sown with wheat (cultivar; Shirogane), followed by growing in a greenhouse for 10 days.
Each of the present compounds 1, 3 to 8 and 12 to 37 was formulated into a flowable formulation according to Formulation Example 6. The flowable formulation was diluted to a predetermined concentration (500 ppm) with water, and foliage application of the dilution was carried out so that the dilution could adhere sufficiently to the surfaces of leaves of the grown wheat seedling.
The wheat which was applied by the dilution was air-dried and then inoculated by spraying a water suspension of spores of *Fusarium culmorum.*
After the inoculation, the plant was held udder darkness and high humidity conditions at 23°C for 4 days and then under lighting conditions for 3 days, and then the area oflesion spots was investigated.
As a result, it was found that the area of lesion spots on the plant treated with any one of the present compounds 15, 16, 17 and 22 was 30% or less of that on an untreated plant.

### Test Example 4

### Test of preventive effect on cucumber gray mold (Botrytis cinerea)

Each of plastic pots was filled with sandy loam and sown with cucumber (variety; Sagamihanjiro), followed by growing in a greenhouse for 12 days.
Each of the present compounds 1, 3 to 48 was formulated into a flowable formulation according to Formulation Example 6. The flowable formulation was diluted to a predetermined concentration (500 ppm) with water, and foliage application of the dilution was carried out so that the dilution could adhere sufficiently to the surfaces of leaves of the grown cucumber seedling.
The cucumber which was applied by the dilution was air-dried and a PDA medium containing spores of Botrytis cinerea was placed on the surfaces of the cucumber leaves. After the inoculation, the plant was grown at 12°C and high humidity for 4 days. Then, the area of lesion spots was investigated.
As a result, it was found that the area of lesion spots on the plant treated with any one of the present compounds 1, 18, 22, 26 and 35 was 30% or less of that on an untreated plant.

### Test Example 5

### Test of preventive effect on cucumber Sclerotinia rot

### (Sclerotinia sclerotiorum)

Each of plastic pots was filled with sandy loam and sown with cucumber (variety; Sagamihanjiro), followed by growing in a greenhouse for 12 days.
Each of the present compounds 3 to 48 was formulated into a flowable formulation according to Formulation Example 6. The flowable formulation was diluted to a predetermined concentration (500 ppm) with water, and foliage application of the dilution was carried out so that the dilution could adhere sufficiently to the surfaces of leaves of the grown cucumber seedling.
The cucumber which was applied by the dilution was air-dried and a PDA medium containing mycelia of Sclerotinia sclerotiorum was placed on the surfaces of the cucumber leaves. After the inoculation, the plant was grown at 18°C and high humidity for 4 days. Then, the area of lesion spots was investigated.
As a result, it was found that the area of lesion spots on the plant treated with any one of the present compounds 3, 4, 24, 26 and 35 was 30% or less of that on an untreated plant.

### Test Example 6

### Test of preventive effect on Alternaria leaf spot

### (Alternaria brassicicola)

Each of plastic pots was filled with sandy loam and sown with Japanese radish (cultivar: Wase 40-nichi), followed by growing in a greenhouse for 5 days.
Each of the present compounds 1, and 3 to 48 was formulated into a flowable formulation according to Formulation Example 6. The flowable formulation was diluted to a predetermined concentration (500 ppm) with water, and foliage application of the dilution was carried out so that the dilution could adhere sufficiently to the surfaces of leaves of the grown Japanese radish seedling.
After the foliage application, the plant was air-dried and then inoculated by spraying a water suspension of spores of Alternaria brassicicola. After the inoculation, the plant was held under high humidity conditions at 24°C for 1 day and then in a greenhouse for 3 days, and then the area oflesion spots was investigated.
As a result, it was found that the area of lesion spots on the plant treated with the present compound 4 or 35 was 30% or less of that on an untreated plant.

### Test Example 7

### Test of curative effect on grape downy mildew (Plasmopara viticola)

Each of plastic pots was filled with sandy loam and sown with grape (cultivar: Berry-A), followed by growing in a greenhouse for 40 days. The grown grape seedling was inoculated by spraying a water suspension of sporangia of Plasmopara viticola. After that, the plant was held under high humidity conditions at 23°C for 1 day and then air-dried to give a Plasmopara viticola-infected seedling.
Each of the present compounds 15 to 48 was formulated into a flowable formulation according to Formulation Example 6. The flowable formulation was diluted to a predetermined concentration (500 ppm) with water, and foliage application of the dilution was carried out so that the dilution could adhere sufficiently to the surfaces of leaves of the grape seedling.
After the foliage application, the plant was air-dried, and held in a greenhouse at 23°C for 5 days and then under high humidity conditions at 23°C for 1 day. Then, the area of lesion spots was investigated.
As a result, it was found that the area of lesion spots on the plant treated with any one of the present compounds 15, 16, 18, 21, 22, 24, 26, 28, 31, 34 and 35 was 30% or less of that an untreated plant.

### Test Example 8

### Test of preventive effect on tomato late blight

### (Phytophthora infestans)

Each of plastic pots was filled with sandy loam and sown with tomato (cultivar; Patio), followed by growing in a greenhouse for 20 days.
Each of the present compounds 1 and 3 to 52 was formulated into a flowable formulation according to Formulation Example 6. The flowable formulation was diluted to a predetermined concentration (500 ppm) with water, and foliage application of the dilution was carried out so that the dilution could adhere sufficiently to the surfaces of leaves of the grown tomato seedling. After the plant was air-dried so that the diluted solution on the leaf surfaces was dried, a water suspension of zoospores of *Phytophthora infestans* was sprayed.
After the inoculation, the plant was held under high humidity conditions at 23°C for 1 day and held in a greenhouse for 4 days, and then the area of lesion spots was investigated.
As a result, it was found that the area of lesion spots on the plant treated with any one of the present compounds 1, 4, 7, 8, 10, 13, 15, 23, 32, 34, 38, 39, 41, 43, 45, 46, 49 and 52 was 30% or less of that an untreated plant.

### Test Example 9

### Test of preventive effect on rice blast (Magnaporthe oryzae)

Each of plastic pots was filled with sandy loam and sown with rice (cultivar; Nippon-bare), followed by growing in a greenhouse for 20 days. Each of the present compounds 1 and 3 to 52 was formulated into a flowable formulation according to Formulation Example 6. The flowable formulation was diluted to a predetermined concentration (500 ppm) with water, and foliage application of the dilution was carried out so that the dilution could adhere sufficiently to the surfaces of leaves of the grown rice seedling. After the application, the plant was air-dried, and held under high humidity conditions at 24°C during the daytime and 20°C during the nighttime for 6 days while the plant was placed in contact with a Magnaporthe oryzae-infected rice seedling (cultivar: Nippon-bare). Then, the area of lesion spots was investigated. As a result, it was found that the area of lesion spots on the plant treated with the present compound 51 was 30% or less of that an untreated plant.

### Test Example 10

### Test of preventive effect on wheat leaf blotch (Septoria tritici)

Each of plastic pots was filled with sandy loam and sown with wheat (cultivar: Apogee), followed by growing in a greenhouse for 10 days. Each of the present compounds 49 to 52 was formulated into a flowable formulation according to Formulation Example 6. The flowable formulation was diluted to a predetermined concentration (500 ppm) with water, and foliage application of the dilution was carried out so that the dilution could adhere sufficiently to the surfaces of leaves of the grown wheat seedling. After the application, the plant was air-dried. After 3 or 4 days, the plant was inoculated by spraying a water suspension of spores of *Septoria tritici*. After the inoculation, the plant was held under high humidity conditions at 18°C for 3 days and then under lighting condition for 14 to 18 days. Then, the area of lesion spots was investigated. As a result, it was found that the area of lesion spots on the plant treated with the present compound 50 was 30% or less of that an untreated plant.

### Industrial Applicability

According to the present invention, plant diseases can be controlled.

## Claims

1. An amide compound represented by formula (1): 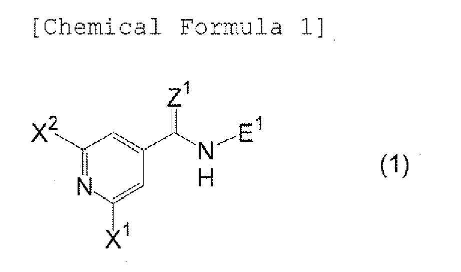 wherein
X¹ represents a fluorine atom, a C1-C4 alkoxy group, a C3-C4 alkenyloxy group, a C3-C4 alkynyloxy group or an NR¹R² group,
X² represents a hydrogen atom, a fluorine atom, a C1-C4 alkyl group, a C2-C4 alkenyl group, a C2-C4 alkynyl group, a C1-C4 haloalkyl group, a C1-C4 alkoxy group, a C1-C4 alkylthio group, a C1-C4 hydroxyalkyl group, a cyano group, a formyl group, an NR³R⁴ group, a CO₂R⁵ group, a CONR⁶R⁷ group, or a phenyl group optionally substituted with at least one member selected from the group consisting of a methyl group, a halogen atom, a cyano group and a nitro group,
Z¹ represents an oxygen atom or a sulfur atom,
E¹ represents an A¹-Cy¹ group or an A²-CR⁸R⁹R¹⁰ group,
A¹ represents a single bond, methylene, -CH(CH₃)-, -C(CH₃)₂-, -CH(CH₂CH₃)-, -CH₂CH₂-, -CH₂CF₂CH₂-, cyclopropylidene, or methylene substituted with at least one member selected from the group consisting of a C1-C3 haloalkyl group, a C2-C4 alkenyl group, a C2-C4 alkynyl group, a cyano group and a C2-C5 alkoxycarbonyl group,
A² represents methylene, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group,
Cy¹ represents a C3-C6 cycloalkyl group optionally substituted with at least one member selected from the group [a-1] shown below, a C3-C6 cycloalkenyl group optionally substituted with at least one member selected from the group [a-1] shown below, a C3-C6 cycloalkyl group optionally substituted with at least one member selected from the group [a-1] shown below wherein one methylene forming ring is replaced by a carbonyl group, or a C3-C6 hydroxyiminocycloalkyl group optionally substituted with at least one member selected from the group [a-1] shown below,
R¹ and R² independently represent a hydrogen atom, a C1-C4 alkyl group, a C3-C4 alkenyl group, a C3-C4 alkynyl group, a C2-C4 haloalkyl group, a C2-C5 alkylcarbonyl group, a C2-C5 alkoxycarbonyl group or a C1-C4 alkylsulfonyl group,
R³ and R⁴ independently represent a hydrogen atom, a C1-C4 alkyl group, a C3-C4 alkenyl group, a C3-C4 alkynyl group, a C2-C4 haloalkyl group, a C2-C5 alkylcarbonyl group, a C2-C5 alkoxycarbonyl group or a C1-C4 alkylsulfonyl group,
R⁵ represents a C1-C4 alkyl group, a C3-C4 alkenyl group or a C3-C4 alkynyl group,
R⁶ represents a hydrogen atom, a C1-C4 alkyl group, a C3-C4 alkenyl group, a C3-C4 alkynyl group, a C2-C4 haloalkyl group, a C2-C5 alkylcarbonyl group, a C2-C5 alkoxycarbonyl group or a C1-C4 alkylsulfonyl group,
R⁷ represents a hydrogen atom, a C1-C4 alkyl group, a C3-C4 alkenyl group, a C3-C4 alkynyl group or a C2-C4 haloalkyl group,
R⁸ and R⁹ independently represent a C1-C4 alkyl group,
R¹⁰ represents a hydrogen atom, a C1-C4 alkyl group, a C2-C4 alkenyl group, a C2-C4 alkynyl group, a cyano group, a carboxyl group, a C2-C5 alkoxycarbonyl group, a halogen atom, a hydroxyl group, a C1-C6 alkoxy group, a C3-C6 alkenyloxy group, a C1-C6 haloalkyl group, a C2-C6 haloalkoxy group, a C1-C3 alkylthio group, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a C2-C6 cyanoalkyl group, a C1-C3 alkylsulfonyl group, or an NR¹¹R¹² group, in which R¹¹ and R¹² independently represent a hydrogen atom, a C1-C4 alkyl group, a C2-C5 alkylcarbonyl group, a C2-C5 alkoxycarbonyl group or a C1-C4 alkylsulfonyl group, and
the group [a-1]:
a halogen atom, a C1-C4 alkyl group, a C2-C4 alkenyl group, a C2-C4 alkynyl group, a hydroxyl group, a cyano group, a carboxyl group, a C2-C5 alkoxycarbonyl group, a C1-C6 alkoxy group, a C3-C6 alkenyloxy group, a C1-C6 haloalkyl group, a C1-C6 haloalkoxy group, a C1-C3 alkylthio group, a C1-C3 alkylidene group which forms a double bond with a ring-forming carbon atom, a C1-C6 hydroxyalkyl group, C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a formyl group, a C2-C6 cyanoalkyl group, a C1-C3 alkylsulfonyl group, a phenoxy group, or an NR¹³R¹⁴ group, in which R¹³ and R¹⁴ independently represent a hydrogen atom, a C1-C4 alkyl group, a C2-C5 alkylcarbonyl group, a C2-C5 alkoxycarbonyl group and a C1-C4 alkylsulfonyl group.

2. The amide compound according to claim 1, wherein A¹ is a single bond, methylene, -CH(CH₃)-, -C(CH₃)₂-, -CH(CH₂CH₃)-, or methylene substituted with at least one member selected from the group consisting of a C2-C4 alkynyl group and a C2-C4 alkynyl group,
A² is methylene, -CH(CH₃)-, -C(CH₃)₂-, -CH(CH₂CH₃)-, or methylene substituted with at least one member selected from the group consisting of a C2-C4 alkenyl group and a C2-C4 alkynyl group,
R¹⁰ is a hydrogen atom, a C1-C4 alkyl group, a C2-C4 alkenyl group, a C2-C4 alkynyl group, a halogen atom, a hydroxyl group, a C1-C6 alkoxy group, a C3-C6 alkenyloxy group, a C1-C3 alkylthio group or a C1-C6 hydroxyalkyl group, and
the group [a-1] consists of a halogen atom, a C1-C4 alkyl group, a C2-C4 alkenyl group, a C2-C4 alkynyl group, a hydroxyl group, a cyano group, a C1-C6 alkoxy group, a C3-C6 alkenyloxy group, a C1-C6 haloalkyl group, a C1-C6 haloalkoxy group, a C1-C3 alkylthio group, a C1-C3 alkylidene group which forms a double bond with a ring-forming carbon atom, a C1-C6 hydroxyalkyl group and a C2-C4 alkylcarbonyloxy group.

3. The amide compound according to claim 1 or 2, wherein A¹ is a single bond, methylene or -CH(CH₃)-,
A² is methylene, -CH(CH₃)- or -C(CH₃)₂-,
R¹⁰ is a hydrogen atom, a C1-C4 alkyl group, a C2-C4 alkenyl group, a C2-C4 alkynyl group, a halogen atom, a hydroxyl group, a C1-C6 alkoxy group, a C3-C6 alkenyloxy group, a C1-C3 alkylthio group or a C1-C6 hydroxyalkyl group,
Cy¹ is a C3-C6 cycloalkyl group optionally substituted with at least one member selected from a halogen atom, a C1-C4 alkyl group and a hydroxyl group, or a C3-C6 cycloalkenyl group optionally substituted with at least one member selected from a halogen atom, a C1-C4 alkyl group and a hydroxyl group.

4. The amide compound according to claim 1 or 2, wherein E¹ is an A¹-Cy¹ group, A¹ is a single bond, methylene, -CH(CH₃)-, -C(CH₃)₂-, -CH(CH₂CH₃)-, or methylene substituted with at least one member selected from the group consisting of a C2-C4 alkenyl group and a C2-C4 alkynyl group, and
the group [a-1] consists of a halogen atom, a C1-C4 alkyl group, a C2-C4 alkenyl group, a C2-C4 alkynyl group, a hydroxyl group, a cyano group, a C1-C6 alkoxy group, a C3-C6 alkenyloxy group, a C1-C6 haloalkyl group, a C1-C6 haloalkoxy group, a C1-C3 alkylthio group, a C1-C3 alkylidene group which forms a double bond with a ring-forming carbon atom, a C1-C6 hydroxyalkyl group and a C2-C4 alkylcarbonyloxy group.

5. The amide compound according to claim 1 or 2, wherein E¹ is an A²-CR⁸R⁹R¹⁰ group, A² is methylene,
- CH(CH₃)-, -C(CH₃)₂-, -CH(CH₂CH₃)-, or a methylene group substituted with at least one member selected from the group consisting of a C2-C4 alkenyl group and a C2-C4 alkynyl group, and R¹⁰ is a hydrogen atom, a C1-C4 alkyl group, a C2-C4 alkenyl group, a C2-C4 alkynyl group, a halogen atom, a hydroxyl group, a C1-C6 alkoxy group, a C3-C6 alkenyloxy group, a C1-C3 alkylthio group or a C1-C6 hydroxyalkyl group.

6. The amide compound according to any one of claims 1 to 5, wherein X¹ is a fluorine atom, a C1-C4 alkoxy group or an NR¹R² group, R¹ is a hydrogen atom or a C1-C4 alkyl group, R² is a hydrogen atom or a C1-C4 alkyl group, and Z¹ is an oxygen atom.

7. The amide compound according to any one of claims 1 to 5, wherein X¹ is a fluorine atom, a methoxy group or an amino group, X² is a hydrogen atom, a fluorine atom, a methoxy group, a methylthio group or an amino group, and Z¹ is an oxygen atom.

8. A plant disease control composition comprising the amide compound according to any one of claims 1 to 7.

9. A method for controlling plant diseases, which comprises applying an effective amount of the amide compound according to any one of claims 1 to 7 to plants or soil.
